(19) Europäisches Patentamt
European Patent Office
Office européen des brevets

(11) **EP 4 190 360 A1**

(12) **EUROPEAN PATENT APPLICATION**
published in accordance with Art. 153(4) EPC

(43) Date of publication:
**07.06.2023 Bulletin 2023/23**

(21) Application number: **21850002.3**

(22) Date of filing: **21.07.2021**

(51) International Patent Classification (IPC):
***A61K 47/55*** *(2017.01)* ***A61K 47/60*** *(2017.01)*
***A61K 31/517*** *(2006.01)* ***A61P 35/00*** *(2006.01)*

(52) Cooperative Patent Classification (CPC):
**A61K 31/517; A61K 45/00; A61K 47/55; A61K 47/60; A61K 47/64; A61P 35/00; A61P 35/02; A61P 35/04; C08G 65/332; C08G 65/333**

(86) International application number:
**PCT/CN2021/107555**

(87) International publication number:
**WO 2022/022354 (03.02.2022 Gazette 2022/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.07.2020 CN 202010737415**

(71) Applicant: **Chongqing Upgra Biotechnology Co., Ltd.**
**Chongqing 401120 (CN)**

(72) Inventors:
- **LI, Gaoquan**
  **Chongqing 401120 (CN)**
- **LIU, Nian**
  **Chongqing 401120 (CN)**
- **ZENG, Xiafan**
  **Chongqing 401120 (CN)**
- **PENG, Yongchen**
  **Chongqing 401120 (CN)**
- **MEI, Gang**
  **Chongqing 401120 (CN)**
- **YANG, Shuai**
  **Chongqing 401120 (CN)**
- **GAO, Yang**
  **Chongqing 401120 (CN)**
- **GAUN, Sheng**
  **Chongqing 401120 (CN)**
- **YIN, Yifeng**
  **Chongqing 401120 (CN)**
- **CHEN, Huiyu**
  **Chongqing 401120 (CN)**
- **LOU, Jie**
  **Chongqing 401120 (CN)**
- **QIAN, Kun**
  **Chongqing 401120 (CN)**
- **YANG, Xiangwei**
  **Chongqing 401120 (CN)**
- **LIU, Liwei**
  **Chongqing 401120 (CN)**
- **LI, Zhenwei**
  **Chongqing 401120 (CN)**
- **HU, Kaixiong**
  **Chongqing 401120 (CN)**
- **LIU, Hua**
  **Chongqing 401120 (CN)**
- **ZHANG, Qian**
  **Chongqing 401120 (CN)**
- **LI, Dajun**
  **Chongqing 401120 (CN)**
- **WEI, Yusong**
  **Chongqing 401120 (CN)**
- **DING, Xiaoling**
  **Chongqing 401120 (CN)**
- **LIU, Xi**
  **Chongqing 401120 (CN)**
- **HUANG, Liqun**
  **Chongqing 401120 (CN)**
- **TU, Tao**
  **Chongqing 401120 (CN)**

(74) Representative: **Cabinet Nony**
**11 rue Saint-Georges**
**75009 Paris (FR)**

(54) **POLYETHYLENE GLYCOL CONJUGATE DRUG SYNERGIST, PREPARATION METHOD THEREFOR, AND USE THEREOF**



Processed by Luminess, 75001 PARIS (FR)

**(Cont. next page)**

(57) A polyethylene glycol conjugate drug synergist, a preparation method therefor, and use thereof, specifically relating to a polyethylene glycol conjugate drug synergist as shown in formula I or a pharmaceutically acceptable salt thereof, a preparation method for the polyethylene glycol conjugate drug synergist or the pharmaceutically acceptable salt thereof, an intermediate for preparing the polyethylent glycol conjugate drug synergist or the pharmaceutically acceptable salt thereof, a composition containing the polyethylene glycol conjugate drug synergist or the pharmaceutically acceptable salt thereof, and use of the polyethylene glycol conjugate drug synergist or the pharmaceutically acceptable salt thereof in preparation of a drug.

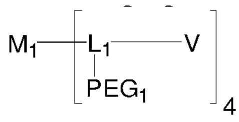

formula I

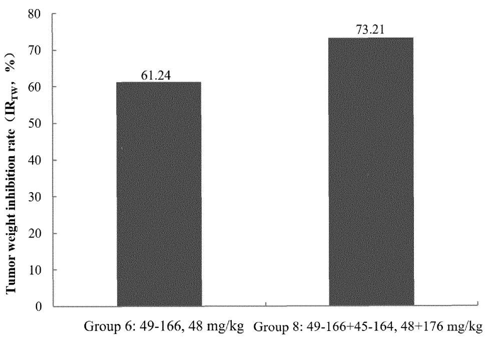


Figure 2

## Description

TECHNICAL FIELD

The present invention belongs to the technical field of medicine, and relates to a polyethylene glycol conjugated drug synergist, preparation method thereof and use thereof.

BACKGROUND

Polymer conjugated drug can greatly increase water solubility of drug molecules. Most small-molecule drugs can only stay in the blood circulation for a few minutes, while polymer conjugated drugs may stay for tens or hundreds of hours or even longer, which is beneficial for the "enhanced permeability and retention" effect (i.e., the EPR effect) caused by leakage of tumor capillaries. Due to the increased hydrodynamic volume of the polymer conjugated drugs, the renal elimination of the drugs is weakened, the drugs are protected from enzymatic degradation, the half-life of the drugs in plasma is extended, and the bioavailability of the drugs is increased. Moreover, the anticancer drugs can be highly enriched in diseased organs, tissues or cells through active targeting or the EPR passive targeting, thereby greatly reducing the toxic side effects caused by small molecule anticancer drugs spreading all over the body. In addition, the polymer conjugated drugs can limit the cell absorption of drugs to the endocytic pathway, which is conducive to drug delivery to the lysosome, thereby avoiding drug resistance caused by p-glycoprotein pumping out; the polymer conjugated drugs can also stimulate or restore immune function, and this is conducive to killing cancer cells.

Polyethylene glycol is the most successful carrier in the field of polymer conjugated drug nanomedicine, and is called as a "gold standard" carrier. In the last 30 years, the technology of pegylated drugs has achieved tremendous success, and there are 17 pegylated drugs which have been approved by the FDA in USA to enter the market, and 1 pegylated drug which has been approved by the NMPA in China to enter the market. In addition, nearly 40 new clinical drugs are in the first-phase, second-phase, third-phase clinical trials or in the NDA phase, wherein half of the new clinical drugs are pegylated small-molecule drugs.

SUMMARY

The present application is based on the inventors' discovery and recognition of the following facts and problems: The inventors find that the recently developed internalization RGD polypeptide (iRGD, CRGDK/RGPD/EC), which is a cyclic peptide with tumor targeting and cell penetration, can increase the capacity of an anticancer drug to enter extravascular tumor tissues in a tumor-specific manner depending on neuropeptide-1, thereby enhancing the therapeutic efficacy of combined anticancer drug. However, iRGD is clinically very easy to hydrolyze and has a short half-life, so it is difficult to exert its effect, while iRGD-regulated polymer conjugated drug can significantly improve the effect of tumor therapy by enhancing the enrichment and penetration of the drug at the tumor site. Therefore, the inventors provide a polyethylene glycol conjugated drug synergist. The inventors connect iRGD onto a polyethylene glycol polymer carrier through a special chemical synthesis method to synthesize a polyethylene glycol conjugated iRGD, which is transported to a tumor site through blood circulation. When polyethylene glycol conjugated iRGD is combined with an anticancer drug, the iRGD can be degraded in a tumor microenvironment, and the therapeutic efficacy of the anticancer drug is enhanced.

In the first aspect of the present invention, the present invention provides a polyethylene glycol conjugated drug synergist of formula (I) or a pharmaceutically acceptable salt thereof,

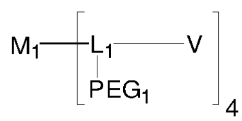

formula I

wherein:

$M_1$ is

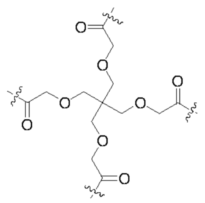 or 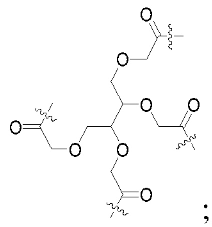 ;

$PEG_1$ is single-arm polyethylene glycol segment, $PEG_1$ is connected to $L_1$ through carbonyl group or $PEG_1$ has amino group or activated amino group at its terminal, the number-average molecular weight of $PEG_1$ is 5k-40k, preferably 5k-10k or 10k-40k, and more preferably 10k;

$L_1$ is

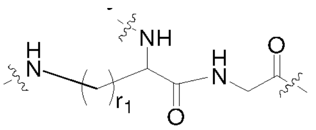 or 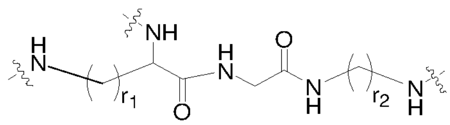 ,

each $r_1$ independently is 1, 2, 3, 4, 5 or 6, preferably 1, 2, 3 or 4, more preferably 3 or 4; $r_2$ is 1 2, 3, 4, 5 or 6, preferably 1 2, 3 or 4, more preferably 1 or 2;

V is

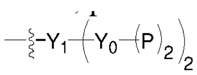 ;

Y1, Y0 are each independently selected from

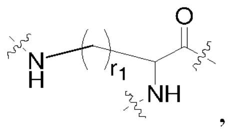 ,

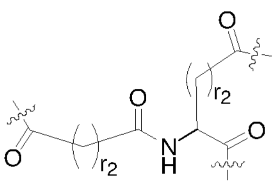 or 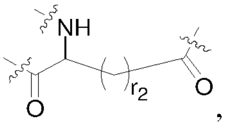 ,

$r_1$ is 1, 2, 3, 4, 5 or 6, $r_1$ is preferably 1, 2, 3 or 4, $r_1$ is more preferably 3 or 4, each $r_2$ independently is 1, 2, 3, 4, 5 or 6, each $r_2$ independently is preferably 1, 2, 3 or 4, each $r_2$ independently is more preferably 1 or 2;

P is $-L_V-T$;

$L_V$ is selected from

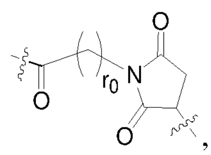 , 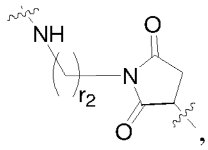 ,

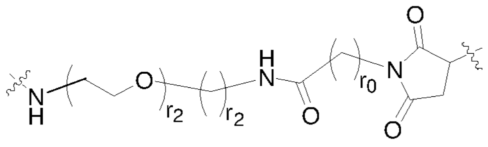 or 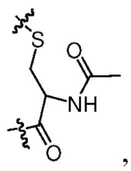 ,

each $r_0$ independently is 1, 2, 3, 4, 5 or 6, each $r_0$ independently is preferably 3, 4, 5 or 6, each $r_0$ independently is more preferably 5 or 6, each $r_2$ independently is 1, 2, 3, 4, 5 or 6, each $r_2$ independently is preferably 1, 2, 3 or 4, each $r_2$ independently is more preferably 1 or 2;

T is

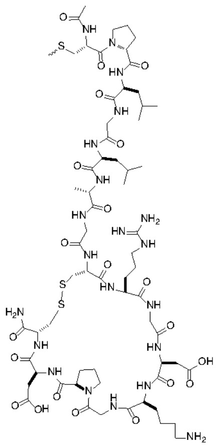 or 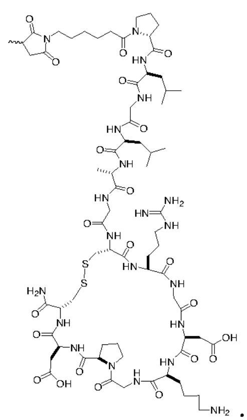 .

In some embodiments, $L_1$ is

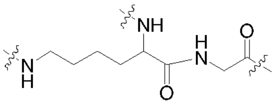 or 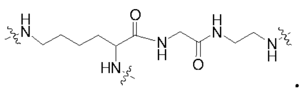 .

In some embodiments, Y1, Y0 are each independently selected from

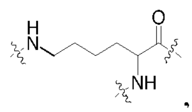 , 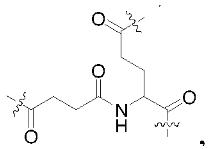 , 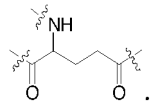 .

In some embodiments Y1 is selected from

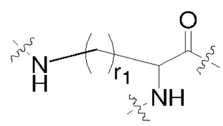 or

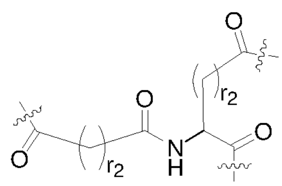 ,

$r_1$ is 3 or 4, each $r_2$ independently is 1 or 2.

In some embodiments, Y1 is selected from

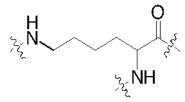 or 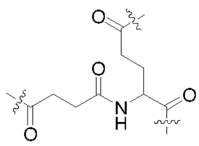.

In some embodiments, Y0 is selected from

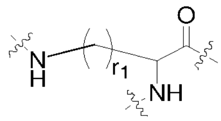

or

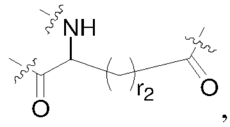,

$r_1$ is 3 or 4, $r_2$ is 1 or 2.

In some embodiments, Y0 is selected from

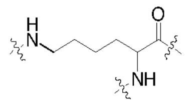 or 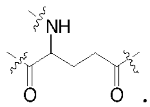.

In some embodiments, $L_V$ is selected from

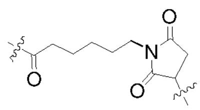, 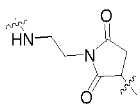,

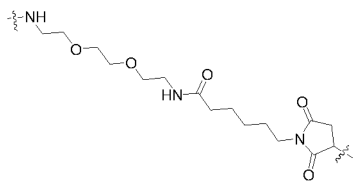 or 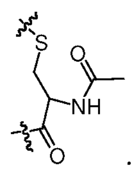.

In the second aspect of the present invention, the present invention provides a polyethylene glycol conjugated drug synergist of formula (I') or a pharmaceutically acceptable salt thereof

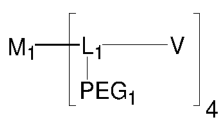

formula I'

wherein:

$M_1$ is

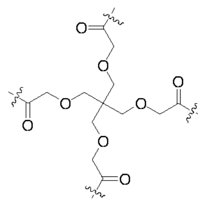 or 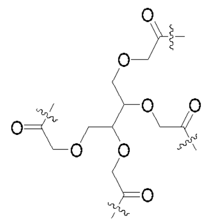 ;

$PEG_1$ is single-arm polyethylene glycol segment, $PEG_1$ has amino group or activated amino group at its terminal, the number-average molecular weight of $PEG_1$ is 5k-40k, preferably 5k-10k or 10k-40k, and more preferably 10k;

$L_1$ is

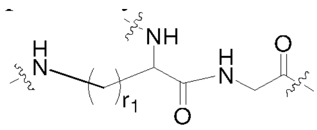 ,

$r_1$ is 1 2, 3 4, 5 or 6, preferably 1 2, 3 or 4, more preferably 3 or 4;

V is

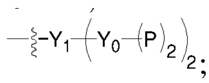

Y1, Y0 are each independently selected from

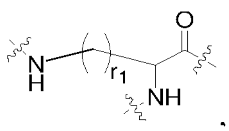 ,

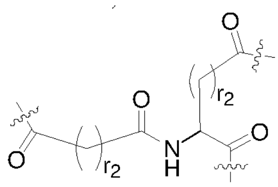 or 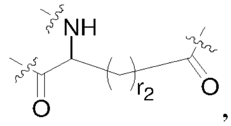 ,

$r_1$ is 1, 2, 3, 4, 5 or 6, $r_1$ is preferably 1, 2, 3 or 4, $r_1$ is more preferably 3 or 4, each $r_2$ independently is 1, 2, 3, 4, 5 or 6, each $r_2$ independently is preferably 1, 2, 3 or 4, each $r_2$ independently is more preferably 1 or 2;

P is $-L_V-T$

$L_V$ is selected from

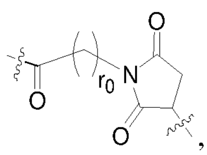 , 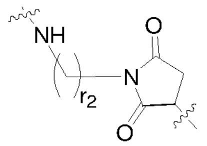 or

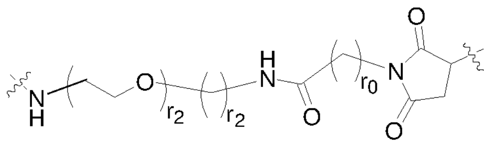

each $r_0$ independently is 1, 2, 3, 4, 5 or 6, each $r_0$ independently is preferably 3, 4, 5 or 6, each $r_0$ independently is more preferably 5 or 6, each $r_2$ independently is 1, 2, 3, 4, 5 or 6, each $r_2$ independently is preferably 1, 2, 3 or 4, each $r_2$ independently is more preferably 1 or 2;

T is

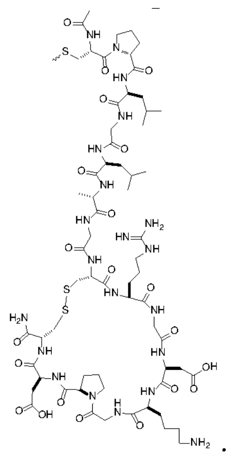

In some embodiments, $L_1$ is

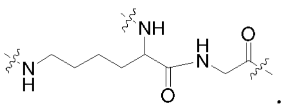

In some embodiments, Y1, Y0 are each independently selected from

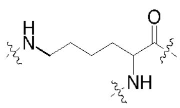,
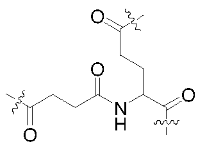,
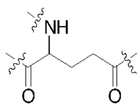.

In some embodiments, Y1 is selected from

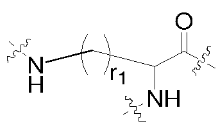

or

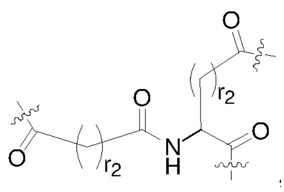

$r_1$ is 3 or 4, each $r_2$ independently is 1 or 2.

In some embodiments, Y1 is selected from

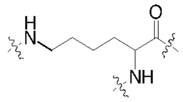

or

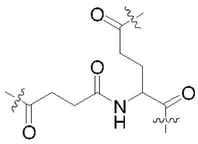

.

In some embodiments, Y0 is selected from

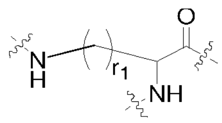

or

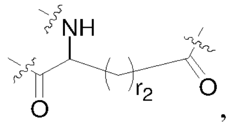

,

$r_1$ is 3 or 4, $r_2$ is 1 or 2.

In some embodiments, Y0 is selected from

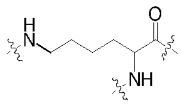

or

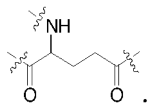

.

In some embodiments $L_V$ is selected from

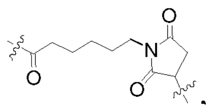

,

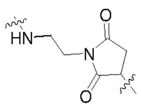

or

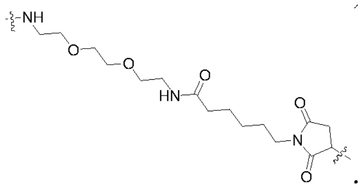

.

In the third aspect of the present invention, the present invention provides a polyethylene glycol conjugated drug synergist or a pharmaceutically acceptable salt thereof, wherein, the polyethylene glycol conjugated drug synergist is selected from:

| No. | Structural formula |
| --- | --- |
| 45-164 | 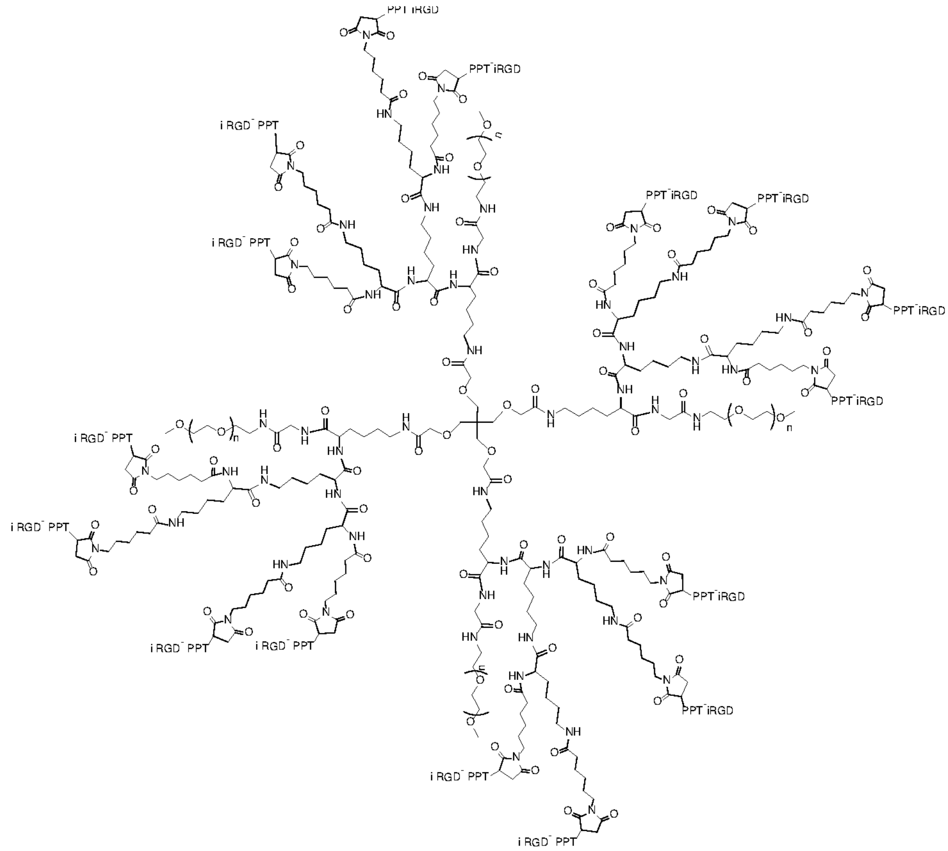 |
| | wherein, 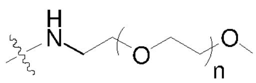 has a number-average molecular weight of 10k |

(continued)

| No. | Structural formula |
|---|---|
| 38-161 | 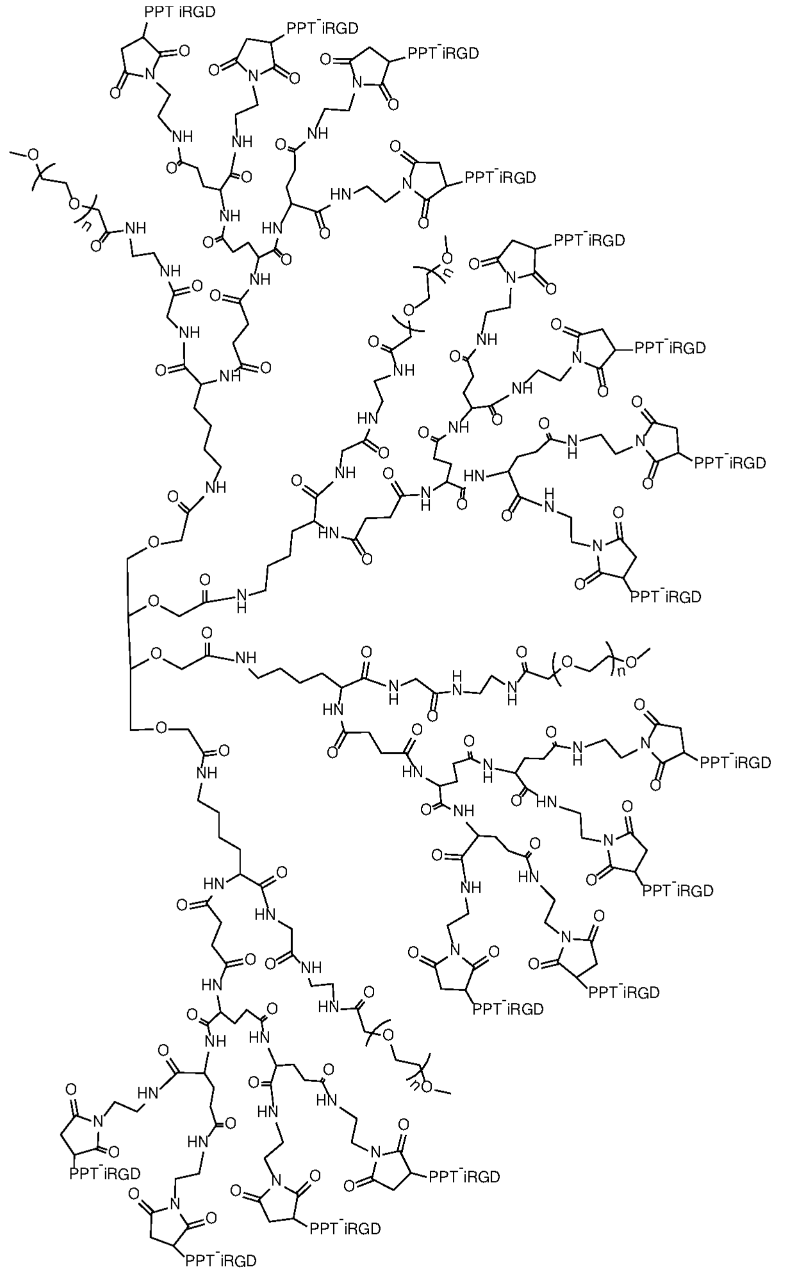<br>wherein, 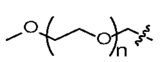 has a number-average molecular weight of 10k |

(continued)

| No. | Structural formula |
|---|---|
| 38-192 | 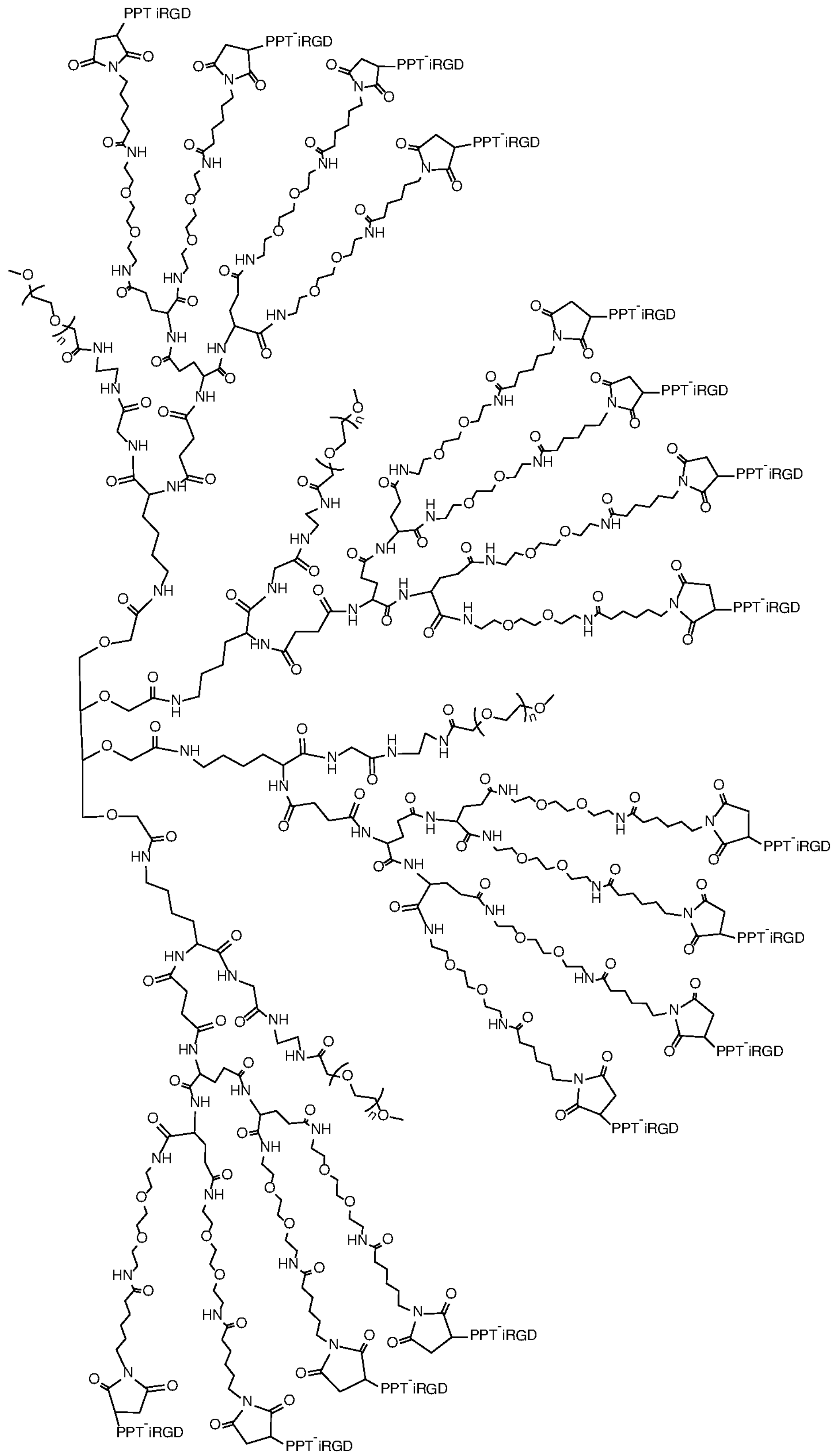<br>wherein, 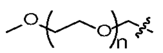 has a number-average molecular weight of 10k |

(continued)

| No. | Structural formula |
|---|---|
| 52-95 | 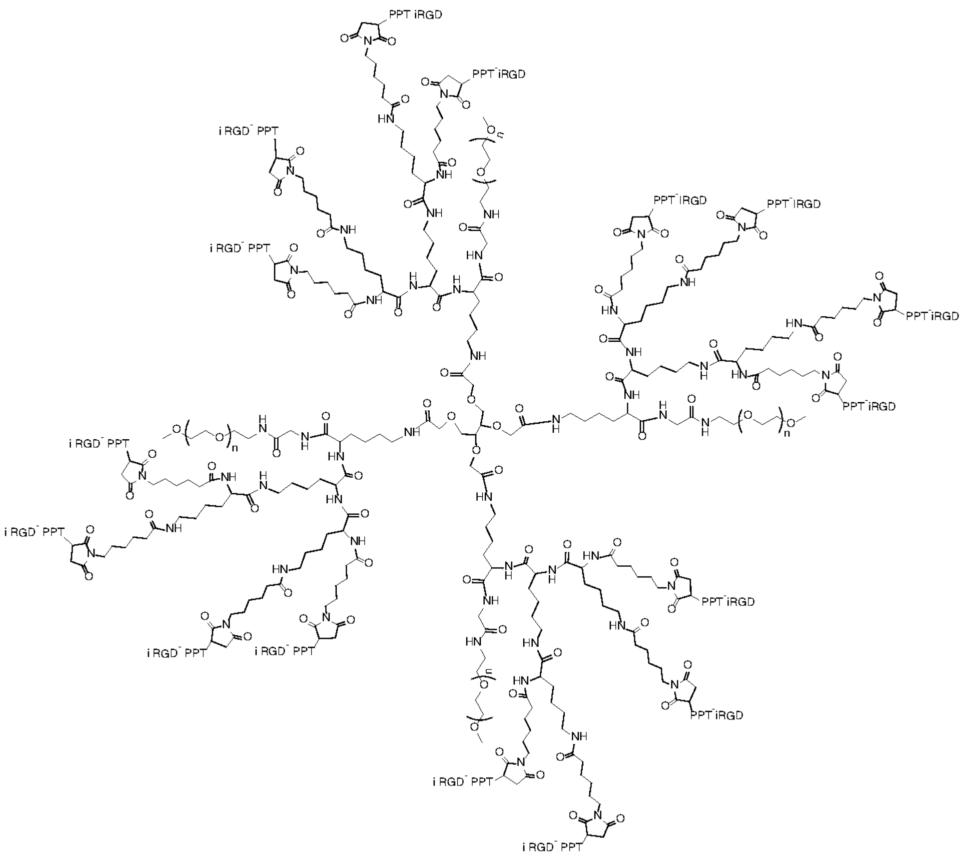<br>wherein, 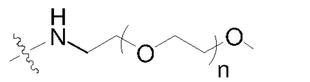 has a number-average molecular weight of 10k |

(continued)

| No. | Structural formula |
|---|---|
| 56-89 | 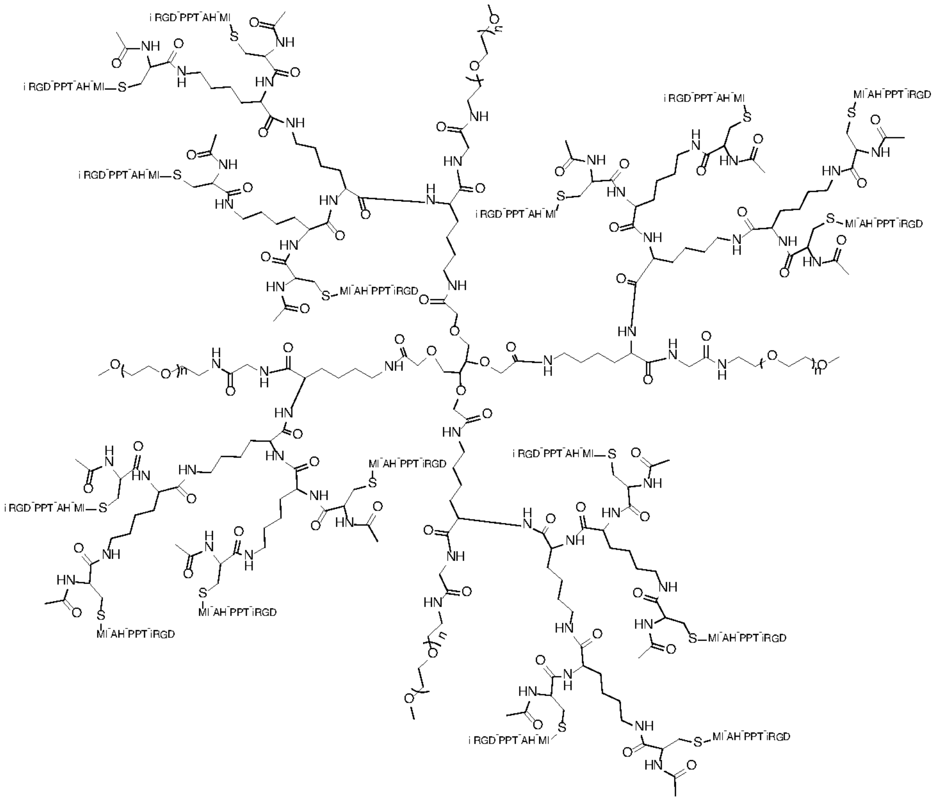 |
| | wherein, 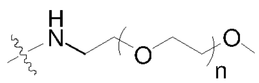 has a number-average molecular weight of 10k |

In the fourth aspect of the present invention, the present invention provides a method for preparing the above-mentioned polyethylene glycol conjugated drug synergist or a pharmaceutically acceptable salt thereof, comprising the following steps:

(1) preparing the intermediate

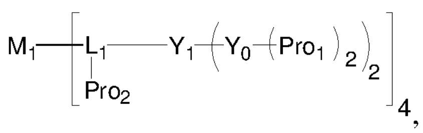

wherein:

$M_1$, $L_1$, $Y_1$, $Y_0$ are as defined above,
$Pro_2$ is a protecting group for carboxyl group, preferably, $Pro_2$ is benzyloxy group,
$Pro_1$ is a protecting group for amino group or carboxyl group, preferably, when $Pro_1$ is a protecting group for amino group, $Pro_1$ is tert-butoxy carbonyl group, preferably, when $Pro_1$ is a protecting group for carboxyl group, $Pro_1$ is tert-butoxy group;

(2) subjecting the intermediate

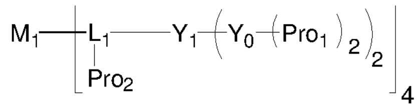

to the first deprotection, to obtain the intermediate

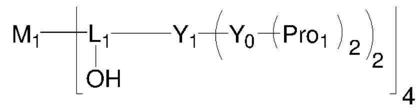

, wherein,

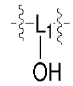

has carboxyl group at its terminal;

(3) allowing the intermediate

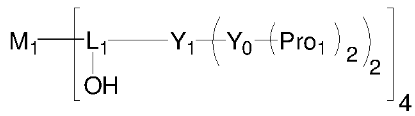

and $PEG_1$ to carry out amidation reaction to obtain the intermediate

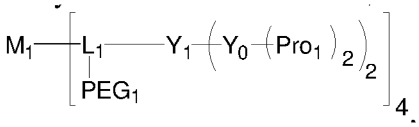

wherein, $PEG_1$ is as defined above;

(4) when $Pro_1$ is a protecting group for amino group, subjecting the intermediate

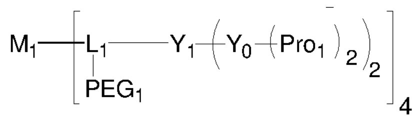

to the second deprotection, to obtain the intermediate

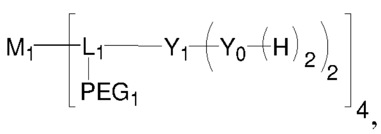

wherein,

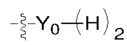

has amino group at its terminal; or when $Pro_1$ is a protecting group for carboxyl group, subjecting the intermediate

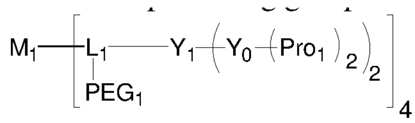

to the third deprotection, to obtain the intermediate

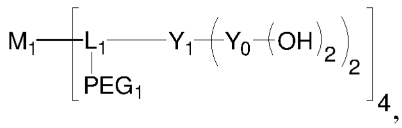

wherein,

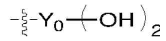

has carboxyl group at its terminal;
(5) allowing the intermediate

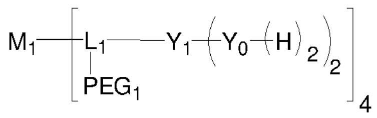

and

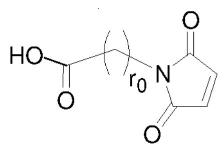

to carry out amidation reaction, to obtain the intermediate

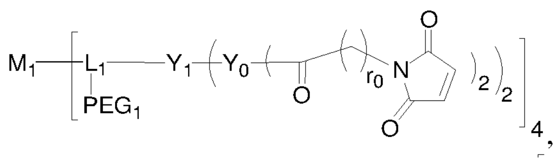

wherein, $r_0$ is as defined above; or, allowing the intermediate

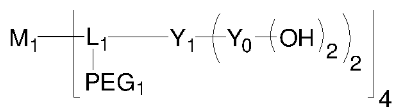

and

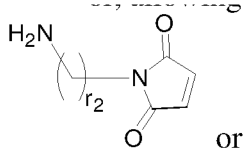

or

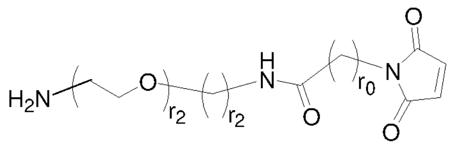

to carry out amidation reaction, to obtain the intermediate

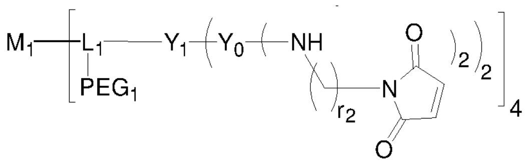

or

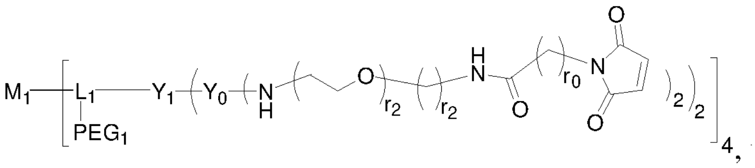

wherein, $r_0$, $r_2$ are as defined above;
(6) allowing the intermediate

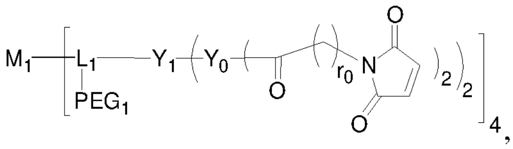

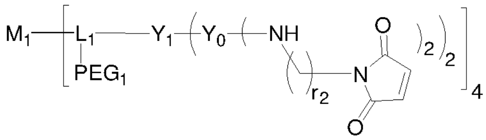

or

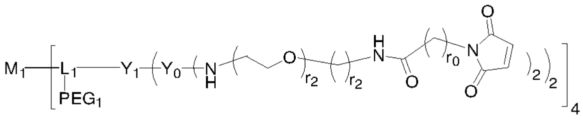 and 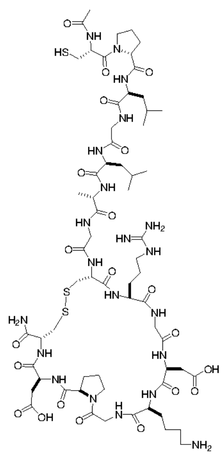

to carry out addition reaction, to obtain the above-mentioned polyethylene glycol conjugated drug synergist.

In some embodiments, the intermediate

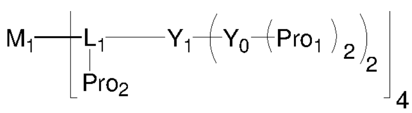

is selected from:

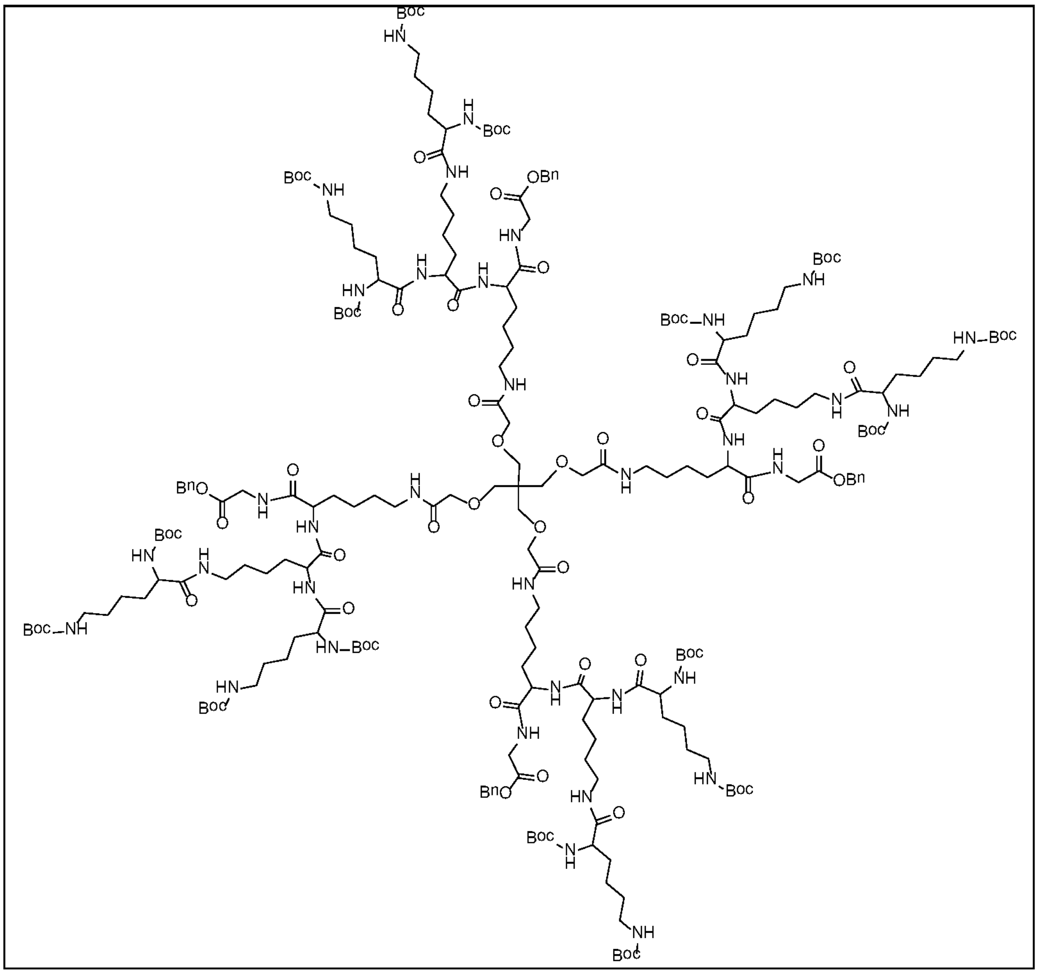

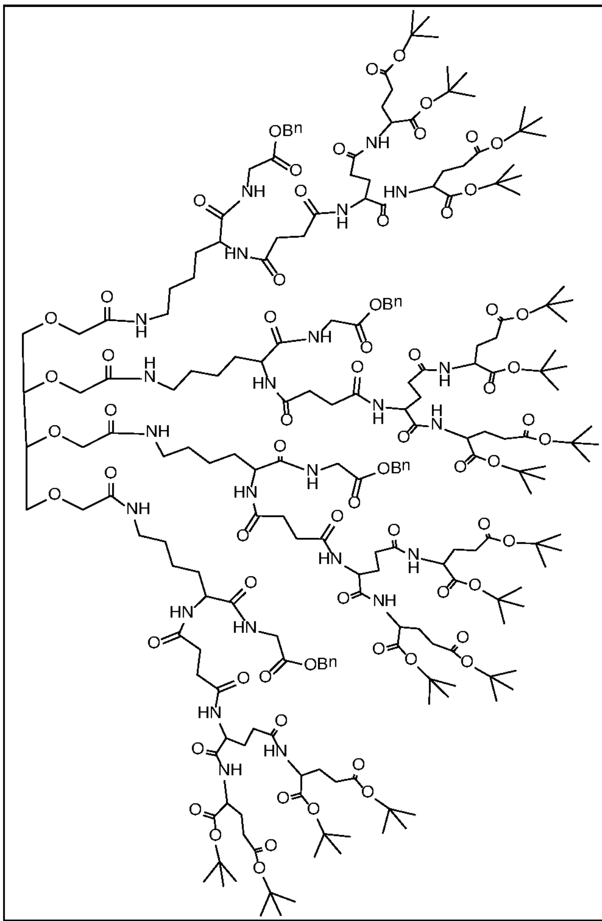

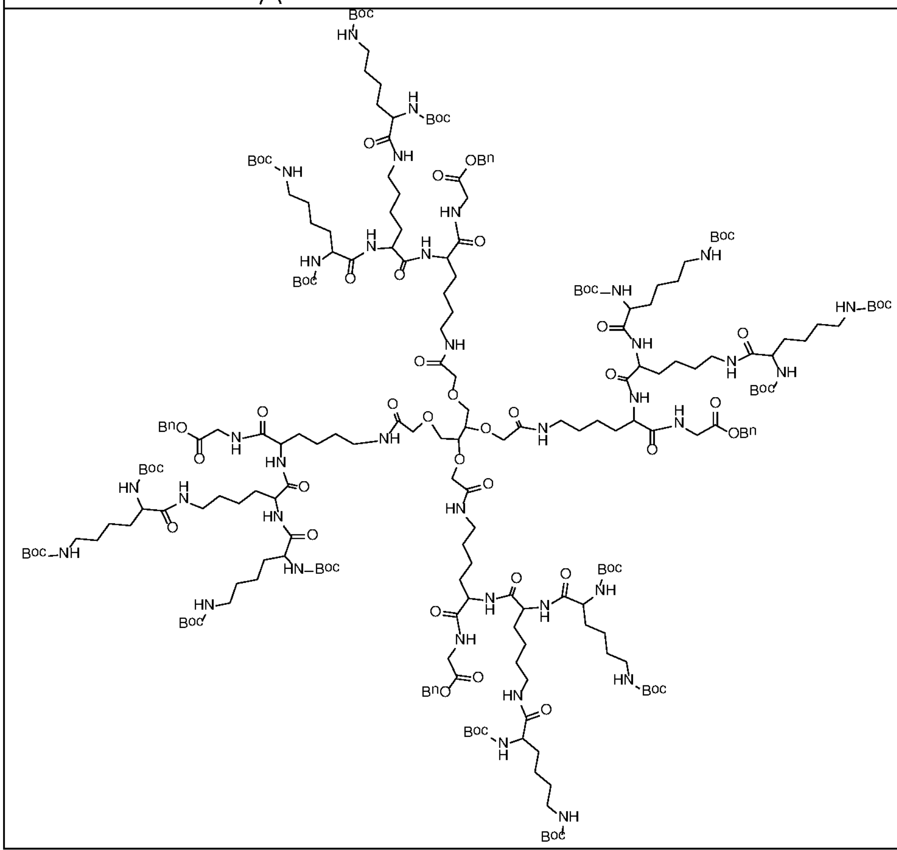

In the fifth aspect of the present invention, the present invention provides an intermediate for preparing the above-mentioned polyethylene glycol conjugated drug synergist or a pharmaceutically acceptable salt thereof, the intermediate being selected from:

| No. | Structural formula |
|---|---|
| 45-136 | 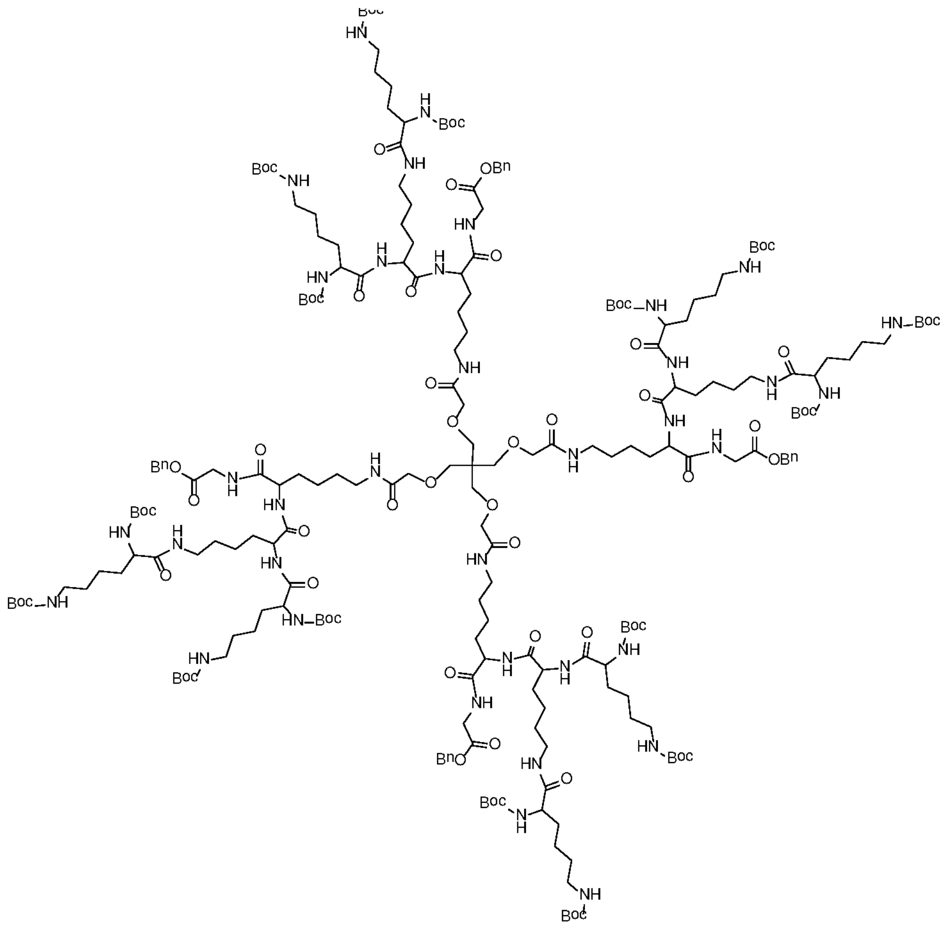 |

(continued)

| No. | Structural formula |
| --- | --- |
| 38-146 | 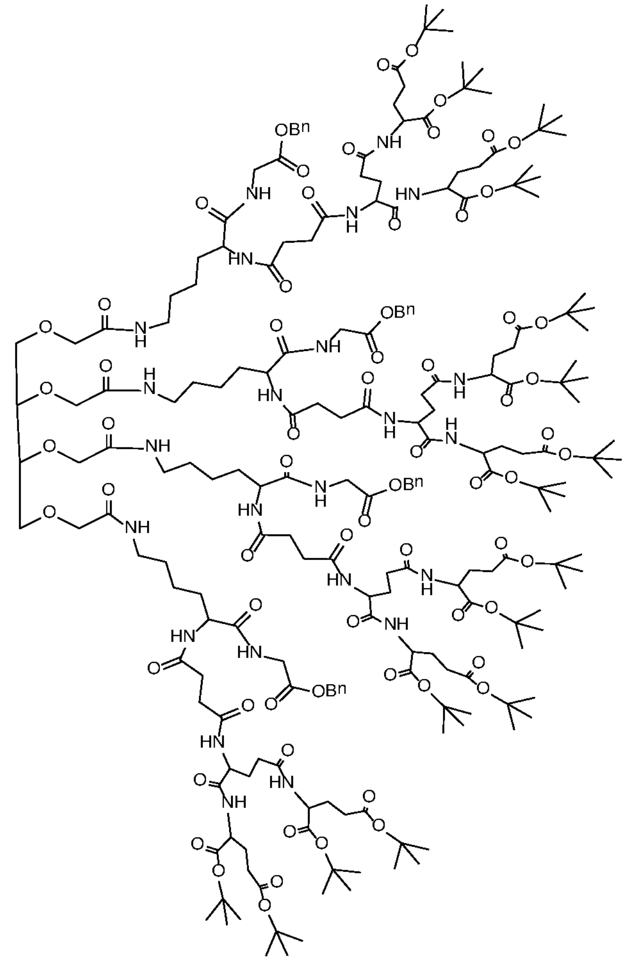 |

(continued)

| No. | Structural formula |
|---|---|
| 52-86 | 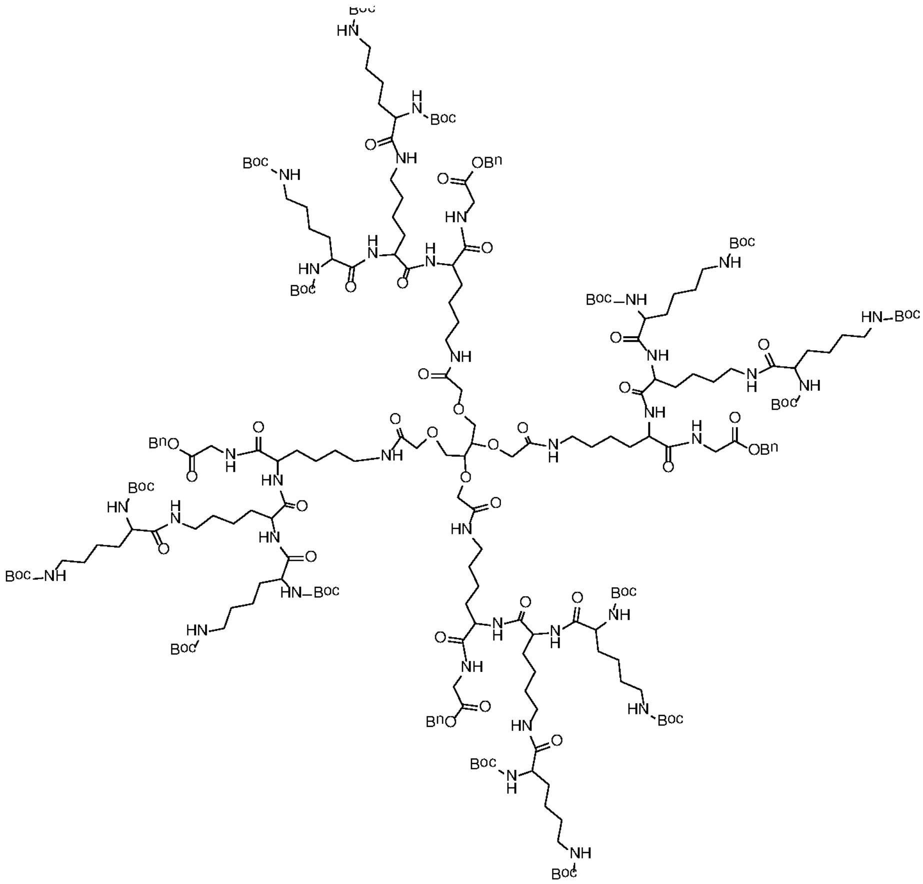 |

In the sixth aspect of the present invention, the present invention provides a composition comprising the above-mentioned polyethylene glycol conjugated drug synergist or a pharmaceutically acceptable salt thereof.

In some embodiments, the composition further comprises one or more pharmaceutically acceptable excipients, such as carriers and/or vehicles. The carriers and/or vehicles include, but are not limited to: ion exchangers, alumina, aluminum stearate, lecithin, serum proteins such as human serum protein, buffer substances such as phosphate, glycerin, sorbic acid, potassium sorbate, a mixture of partial glycerides of saturated plant fatty acids, water, salt or electrolyte, such as protamine sulfate, disodium hydrogen phosphate, potassium hydrogen phosphate, sodium chloride, zinc salt, colloidal silica, magnesium trisilicate, polyvinylpyrrolidone, cellulose material, polyethylene glycol, sodium carboxymethyl cellulose, polyacrylate, beeswax, polyethylene- polyoxypropylene block polymer, and lanolin.

The pharmaceutical composition may be prepared into any pharmaceutically acceptable dosage form. The pharmaceutical composition may also be applied to individuals in need of such treatment in any suitable way of administration, such as oral, parenteral, rectal or pulmonary administration. In the case of oral administration, the pharmaceutical composition may be made into conventional solid preparations, such as tablets, capsules, pills, granules, etc.; it may also be made into oral liquid preparations, such as oral solutions and oral suspensions, and syrup. When the pharmaceutical composition is made into oral preparations, suitable fillers, binders, disintegrants, lubricants, etc. may be added. In the case of parenteral administration, the pharmaceutical composition may be made into injection preparations, including injection solutions, sterile powders for injection, and concentrated solutions for injection. When the pharmaceutical composition is made into injection preparations, they may be produced by a conventional method in the current pharmaceutical field. In the case of preparation of injection preparations, it is not required to add additives, or appropriate additives may be added according to the nature of the drug. In the case of rectal administration, the pharmaceutical composition may be made into suppositories and the like. In the case of pulmonary administration, the pharmaceutical composition may be made into an inhalant or a spray. Preferably, the pharmaceutical composition of the present invention may be made into an injection preparation, such as an injection solution. Alternatively, normal saline is used as the carrier of the injection solutions.

In some embodiments, the composition further comprises an anticancer drug.

In some embodiments, the anticancer drug is polyethylene glycol conjugated drug of formula (A) or a pharmaceutically acceptable salt thereof,

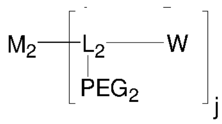

formula A

wherein:

$M_2$ is

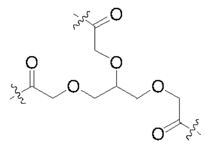 or 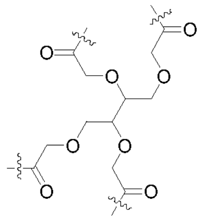 ;

j is 3 or 4;

$PEG_2$ is single-arm polyethylene glycol segment, $PEG_2$ is connected to $L_2$ through carbonyl group or $PEG_2$ has amino group or activated amino group at its terminal, $PEG_2$ has a number-average molecular weight of 5k-40k, preferably 5k-10k or 10k-40k, more preferably 5k;

$L_2$ is

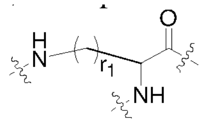 or 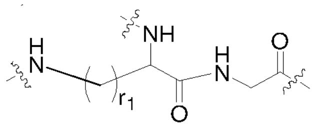 ,

each $r_1$ independently is 1, 2, 3, 4, 5 or 6, each $r_1$ independently is preferably 1, 2, 3 or 4, each $r_1$ independently is more preferably 3 or 4;

Wis

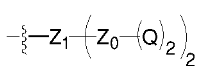 or 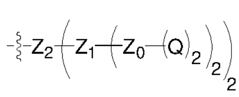;

$Z_2$, $Z_1$, $Z_0$ each independently are

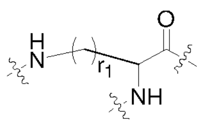 , 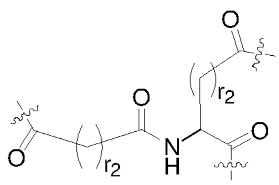

or

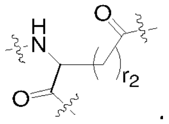 .

$r_1$ is 1 2, 3 4, 5 or 6, $r_1$ is preferably 1 2, 3 or 4, $r_1$ is more preferably 3 or 4, each $r_2$ independently is 1, 2, 3, 4, 5 or 6, each $r_2$ independently is preferably 1, 2, 3 or 4, each $r_2$ independently is more preferably 1 or 2,
Q is N-AC
N is

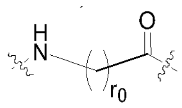

or G, $r_0$ is 1, 2, 3, 4, 5 or 6, preferably 3, 4, 5 or 6, more preferably 5 or 6;
AC is SB7, NPB, SN38, LPT, PCB, DOX, PTX or AXT, preferably PTX or SN38.

In some embodiments, $L_2$ is

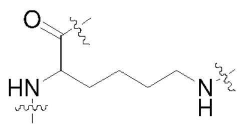 or 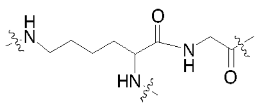 .

In some embodiments, $Z_2$, $Z_1$, $Z_0$ each independently are

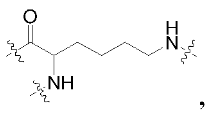 ,

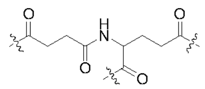 or 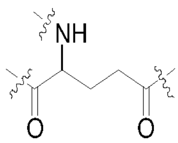 .

In some embodiments, $Z_2$ is

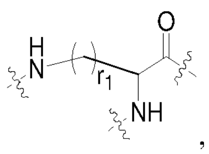 ,

, $r_1$ is 3 or 4.

In some embodiments, $Z_2$ is

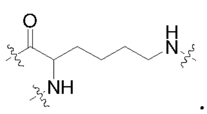 .

In some embodiments, $Z_1$ is

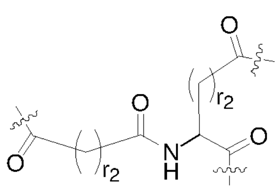 or 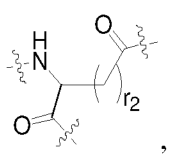 ,

each $r_2$ independently is 1 or 2.

In some embodiments $Z_1$ is

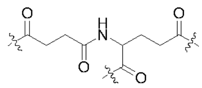 or 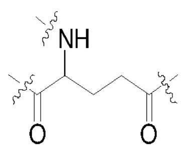 .

In some embodiments, $Z_0$ is

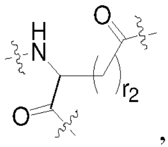 ,

$r_2$ is 1 or 2.

In some embodiments, $Z_0$ is

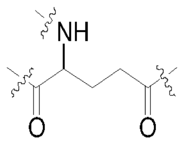 .

In some embodiments, N is

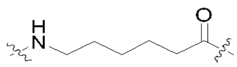

or G.

In some embodiments, the polyethylene glycol conjugated drug is selected from:

| No. | Structural formula |
|---|---|
| 49-166 | 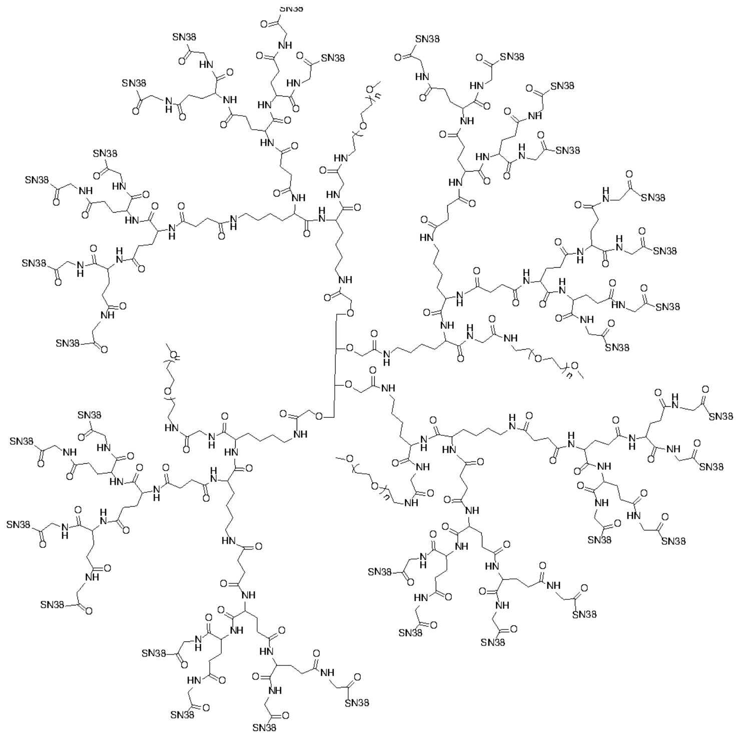<br>wherein, 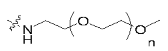 has a number-average molecular weight of 5k |

In the seventh aspect of the present invention, the present invention provides use of the above-mentioned polyethylene glycol conjugated drug synergist or a pharmaceutically acceptable salt thereof or the above-mentioned composition in the preparation of a medicament for enhancing the therapeutic efficacy of treating and/or preventing a disease.

In some embodiments, the disease is cancer, and the cancer is selected from: colon cancer, leukemia, lymphoma, bladder cancer, bone cancer, brain tumor, medulloblastoma, glioma, breast cancer, adenoma/carcinoid, adrenal cortical cancer, pancreatic islet cell cancer, cervical cancer, endometrial cancer, ovarian cancer, colorectal cancer, skin cancer, esophageal cancer, eye cancer, gallbladder cancer, stomach cancer, head and neck cancer, liver cancer, melanoma, Kaposi’s sarcoma, kidney cancer, oral cancer, lung cancer, nasopharyngeal cancer, neuroblastoma, ovarian cancer, pancreatic cancer, thyroid cancer, parathyroid penile cancer, prostate cancer, urethral cancer, vaginal cancer, vulvar cancer, anal cancer, sarcoma, including metastasis of the aforementioned cancers.

In the eighth aspect of the present invention, the present invention provides a method for enhancing the therapeutic efficacy of treating and/or preventing a disease, comprising administering an effective amount of the above-mentioned polyethylene glycol conjugated drug synergist or a pharmaceutically acceptable salt thereof or the above-mentioned composition to an individual in need thereof.

In some embodiments, the disease is cancer, and the cancer is selected from: colon cancer, leukemia, lymphoma, bladder cancer, bone cancer, brain tumor, medulloblastoma, glioma, breast cancer, adenoma/carcinoid, adrenal cortical cancer, pancreatic islet cell cancer, cervical cancer, endometrial cancer, ovarian cancer, colorectal cancer, skin cancer, esophageal cancer, eye cancer, gallbladder cancer, stomach cancer, head and neck cancer, liver cancer, melanoma, Kaposi’s sarcoma, kidney cancer, oral cancer, lung cancer, nasopharyngeal cancer, neuroblastoma, ovarian cancer, pancreatic cancer, thyroid cancer, parathyroid penile cancer, prostate cancer, urethral cancer, vaginal cancer, vulvar cancer, anal cancer, sarcoma, including metastasis of the aforementioned cancers.

In the ninth aspect of the present invention, the present invention provides the above-mentioned polyethylene glycol conjugated drug synergist or a pharmaceutically acceptable salt thereof or the above-mentioned composition for use in enhancing the therapeutic efficacy of treating and/or preventing a disease.

In some embodiments, the disease is cancer, and the cancer is selected from: colon cancer, leukemia, lymphoma, bladder cancer, bone cancer, brain tumor, medulloblastoma, glioma, breast cancer, adenoma/carcinoid, adrenal cortical cancer, pancreatic islet cell cancer, cervical cancer, endometrial cancer, ovarian cancer, colorectal cancer, skin cancer, esophageal cancer, eye cancer, gallbladder cancer, stomach cancer, head and neck cancer, liver cancer,

melanoma, Kaposi's sarcoma, kidney cancer, oral cancer, lung cancer, nasopharyngeal cancer, neuroblastoma, ovarian cancer, pancreatic cancer, thyroid cancer, parathyroid penile cancer, prostate cancer, urethral cancer, vaginal cancer, vulvar cancer, anal cancer, sarcoma, including metastasis of the aforementioned cancers.

In the tenth aspect of the present invention, the present invention provides use of the above-mentioned composition (including anticancer drug) in the preparation of a medicament for treating and/or preventing a disease, wherein, the disease refers to a disease treated by the above-mentioned anticancer drug.

In some embodiments, the disease is cancer, and the cancer is selected from: colon cancer, leukemia, lymphoma, bladder cancer, bone cancer, brain tumor, medulloblastoma, glioma, breast cancer, adenoma/carcinoid, adrenal cortical cancer, pancreatic islet cell cancer, cervical cancer, endometrial cancer, ovarian cancer, colorectal cancer, skin cancer, esophageal cancer, eye cancer, gallbladder cancer, stomach cancer, head and neck cancer, liver cancer, melanoma, Kaposi's sarcoma, kidney cancer, oral cancer, lung cancer, nasopharyngeal cancer, neuroblastoma, ovarian cancer, pancreatic cancer, thyroid cancer, parathyroid penile cancer, prostate cancer, urethral cancer, vaginal cancer, vulvar cancer, anal cancer, sarcoma, including metastasis of the aforementioned cancers.

In the eleventh aspect of the present invention, the present invention provides a method for treating and/or preventing a disease, comprising administering an effective amount of the above-mentioned composition (including anticancer drug) to an individual in need thereof, wherein, the disease refers to a disease treated by the above-mentioned anticancer drug. The dosage regimen may be adjusted to provide the optimum desired response. For example, a single amount of drug may be administered, several divided doses may be administered over time, or the dose may be proportionally reduced or increased as indicated by the urgent need for the treatment. It should be noted that the dose value may vary with the type and severity of the condition to be alleviated, and may include single or multiple doses. It should be further understood that for any particular individual, the specific dosage regimen should be adjusted over time according to the individual's needs and the professional judgment of the person administering the composition or supervising the administration of the composition.

In some embodiments, the disease is cancer, and the cancer is selected from: colon cancer, leukemia, lymphoma, bladder cancer, bone cancer, brain tumor, medulloblastoma, glioma, breast cancer, adenoma/carcinoid, adrenal cortical cancer, pancreatic islet cell cancer, cervical cancer, endometrial cancer, ovarian cancer, colorectal cancer, skin cancer, esophageal cancer, eye cancer, gallbladder cancer, stomach cancer, head and neck cancer, liver cancer, melanoma, Kaposi's sarcoma, kidney cancer, oral cancer, lung cancer, nasopharyngeal cancer, neuroblastoma, ovarian cancer, pancreatic cancer, thyroid cancer, parathyroid penile cancer, prostate cancer, urethral cancer, vaginal cancer, vulvar cancer, anal cancer, sarcoma, including metastasis of the aforementioned cancers.

In the twelfth aspect of the present invention, the present invention provides the above-mentioned composition (including anticancer drug) for use in treating and/or preventing a disease, wherein, the disease refers to a disease treated by the above-mentioned anticancer drug.

In some embodiments, the disease is cancer, and the cancer is selected from: colon cancer, leukemia, lymphoma, bladder cancer, bone cancer, brain tumor, medulloblastoma, glioma, breast cancer, adenoma/carcinoid, adrenal cortical cancer, pancreatic islet cell cancer, cervical cancer, endometrial cancer, ovarian cancer, colorectal cancer, skin cancer, esophageal cancer, eye cancer, gallbladder cancer, stomach cancer, head and neck cancer, liver cancer, melanoma, Kaposi's sarcoma, kidney cancer, oral cancer, lung cancer, nasopharyngeal cancer, neuroblastoma, ovarian cancer, pancreatic cancer, thyroid cancer, parathyroid penile cancer, prostate cancer, urethral cancer, vaginal cancer, vulvar cancer, anal cancer, sarcoma, including metastasis of the aforementioned cancers.

In the present invention, cancer refers to a disease state characterized by cell proliferative, including but not limited to: colon cancer, leukemia, lymphoma, bladder cancer, bone cancer, brain tumor, medulloblastoma, glioma, breast cancer, adenoma/carcinoid, adrenal cortical cancer, pancreatic islet cell cancer, cervical cancer, endometrial cancer, ovarian cancer, colorectal cancer, skin cancer, esophageal cancer, eye cancer, gallbladder cancer, stomach cancer, head and neck cancer, liver cancer, melanoma, Kaposi's sarcoma, kidney cancer, oral cancer, lung cancer, nasopharyngeal cancer, neuroblastoma, ovarian cancer, pancreatic cancer, thyroid cancer, parathyroid penile cancer, prostate cancer, urethral cancer, vaginal cancer, vulvar cancer, anal cancer, sarcoma, etc., including metastasis of the aforementioned cancers.

In the present invention, "individual" includes a human or a non-human animal. Exemplary human individuals include human individuals suffering from diseases such as those described herein (referred to as patients) or normal individuals. In the present invention, "non-human animals" include all vertebrates, such as non-mammals (such as birds, amphibians, and reptiles) and mammals, such as non-human primates, livestock and/or domesticated animals (such as sheep, dogs, cats, cows, pigs, and etc.).

As used herein, the term "effective amount" refers to the amount of a compound that will relieve one or more symptoms of the disease being treated to a certain extent after being administered.

As used herein, the term "treating" means reversing, alleviating, or inhibiting the disease or condition to which such term is applied or the progression of one or more symptoms of such a disease or condition, or preventing such a disease or condition or one or more symptoms of such a disease or condition.

In the polyethylene glycol conjugated drug synergist of the present invention, multiple PPT-iRGD or MI-AH-PPT-iRGD molecules are conjugated together by using an amino acid or a polypeptide as a linking chain, and a dicarboxylic acid or polycarboxylic acid with an amino group (for example, a natural amino acid with two carboxyl groups) or a carboxylic acid with two amino groups or multiple amino groups (for example, a natural amino acid with two amino groups) or a polycarboxylic acid as a linking bridge through the formation of an amide bond. In certain embodiments, activated PEG reacts with an amino group on the main chain through a carboxyl group to form an amide bond. In certain embodiments, PEG reacts with a carboxyl group on the main chain through a terminal amino group to form an amide bond. In certain embodiments, the molecular weight of the PEG comprises the terminal amino group thereof (i.e., the PEG derivative bearing reactive group).

In the polyethylene glycol conjugated drug of the present invention, multiple identical or different drug molecules are conjugated together by using an amino acid or a polypeptide as a linking chain, and a dicarboxylic acid or polycarboxylic acid with an amino group (for example, a natural amino acid with two carboxyl groups) or a carboxylic acid with two amino groups or multiple amino groups (for example, a natural amino acid with two amino groups) or a polycarboxylic acid as a linking bridge through the formation of an amide bond. The type, ratio and drug loading of the drug can be adjusted. In certain embodiments, activated PEG reacts with an amino group on the main chain through a carboxyl group to form an amide bond. In certain embodiments, the molecular weight of the PEG comprises the terminal amino group thereof (i.e., the PEG derivative bearing reactive group).

In the polyethylene glycol conjugated drug of the present invention, the active ingredient suitable for being conjugated with polyethylene glycol may be a drug molecule with at least one amino group, hydroxyl group, carboxyl group or acyl group, for example, a drug molecule having anti-tumor activity with at least one amino group, hydroxyl group, carboxyl group or acyl group, such as SB7, NPB, SN38, LPT, PCB, DOX PTX or AXT, which represent the following meanings:

| Abbreviation | Name | CAS number or structural formula |
|---|---|---|
| LPT | Lapatinib | 231277-92-2 |
| PCB | Palbociclib | 571190-30-2 |
| SB7 | SB-743921 | 940929-33-9 |
| NPB | Niraparib (MK-4827) | 1038915-60-4 |
| SN38 | 7-ethyl-10-hydroxycamptothecin | 86639-52-3 |
| DOX | Adriamvcin | 23214-92-8 |
| PTX | Paclitaxel | 33069-62-4 |
| AXT | Axitinib | 319460-85-0 |

PPT-iRGD has the structural formula

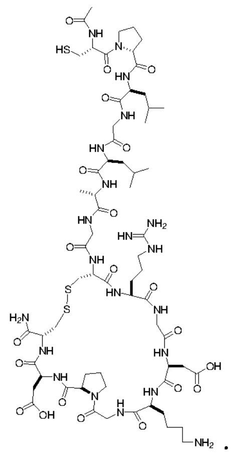.

The connection site of the PPT-iRGD to other part of the overall structure of the polyethylene glycol conjugated drug synergist is terminal sulfhydryl group by "~".

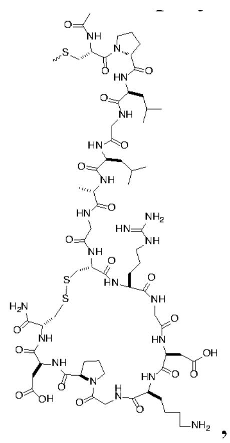

i.e., the position indicated

MI-AH-PPT-iRGD has the structural formula

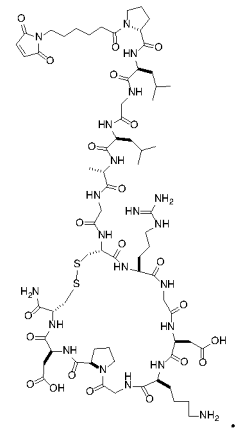

The connection site of the MI-AH-PPT-iRGD to other part of the overall structure of the polyethylene glycol conjugated drug synergist is maleimide group

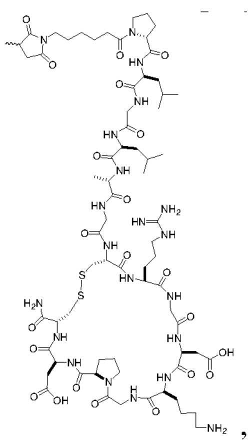

i.e., the position indicated by "〰". In some embodiments, the MI-AH-PPT-iRGD is subjected to addition connection on the maleimide group with other sulfydryl group.

In addition, the connection sites of the following drug molecules to other part of the overall structure of the polyethylene glycol conjugated drug are shown in the following table, i.e., the positions indicated by "〰".

| Abbreviation | Connection site |
|---|---|
| LPT | 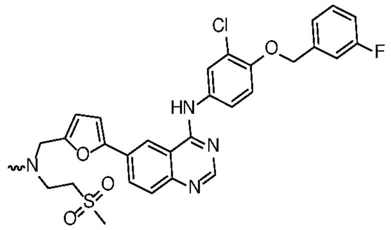 |
| PCB | 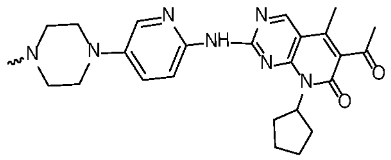 |
| SB7 | 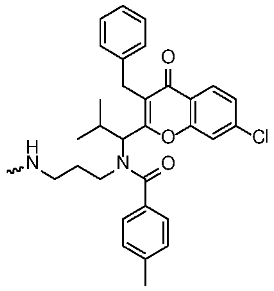 |
| NPB | 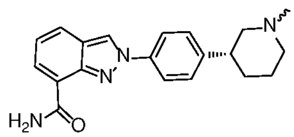 |
| SN38 | 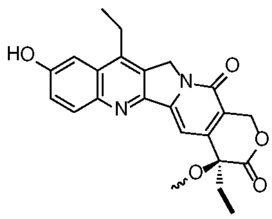 |
| DOX | 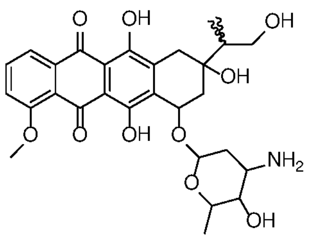 |
| PTX | 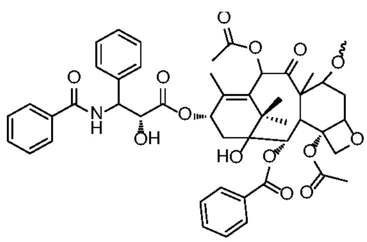 |

(continued)

| Abbreviation | Connection site |
|---|---|
| AXT | 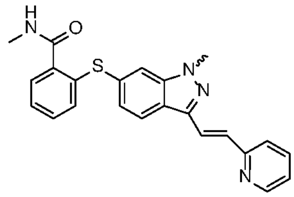 |

As used herein, "PEG" is an abbreviation for polyethylene glycol, which refers to a homopolymer with a repeating unit of $-CH_2CH_2O-$, including single-arm polyethylene glycol, multi-arm polyethylene glycol and their derivatives, such as a derivative with a reactive functional group such as amino or carboxyl group at the terminal. In the structural formula of the present invention, the letter "n" in the subscript of the repeating unit of polyethylene glycol represents the degree of polymerization of polyethylene glycol.

As used herein, unless it is clearly indicated in other ways, the expressions "each ... independently are/are selected from" and "... and ... each independently are/are selected from" used throughout this disclosure are interchangeable, and both should be understood in a broad sense. It can mean that the specific options expressed by the same symbol in different groups do not affect each other, or it can mean that the specific options expressed by the same symbol in the same group do not affect each other.

As used herein, the "pharmaceutically acceptable salt" of the compound of the present invention includes an acid addition salt and base addition salt of the compound, such as hydrochloride, hexafluorophosphate, and meglumine salt.

As used herein, the wavy line " in the structural formula means the position where another group is bonded to the structure represented by the structural formula.

## BRIEF DESCRIPTION OF THE DRAWINGS

FIG. 1 is a schematic diagram of the tumor growth trend of each group in the examples of the present invention.
FIG. 2 is a schematic diagram of the tumor weight inhibition rate of each group in the examples of the present invention.

## DETAILED DESCRIPTION

The embodiments of the present invention will be described in detail below in conjunction with examples. However, those skilled in the art will understand that the following examples are only used to illustrate the present invention, not to limit the scope of the present invention. Those without specific conditions among the examples are generally implemented under conventional conditions or under conditions recommended by the manufacturers. The reagents or instruments used without specifying the manufacturers are all conventional products that may be purchased commercially.

The meanings of abbreviations in the examples are as follows:

| | | | |
|---|---|---|---|
| G | Glycine residue | L | Leucine residue |
| F | Phenylalanine residue | Asp | Aspartic acid residue |
| E | Glutamate residue | Glu | Glutamate residue |
| DMF | N,N-dimethylformamide | TFA | Trifluoroacetic acid |
| t-Bu | Tert-butyl | Bn | Benzyl |
| Boc | Tert-butoxycarbonyl | Fmoc | Fluorenyl methoxycarbonyl |
| HOBT | 1-hydroxybenzotriazole | Ts | p-toluenesulfonyl |
| HBTU | o-Benzotriazole-N,N,N',N'-tetramethyl-uronium-hexafluorophosphate | LPT | Lapatinib |

(continued)

| | | | |
|---|---|---|---|
| DIEA | N,N-diiso-propylethylamine | SB7 | SB-743921 |
| EA | Ethyl acetate | PCB | Palbociclib |
| TMP | 2,4,6-trimethylpyridine | NPB | Niraparib |
| PyAOP | (3H-1,2,3-triazolo[4,5-b]pyridinato-O)tri-1-pyrrolidinylphosphonium hexafluorophosphate | | |
| LC | $NH_2$-$CH_2CH_2O$-$CH_2CH_2O$-$CH_2$-COOH or -NH-$CH_2CH_2O$-$CH_2CH_2O$-$CH_2$-CO- | | |

The source and structure of some raw materials are as follows:

M-$NH_2$-10K.HCl

JenKem, **mPEG**-$CH_2CH_2$-$NH_2$HCl

M-$NH_2$-5K.HC1

JenKem, **mPEG**-$CH_2CH_2$-$NH_2$HCl

M-SCM-5K

JenKem,

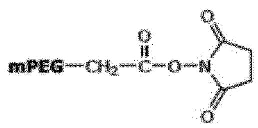

M-SCM-10K
JenKem,

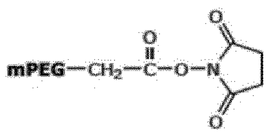

The present invention is further illustrated by the following examples.

**Example 1 Synthesis of compound**

Synthesis of 45-164:

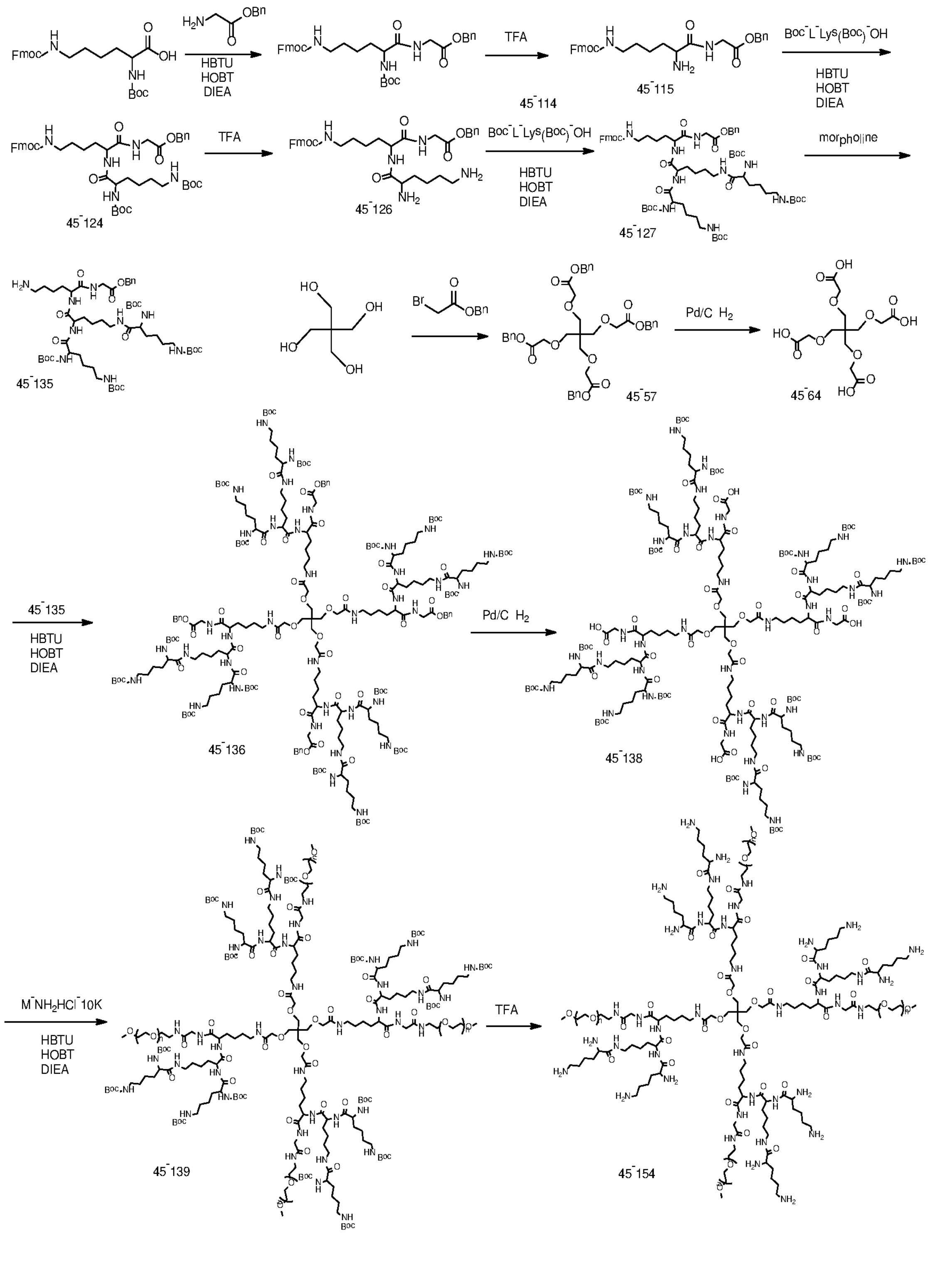
HBTU
HOBT
DIEA
TFA
45-114
45-115
Boc-L-Lys(Boc)-OH
HBTU
HOBT
DIEA
45-124
TFA
45-126
Boc-L-Lys(Boc)-OH
HBTU
HOBT
DIEA
45-127
morpholine
45-135
45-57
Pd/C $H_2$
45-64
45-135
HBTU
HOBT
DIEA
45-136
Pd/C $H_2$
45-138
M-$NH_2$HCl-10K
HBTU
HOBT
DIEA
45-139
TFA
45-154

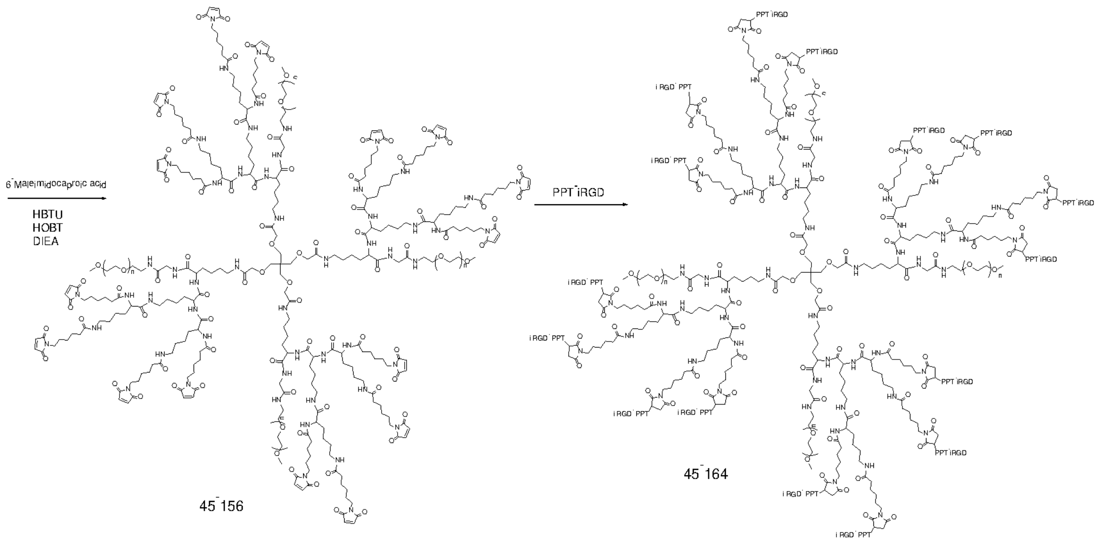


45-156

45-164

45-114

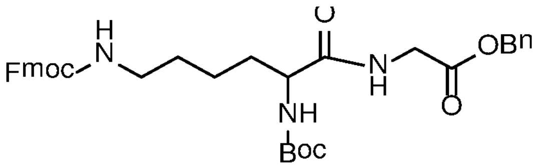

Boc-L-Lys(Fmoc)-OH (20g, 42.6857mmol, purchased from Aladdin), Gly-OBn (14.4g, 42.6857mmol, purchased from innochem), HBTU (24g, 64.0286mmol), HOBT (8.65g, 64.0286mmol) were added in a 500 mL flask, and dissolved with DMF (40 mL), and the mixed solution was stirred at -5°C for 30 minutes. DIEA (31.7mL, 192.085mmol) was slowly added dropwise, and then the mixed solution reacted at -5°C overnight. At the end of the reaction, the reaction solution was extracted with pure water and ethyl acetate. The organic phase was washed with saturated saline solution, concentrated and evaporated to dryness, thus obtaining the product 26 g.

45-115

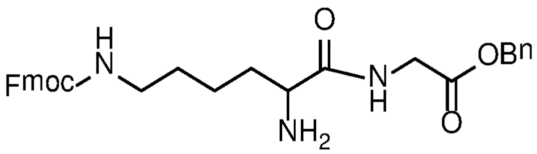

Compound 45-114 (26g, 42.6857mmol) was dissolved with dichloromethane (20mL) and TFA (14mL, 190.2855mmol) in a condition of ultrasonic, and then the mixed solution was stirred to react. At the end of the reaction, the reaction solution was concentrated. The concentrated product was dissolved with ethyl acetate (100 mL), saturated sodium chloride solution was added, the organic phase was separated, and the aqueous phase was extracted three times with ethyl acetate (50mL×3) until there was no product in the aqueous phase. The obtained organic phases were combined, then washed once with saturated saline solution (50mL), and evaporated to dryness, thus obtaining the product 22g.

45-124

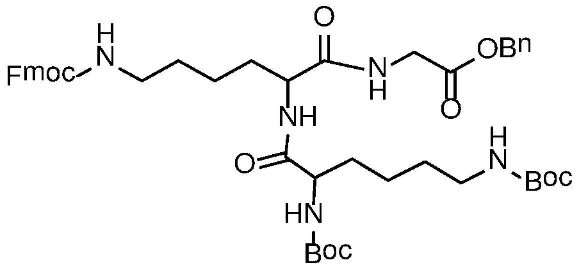

Boc-L-Lys(Boc)-OH (7.3g, 21.334mmol, purchased from Aladdin), 45-115 (11g, 21.334mmol), HBTU (12.1363g, 32.0016mmol), HOBT (4.3241g, 32.0016mmol) were added in a 500 mL flask, and dissolved with DMF (80 mL), and the mixed solution was stirred at -5°C for 30 minutes. DIEA (15mL, 96.003mmol) was slowly added dropwise, and then the mixed solution reacted at -5°C overnight. At the end of the reaction, the reaction solution was extracted with pure water and ethyl acetate. The organic phase was washed with saturated saline solution, and concentrated. The operations of dry sample loading, column chromatography, and gradient elution with 50%-100% ethyl acetate/petroleum ether were carried out, thus obtaining the product 15 g, yield 83%.

45-126

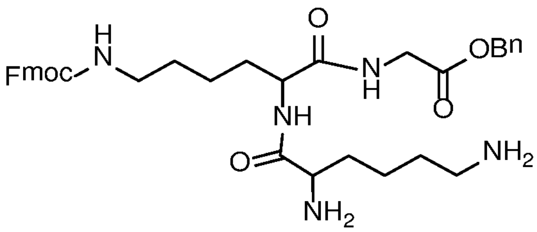

Compound 45-124 (15g, 17.7725mmol) was dissolved with dichloromethane (30mL) and TFA (39mL, 533.1754mmol) in a condition of ultrasonic, and then the mixed solution was stirred to react. At the end of the reaction, the reaction solution was concentrated. The concentrated product was dissolved with ethyl acetate (100 mL), and a saturated sodium bicarbonate solution was added until the aqueous phase became alkaline. Then, the organic phase was separated, and the aqueous phase was extracted three times with ethyl acetate (150mL×3) until there was no product in the aqueous phase. The obtained organic phases were combined, then washed once with saturated saline solution (150mL), and evaporated to dryness, thus obtaining the product 11.44g.

45-127

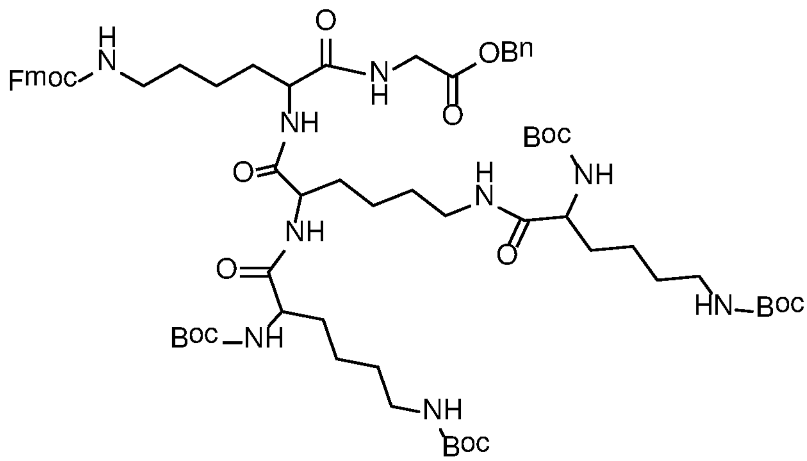

Boc-L-Lys(Boc)-OH (13.022g, 37.5911mmol), 45-126 (11.4g, 17.0868mmol), HBTU (19.5538g, 51.5604mmol), HOBT (6.9668g, 51.5604mmol) were added in a 500 mL flask, and dissolved with DMF (80 mL), and the mixed solution was stirred at -5°C for 30 minutes. DIEA (25.4mL, 153.7812mmol) was slowly added dropwise, and then the mixed solution reacted at -5°C overnight. At the end of the reaction, the reaction solution was extracted with pure water and ethyl acetate. The organic phase was washed with saturated saline solution, and concentrated. The

operations of dry sample loading, column chromatography, and gradient elution with 1%-55% methanol/ dichloromethane were carried out, thus obtaining the product 18 g, yield 81%.

45-135

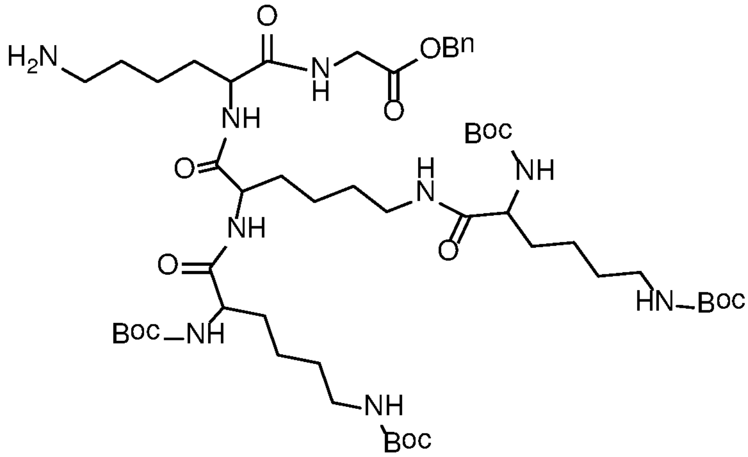

Reactant 45-127 (1.1g, 0.8458mmol) was added in a 250 mL flask, and dissolved with DMF (50mL), morpholine (1.4745mL, 16.9155mmoL) was added, and then the mixed solution was stirred to react at room temperature for 3 hours. At the end of the reaction, saturated saline solution (150mL) and ethyl acetate (200mL) were added to the reaction solution, and the organic phase was separated. The aqueous phase was extracted three times with ethyl acetate (150mL×3) until there was no product in the aqueous phase. The obtained organic phases were combined, washed two times with saturated saline solution (150mL×2), and evaporated to dryness, thus obtaining the product 0.912 g.

45-57

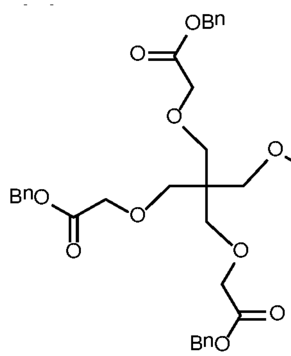

Pentaerythritol (10g, 73.4484mmol) was added in a 500 mL two-neck flask, and dissolved with THF (20mL) in a condition of ultrasonic, and then the obtained solution was stirred at 0°C. After introducing nitrogen for protective purpose, potassium tert-butoxide (352mL, 352.55mmol), and the obtained solution was stirred at 0°C for 2 hours. Then, benzyl bromoacetate (55mL, 352.55mmol) was added, and the obtained solution was stirred for 3 hours and then reacted at room temperature. At the end of the reaction, the reaction solution was extracted with pure water and ethyl acetate, and the organic phase was concentrated. The operations of dry sample loading, column chromatography and gradient elution with 1%-2% ethyl acetate/petroleum ether were carried out, thus obtaining the product 15g, yield 28%.

45-64

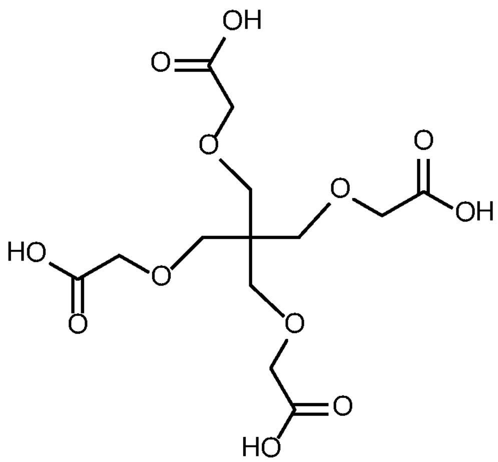

45-57 (0.76g, 1.0538mmol) and 10%Pd/C (100mg) were added in a hydrogenation reactor, DMF (30mL) was slowly added to dissolve the reactant with stirring, hydrogen was introduced to a pressure of 300 psi, and then the mixed solution was stirred to react at room temperature overnight. Next day, the reaction solution was filtered by suction through a sand core funnel filled with diatomaceous earth to remove the Pd/C, thus obtaining the DMF solution of 45-64, directly used for next reaction.

45-136

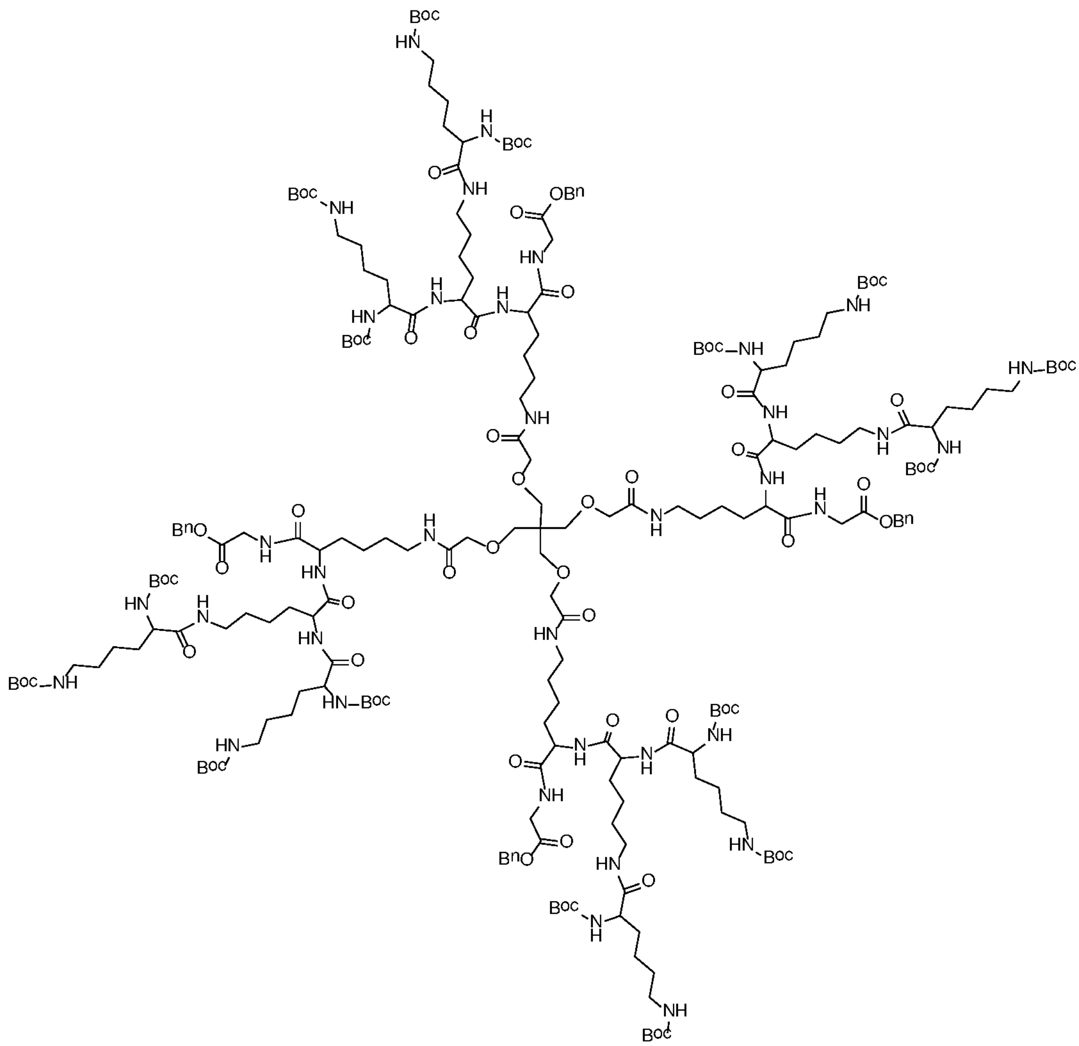

45-135 (0.9121g, 0.8458mmol), HBTU (0.4009g, 1.0572mmol), HOBT (0.1388g, 10572mmol) were added in a 250 mL flask, and dissolved with the DMF solution of 45-64 (0.1762mmol), and the mixed solution was stirred at -5°C for 30 minutes. DIEA (0.5242mL, 3.1716mmol) was slowly added dropwise, and then the mixed solution reacted at -5°C overnight. At the end of the reaction, pure water was added to the reaction solution, and suction filtering was carried out. The operations of dry sample loading, column chromatography and elution with 1%-5% methanol/dichloromethane were carried out, thus obtaining the product 0.4g, yield 50%.

$^1$H-NMR (600 MHz, DMSO-$d_6$) δ 8.37-8.33 (m, 2H), 7.98 - 7.81 (m, 13H), 7.72-7.70 (m, 9H), 7.63-7.62 (m, 1H), 7.40-7.30 (m, 24H), 6.89-6.86 (m, 2H), 6.74-6.69 (m, 6H), 5.11-5.10 (m, 4H), 4.55-4.54 (m, 3H), 4.24-4.16 (m, 6H), 3.99 - 3.78 (m, 12H), 3.65 -3.62(m, 6H), 3.37-3.33 (s, 48H), 3.08 - 2.82 (m, 42H), 2.71-2.68 (m, 25H), 2.57 -2.53(m, 10H), 1.68 - 1.14 (m, 161H).

45-138

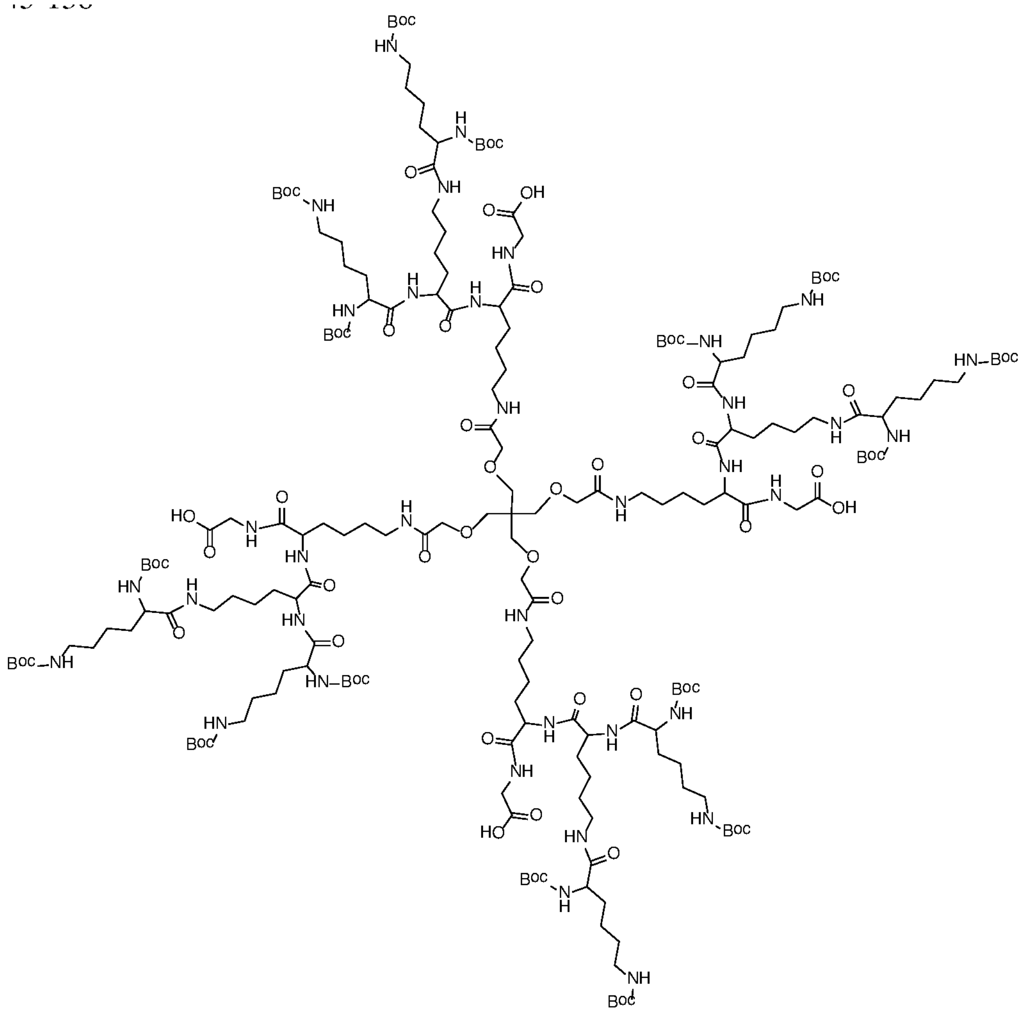

45-136 (0.35g, 0.0759mmol) and 10%Pd/C (75mg) were added in a hydrogenation reactor, DMF (50mL) was slowly added to dissolve the reactant with stirring, hydrogen was introduced to a pressure of 20 psi, and then the mixed solution was stirred to react at room temperature overnight. Next day, the reaction solution was filtered by suction through a sand core funnel filled with diatomaceous earth to remove the Pd/C, thus obtaining the DMF solution of the product, directly used for next reaction.

45-139

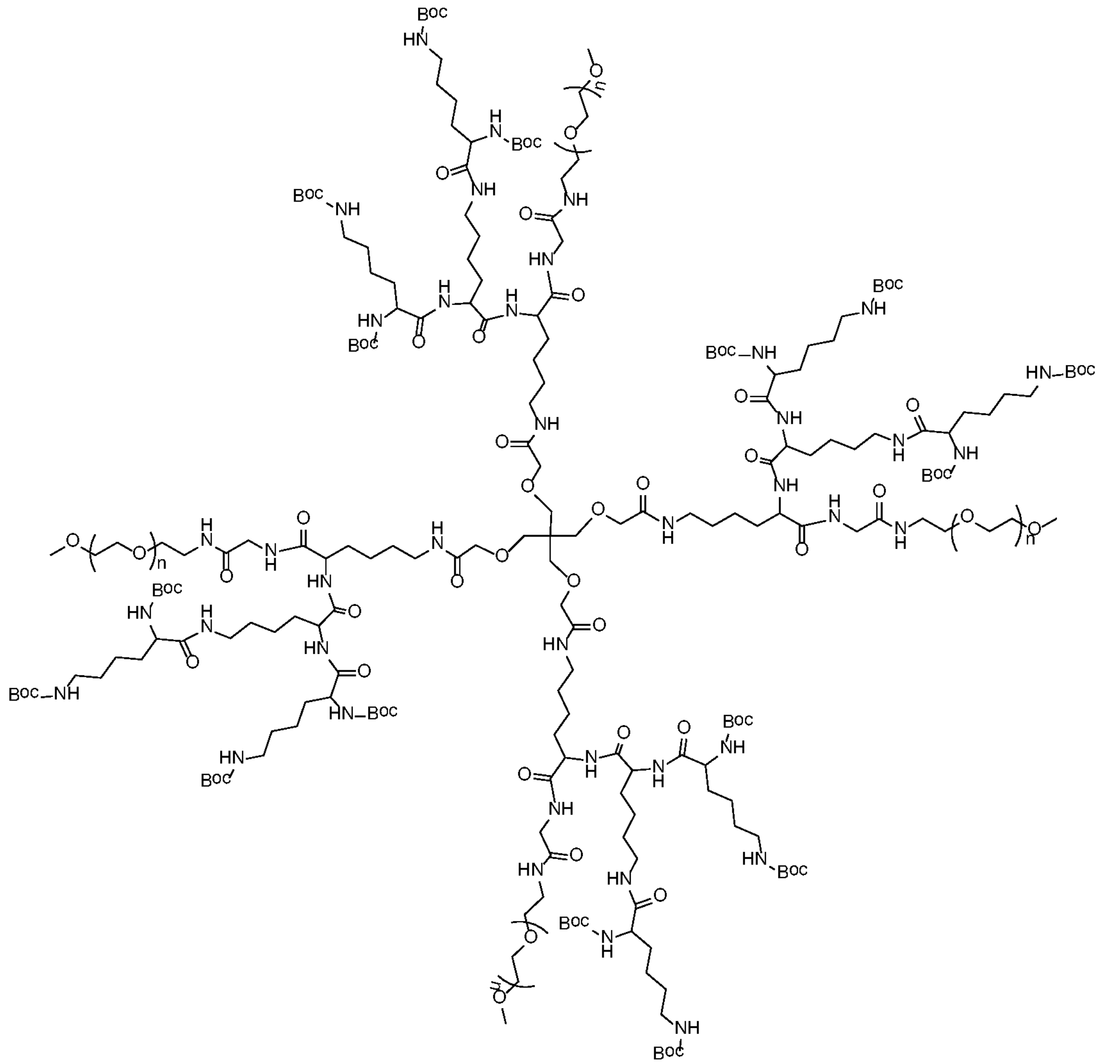

M-NH$_2$HCl-10K (3.3679g, 0.3188mmol, purchased from JenKem), HBTU (0.1727g, 0.4554mmol), HOBT (0.0615g, 0.4554mmol) were added in a 250 mL flask, and dissolved with the DMF solution of 45-138 (0.0667mmol), and the mixed solution was stirred at -5°C for 30 minutes. DIEA (0.1766mL, 1.3662mmol) was slowly added dropwise, and the mixed solution reacted under the condition of -5°C for 1 hour and then moved to room temperature and reacted in the dark at a low speed of stirring. At the end of the reaction, methyl tert-butyl ether was added to the reaction solution, and suction filtering was carried out. The operations of dry sample loading, column chromatography and elution with 5%-8% methanol/dichloromethane were carried out, thus obtaining the product 1.2g.

$^1$H-NMR (600 MHz, DMSO-$d_6$) $\delta$ 9.04-9.02 (m, 2H), 7.96-7.92 (m, 8H), 7.89-7.77 (m, 10H), 7.37-7.34 (m, 12H), 6.78-6.61 (m, 9H), 4.53-4.52 (m, 4H), 3.87-3.85 (m, 9H), 3.64 -3.60(m, 46H), 3.51-3.49(m, 3683H), 3.41-3.39 (m, 18H), 3.24-3.23 (m, 6H), 2.90-2.88 (m, 12H), 2.74-2.72 (m, 14H), 2.62-2.60 (m, 29H), 2.39 -2.37(m, 8H), 1.38 - 1.34 (m, 85H), 1.27 - 1.20 (m, 84H).

45-154

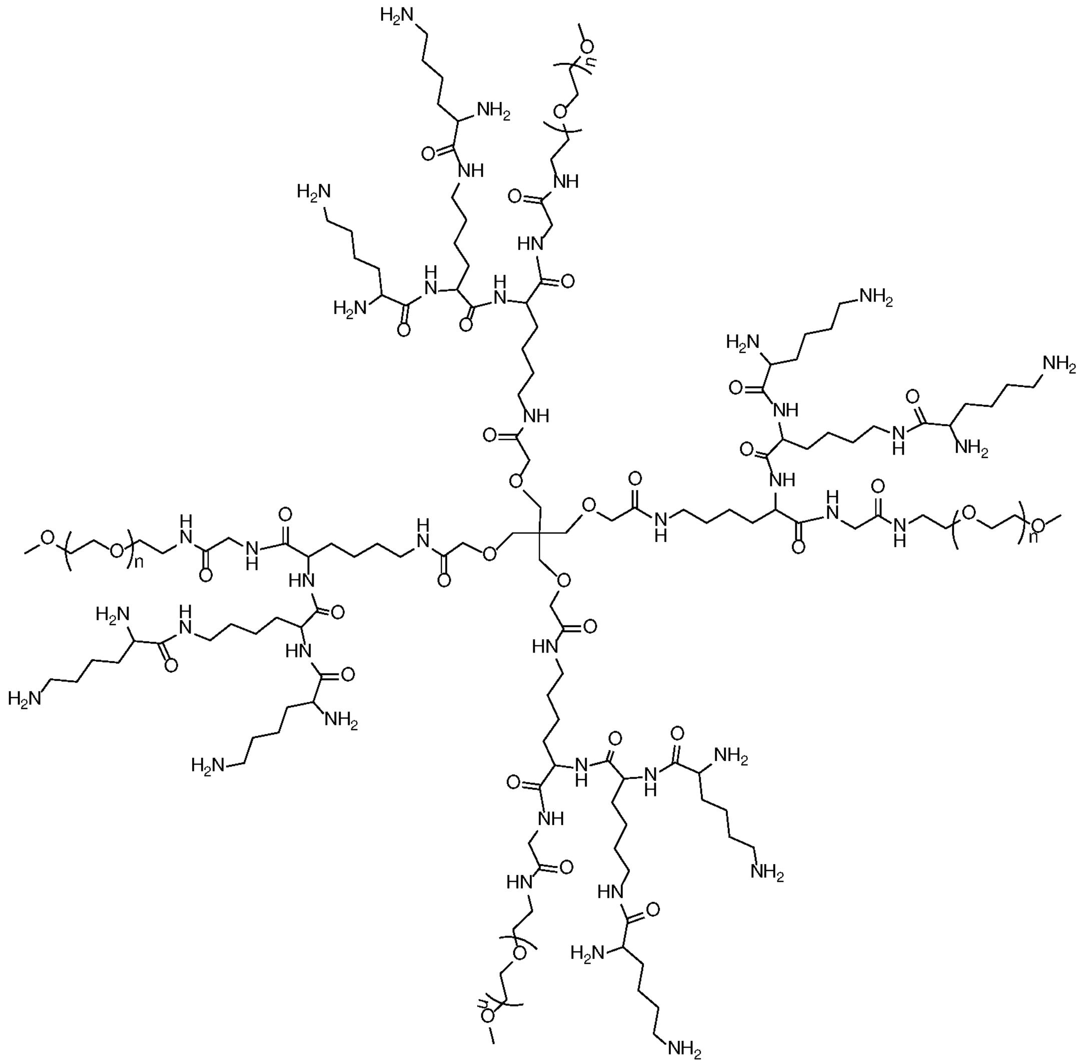

Compound 45-139 (0.4g, 0.0146mmol) was dissolved with dichloromethane (10mL) and TFA (0.25mL, 3.4983mol) in a condition of ultrasonic, and then the mixed solution was stirred to react. At the end of the reaction, methanol (30mL) and an excess amount of potassium carbonate were added to the reaction solution, the mixed solution was stirred for 30 minutes, and suction filtering was carried out. Silica gel powder was added to the filtrate, followed by evaporation to dryness. The operations of sample loading, column chromatography and gradient elution with 6%-50% methanol/dichloromethane were carried out, thus obtaining the product 0.2g, yield 54%.

45-156

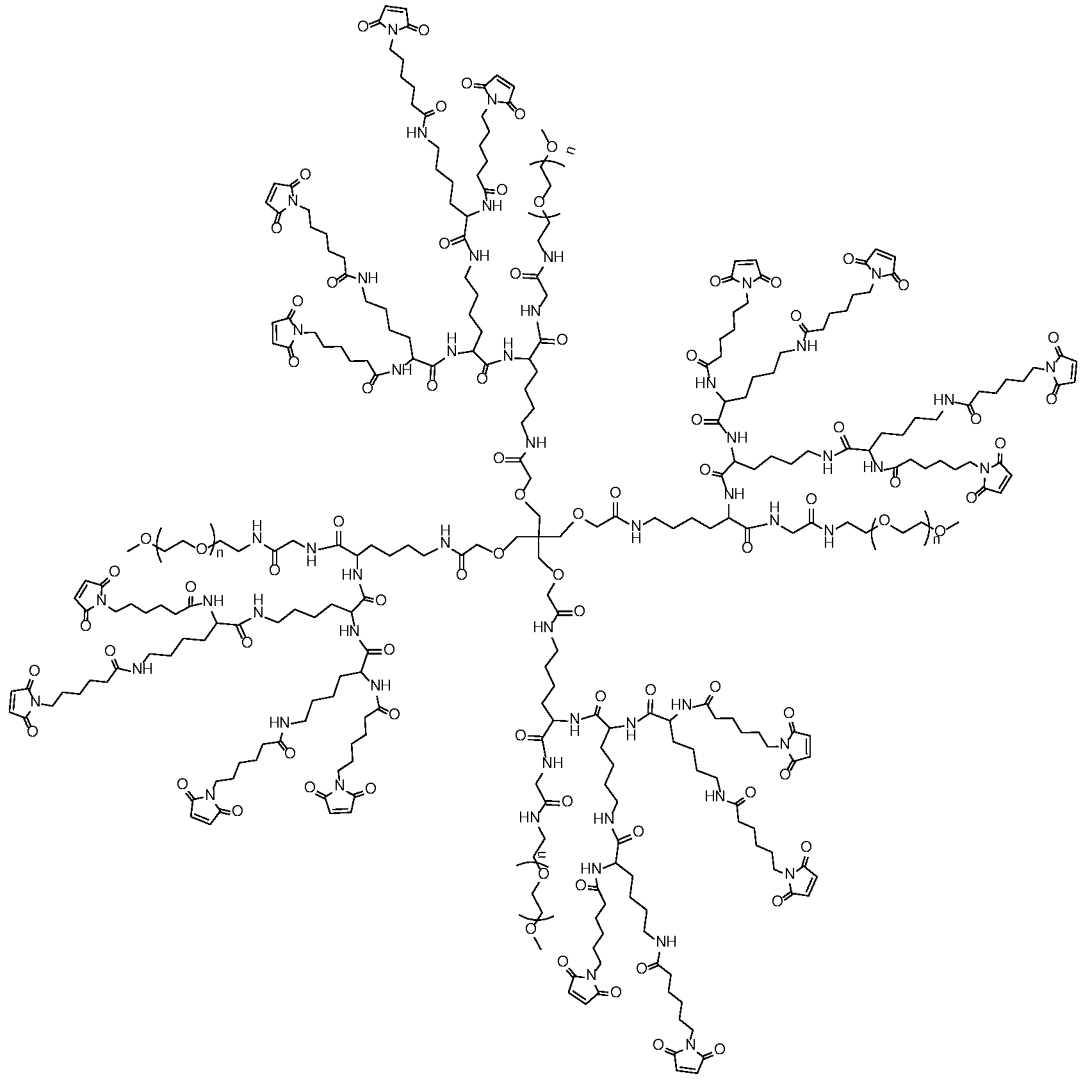

45-154 (0.2g, 0.0044mmol), 6-maleimidohexanoic acid (0.0189g, 0.0895mmol), HBTU (0.0400g, 0.1056mmol), HOBT (0.0143g, 0.1056mmol) were added in a 250 mL flask, and dissolved with DMF, and the obtained solution was stirred in the dark under the condition of -5°C for 30 minutes. Then DIEA (0.0523mL, 0.3168mmol) was slowly added dropwise, and the mixed solution reacted under the condition of -5°C for 1 hour and then moved to room temperature and reacted in the dark. At the end of the reaction, methyl tert-butyl ether was added to the reaction solution to make the product precipitated. The precipitated product was dissolved and precipitated several times to provide 0.1g of the product with a yield of 50%.

45-164

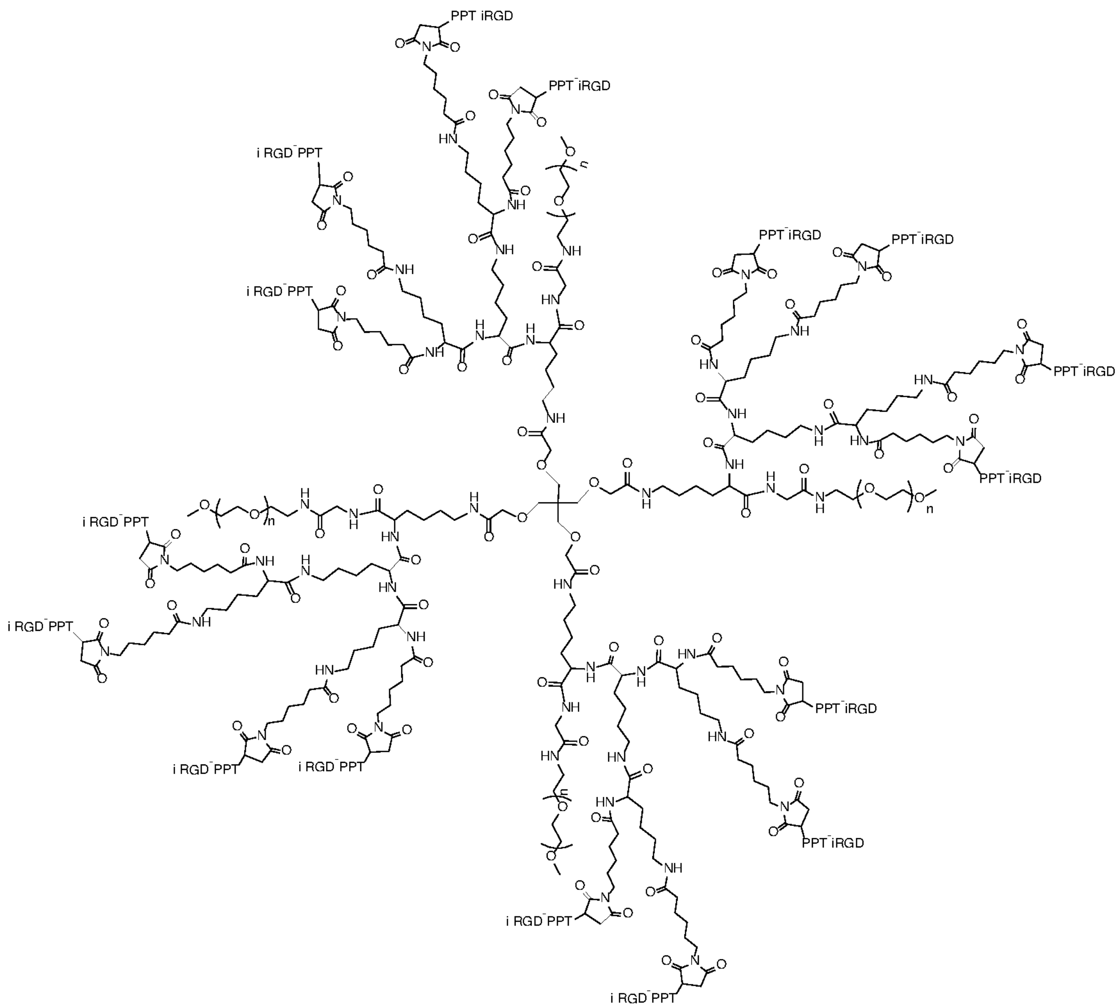

45-156 (0.4477g, 0.0094mmol), PPT-iRGD (0.3g, 0.1874mmol, purchased from Dangang Bio), were added in a 250 mL flask, and dissolved with DMSO (50mL), and the obtained solution was stirred in the dark under the condition of 40°C for 2 days, and then stirred at room temperature for 3 days. At the end of the reaction, methyl tert-butyl ether was added to the reaction solution, the upper layer solution was discarded, and the precipitated solid was dissolved with 10% methanol/dichloromethane, and then silica gel powder (5g) was added. The mixture was dried, loaded on silica gel column, and eluted with 5%-10% methanol/dichloromethane. 0.3 g of the product was obtained with a yield of 44%.

$^1$H-NMR (600 MHz, DMSO-$d_6$) δ 9.13-9.12 (m, 6H), 8.40-7.83 (m, 255H), 7.40-7.26 (m, 94H), 7.13-7.12(m, 6H), 7.01 - 6.88 (m, 3H), 6.70 -6.68(m, 4H), 4.54-4.52 (m, 52H), 4.26-4.24 (m, 83H), 3.78-3.76 (m, 131H), 3.51-3.49 (m, 4200H), 3.09-3.07 (m, 178H), 2.89-2.86 (m, 225H), 2.71-2.69 (m, 212H), 2.37-2.35 (m, 22H), 2.16-1.97 (m, 52H), 1.81-1.76 (m, 102H), 1.55-1.52 (m, 174H), 1.33-1.28 (m, 288H), 0.85-0.81 (m, 192H).

Synthesis of compound 49-166 (anticancer drug)

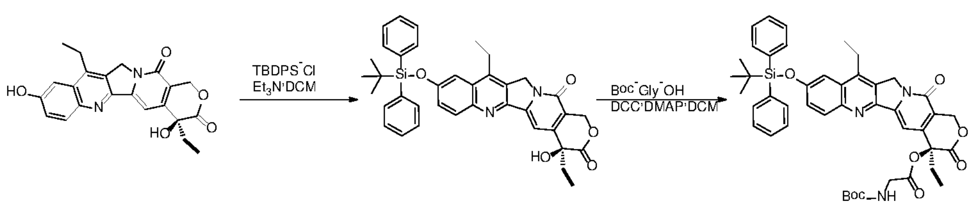


47-96

47-97

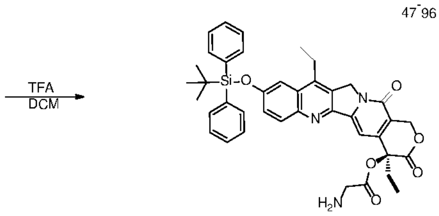


47-98

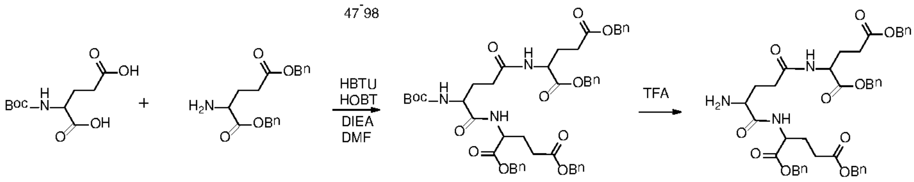


39-80

39-84

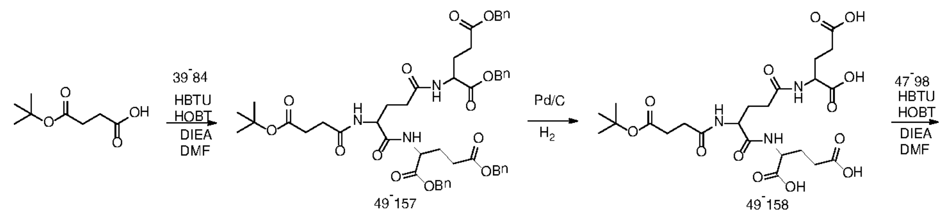


49-157

49-158

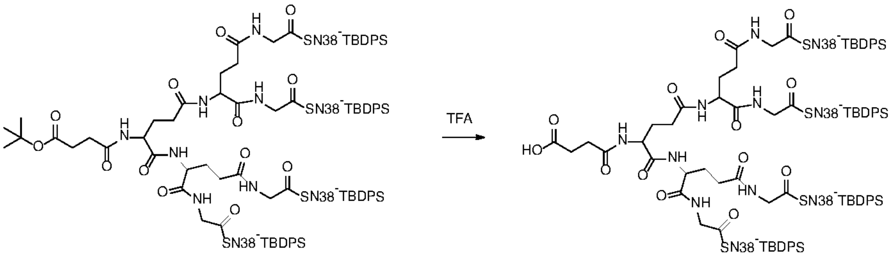


49-159

49-161

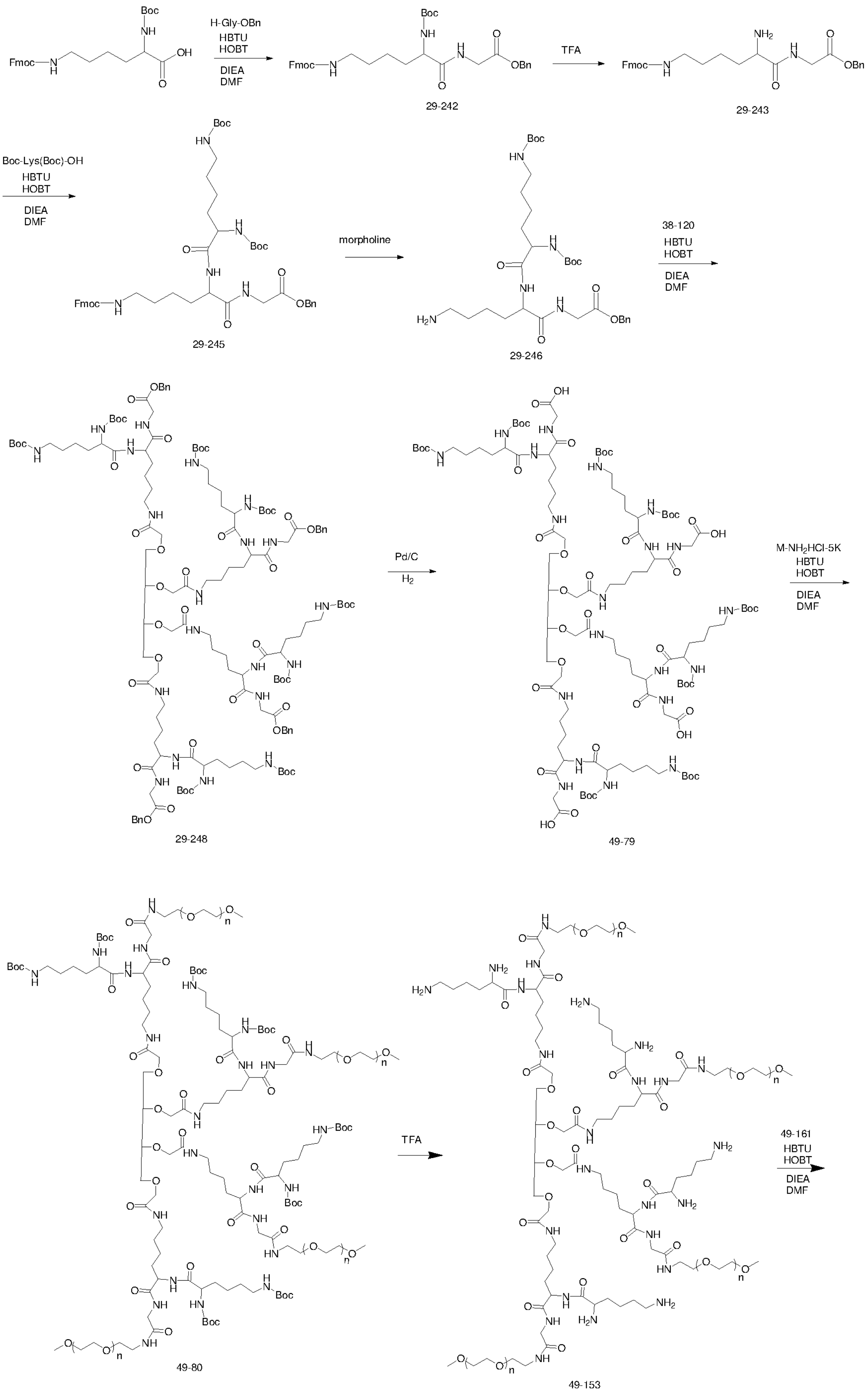


29-242

29-243

29-245

29-246

29-248

49-79

49-80

49-153

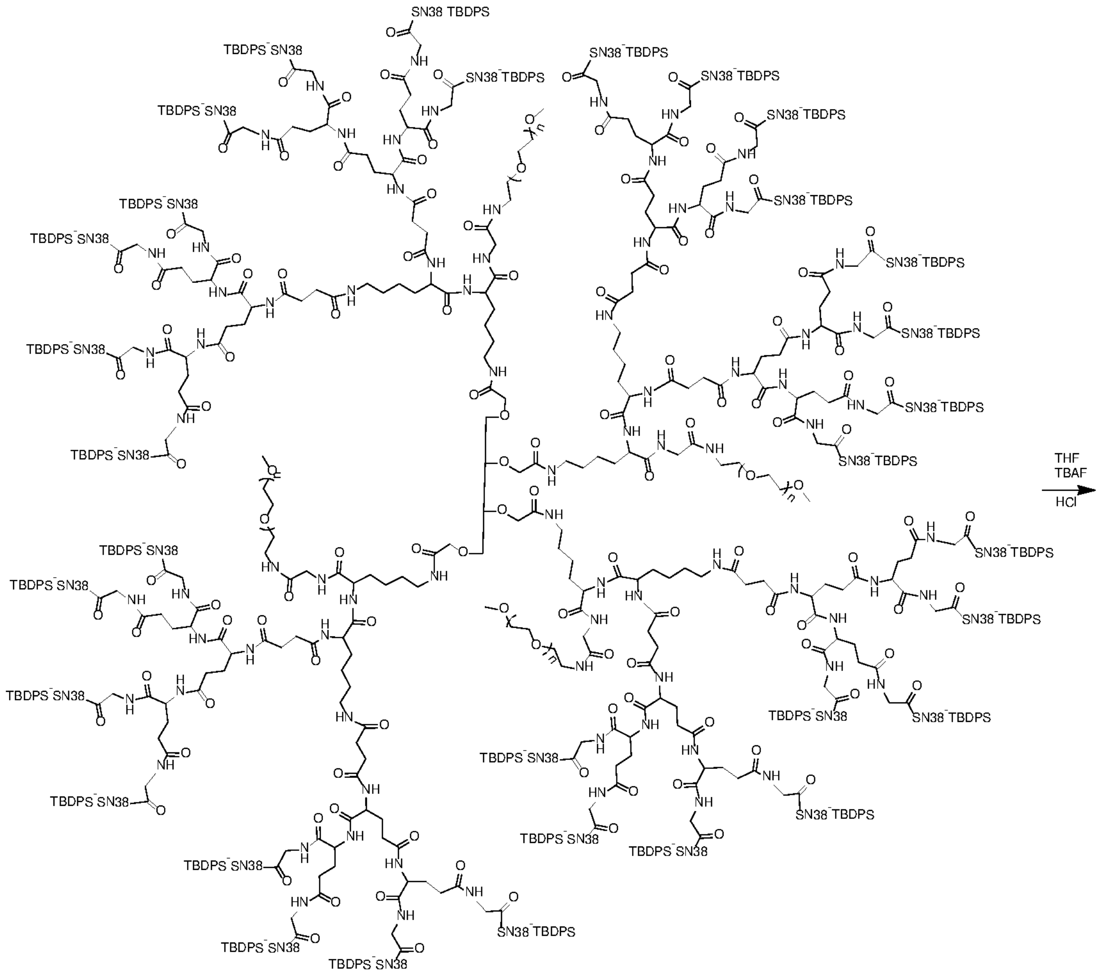


49-162

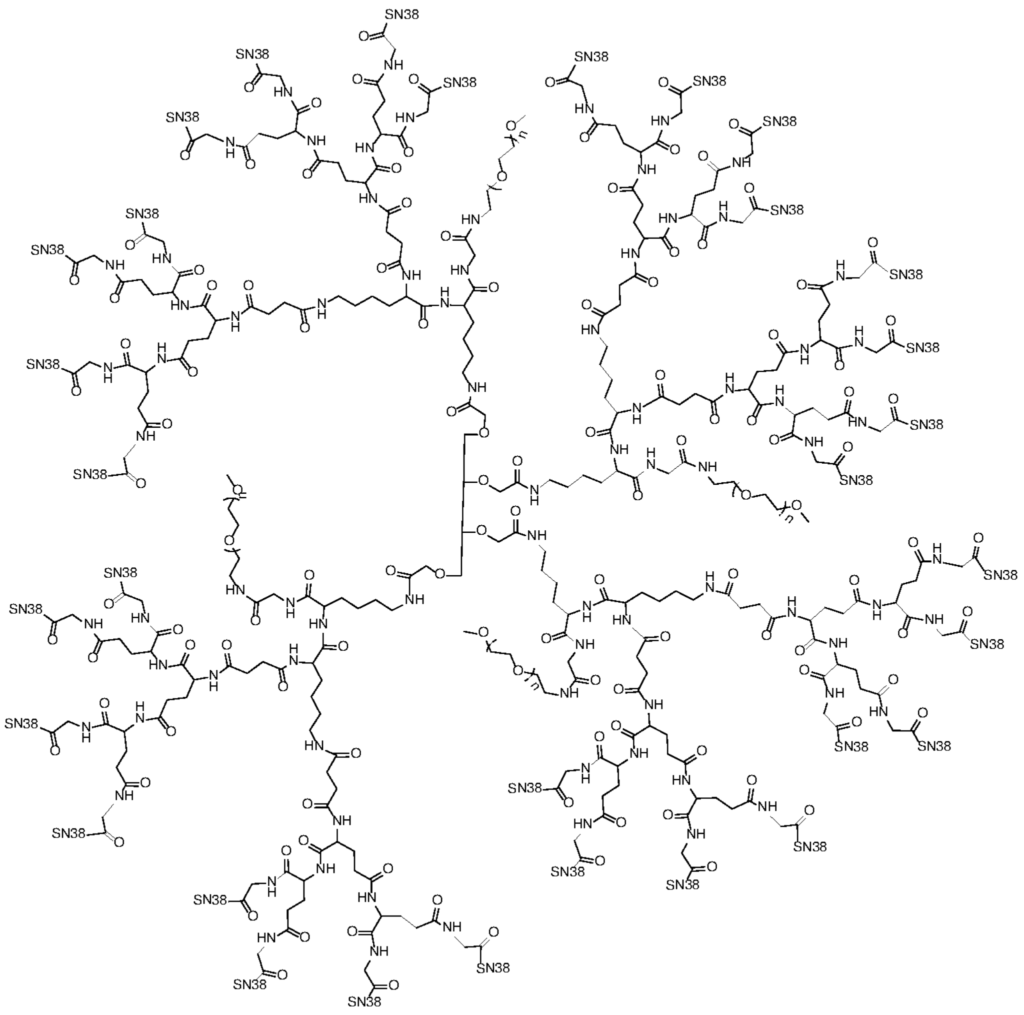

49-166

39-80

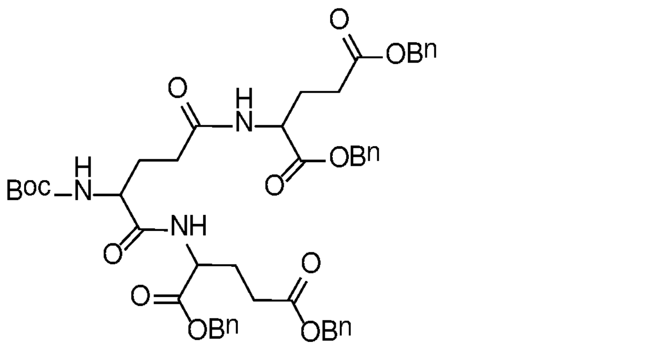

Boc-Glu-OH (5.0g, 20.22mmol, purchased from Aladdin), H-Glu (OBzl)-OBzl.TsOH (21.2g, 42.46mmol, purchased from Ark Pharm), HOBT (8g, 60.66mmol), HBTU (23g, 60.66mmol) were added in a 250 mL flask, and dissolved with DMF (80 mL), and ultrasonic treatment was carried out to completely dissolve the reactants, and then the obtained solution was stirred at -5°C for 30 minutes. Then DIEA (30mL, 181mmol) was slowly added dropwise, and the obtained solution reacted at the low temperature until the reaction ended. At the end of the reaction, deionized water (100 mL) was added to the reaction solution, the obtained solution was extracted three times with ethyl acetate (100 mL×3), and the obtained organic phases were combined. The organic phase was washed two times with saturated sodium chloride solution (100 mL), concentrated and evaporated to dryness. The operations of dry sample loading, column chromatography and gradient elution with 40% ethyl acetate/petroleum ether-50% ethyl acetate/petroleum ether were carried out. The elution product was then collected, concentrated, and evaporated to dryness.
39-84

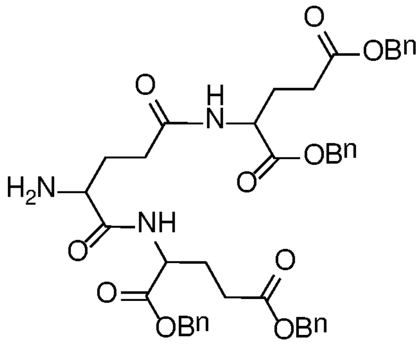

39-80 (19.2mmol) was dissolved with dichloromethane (5mL), TFA (14mL, 192mmol) was added, and ultrasonic treatment was carried out to completely dissolve the compound. A ground glass stopper was used, and the mixed solution was stirred to react at room temperature. At the end of the reaction, saturated sodium bicarbonate solution (300 mL) was added to the reaction solution, the obtained solution was extracted three times with ethyl acetate (100 mL×3), and the obtained organic phases were combined. The organic phase was washed two times with saturated sodium chloride solution (100 mL), concentrated and evaporated to dryness.
47-96

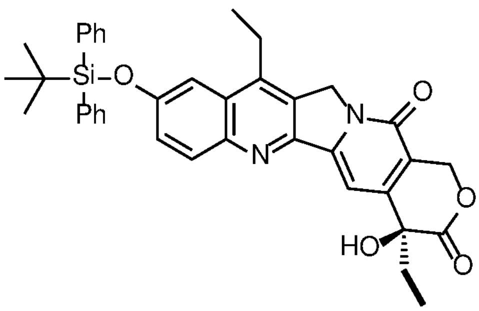

7-Ethyl-10-hydroxycamptothecin (15.00g, 38.23mmol, also referred to as SN38) was added in a 1000 mL round-bottomed flask, and dissolved with dichloromethane (150 mL), tert-butyl diphenylchlorosilane (59.64ml, 229.36mmol, purchased from Accela), triethylamine (31.88ml, 229.36mmol) were added, and then the obtained solution was placed in an oil bath at 37°C and stirred to react overnight. At the end of the reaction, the reaction solution was evaporated to obtain a viscous solution, the viscous solution was precipitated with n-hexane (150ml) to obtain a solid product, and suction filtering was carried out. The filter cake was dried, thus obtaining the product (23.15g, 96%).
47-97

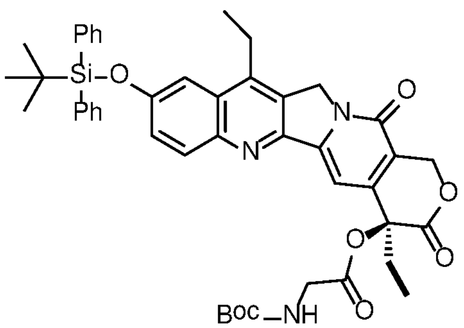

47-96 (23.15g, 36.70 mmol), Boc-Gly-OH (8.71g, 49.70 mmol, purchased from Aladdin), DMAP (0.94g, 7.65 mmol) were added in a 1000 mL round-bottomed flask, and dissolved with dichloromethane (150 mL), and then the mixed solution was stirred at 0°C for 30 minutes. Then, DCC (39.41 g, 191.15 mmol) was added in three batches, with an interval of 30 minutes each batch. At the end of the addition, the obtained solution reacted at 0°C for 2 hours, and

was then moved to room temperature and stirred to react overnight. At the end of the reaction, the reaction solution was precipitated with n-hexane (200 mL) and petroleum ether (50 mL). Such operations were repeated three times, and filtering was carried out to obtain a solid product. The solid product was dried in a vacuum oven, thus obtaining the product (27.53 g, 94%).

47-98

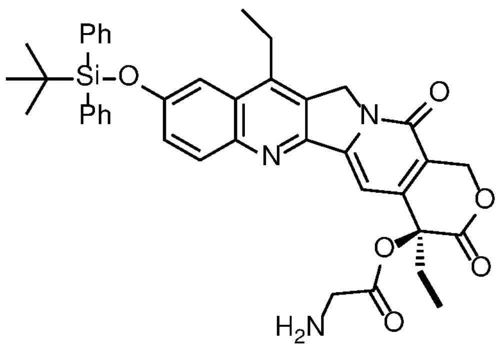

47-97 (27.53g, 34.50mmol) was added in a 1000 mL round-bottomed flask, and dissolved with dichloromethane (50 mL) and trifluoroacetic acid (28.40ml, 382.30mmol), and then the mixed solution reacted at room temperature. At the end of the reaction, deionized water (200 mL) was added to the reaction solution, the obtained solution was extracted with ethyl acetate (100 mL×3), and the obtained organic phases were combined. The organic phase was washed with saturated sodium chloride solution (200 mL×2), and concentrated, silica gel powder was added, and the obtained mixture was then evaporated to dryness to obtain a powdery product. The operations of column chromatography and gradient elution (with 1%-3% $CH_3OH$, the rest of $CH_2Cl_2$) were carried out. The elution product was then collected, and concentrated, thus obtaining the product (16.98g, 72%)

29-242

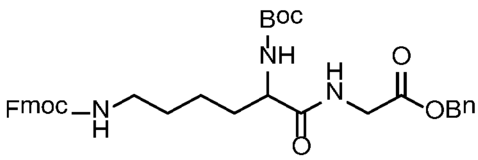

Fmoc-Lys (Boc) -OH (5.0g, 10.6714 mmol, purchased from Aladdin), H-Gly-OBn (3.7802g, 11.2050 mmol, purchased from Innochem), HBTU (6.0705g, 16.0072mmol), HOBT (2.1630g, 16.0072mmol) were added in a 500 mL flask, and dissolved with DMF (50 mL), and then the obtained solution was stirred to react at 0°C for 30 minutes. Then DIEA (7.9371mL, 48.0215mmol) was slowly added dropwise, the obtained solution continued to react at 0°C with stirring overnight. At the end of the reaction, deionized water (200 mL) was added to the reaction solution, the obtained solution was extracted with ethyl acetate (100 mL×3), and the obtained organic phases were combined. The organic phase was washed two times with saturated sodium chloride solution (200 mL), concentrated, evaporated to dryness, and dried in a vacuum oven, thus obtaining a crude product.

29-243

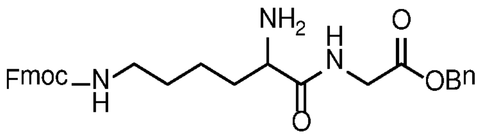

29-242 (6.57g, 10.6714 mmol) was added in a 500 mL flask, and dissolved with a proper amount of dichloromethane, TFA (7.9248mL, 106.714mmol) was added, and then the mixed solution was stirred to react at room temperature overnight. At the end of the reaction, the reaction solution was evaporated to obtain an oily solution, a saturated sodium bicarbonate solution was added to adjust pH until the solution became alkaline, the obtained solution was extracted with ethyl acetate (100 mL×3), and the obtained organic phases were combined. The organic phase was washed two times with saturated sodium chloride solution (200 mL), concentrated, evaporated to dryness, and dried in a vacuum oven, thus obtaining a crude product.

29-245

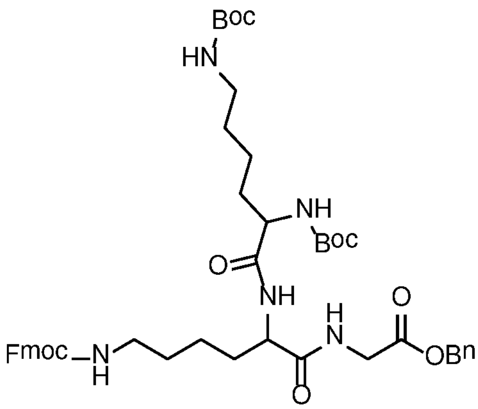

Boc-Lys (Boc) -OH (4.2805g, 11.7385 mmol, purchased from Ark Pharm), 29-243 (5.50g, 10.6714 mmol), HBTU (6.0705g, 16.0072mmol), HOBT (2.1630g, 16.0072mmol) were added in a 500 mL flask, and dissolved with DMF (50 mL), and then the mixed solution was stirred to react at 0°C for 30 min. Then DIEA (7.9371mL, 48.0215mmol) was slowly added dropwise, the obtained solution continued to react at 0°C overnight. At the end of the reaction, deionized water (200 mL) was added to the reaction solution, the obtained solution was extracted with ethyl acetate (100 mL×3), and the obtained organic phases were combined. The organic phase was washed two times with saturated sodium chloride solution (200 mL), concentrated, evaporated to dryness, and dried in a vacuum oven, thus obtaining a crude product.

29-246

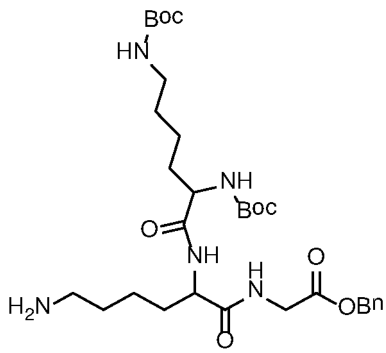

Morpholine (9.24mL, 106.714mmol) was added in a 500 ml flask loaded with 29-245 (9.0g, 10.6714mmol), and dissolved with DMF (10mL), and then the mixed solution was stirred to react at room temperature for 1 hour. At the end of the reaction, deionized water (200 mL) was added to the reaction solution, the obtained solution was extracted with ethyl acetate (100 mL×3), and the obtained organic phases were combined. The organic phase was washed two times with saturated sodium chloride solution (200 mL), and concentrated, silica gel powder was added, and the obtained mixture was then evaporated to dryness to obtain a powdery product. The operations of column chromatography and gradient elution (with 1% ammonia water, 4%-5% methanol, the rest of dichloromethane) were carried out. The elution product was then collected, concentrated, thus obtaining the product 29-246: 3.7g. Yield 56%.

29-248

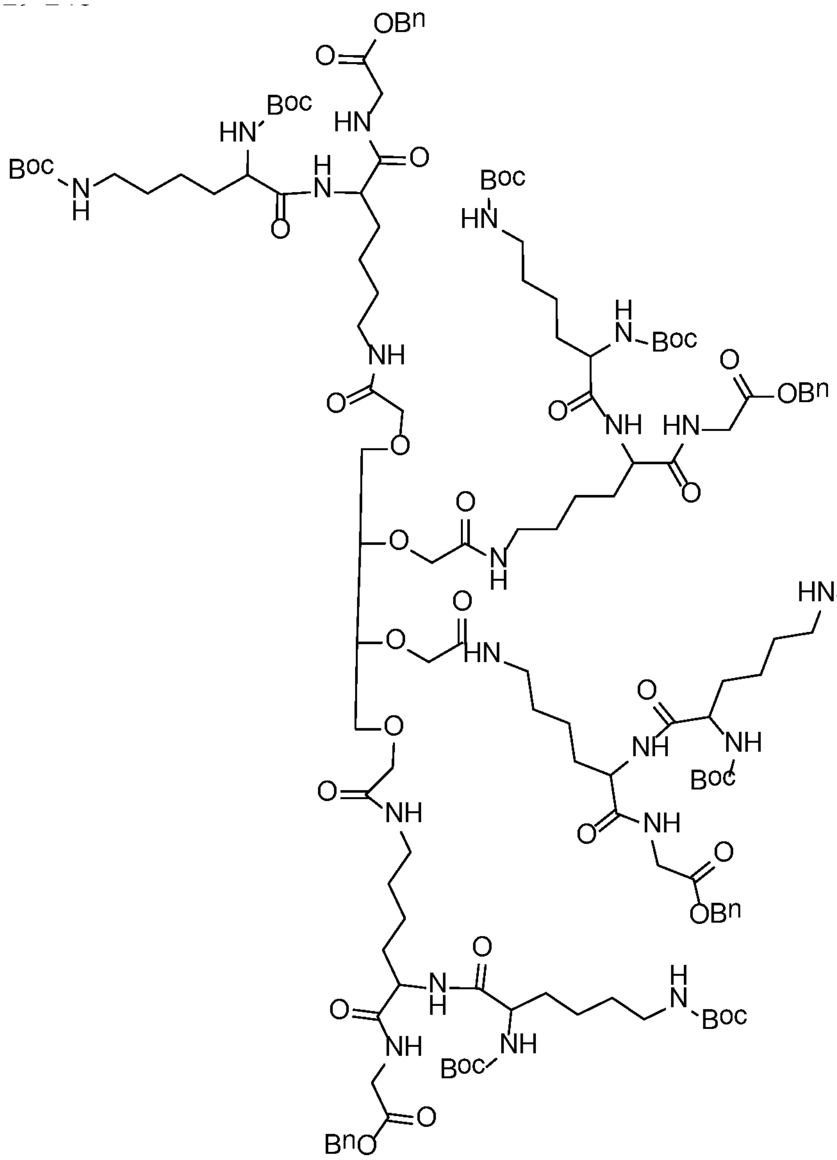

38-120 (0.39g, 1.0966 mmol), 29-246 (3.0g, 4.8249 mmol), HBTU (2.4951g, 6.5795mmol), HOBT (0.8891g, 6.5795 mmol) were added in a 500 mL flask, and dissolved with DMF (50 mL), and then the mixed solution was stirred to react at -5°C for about 30 minutes. Then DIEA (3.2624mL, 19.7384mmol) was slowly added dropwise, the obtained solution continued to react at -5°C with stirring for 1 hour, and was then moved to room temperature and stirred to react overnight. At the end of the reaction, deionized water (200 mL) was added to the reaction solution, the obtained solution was extracted with ethyl acetate (100 mL×3), and the obtained organic phases were combined. The organic phase was washed two times with saturated sodium chloride solution (200 mL), and concentrated, silica gel powder was added, and the obtained mixture was then evaporated to dryness to obtain a powdery product. The operations of column chromatography and gradient elution (with 50%-80% ethyl acetate, the rest of petroleum ether) were carried out. The elution product was then collected, concentrated, thus obtaining the product (1.6g, 53%).

49-79

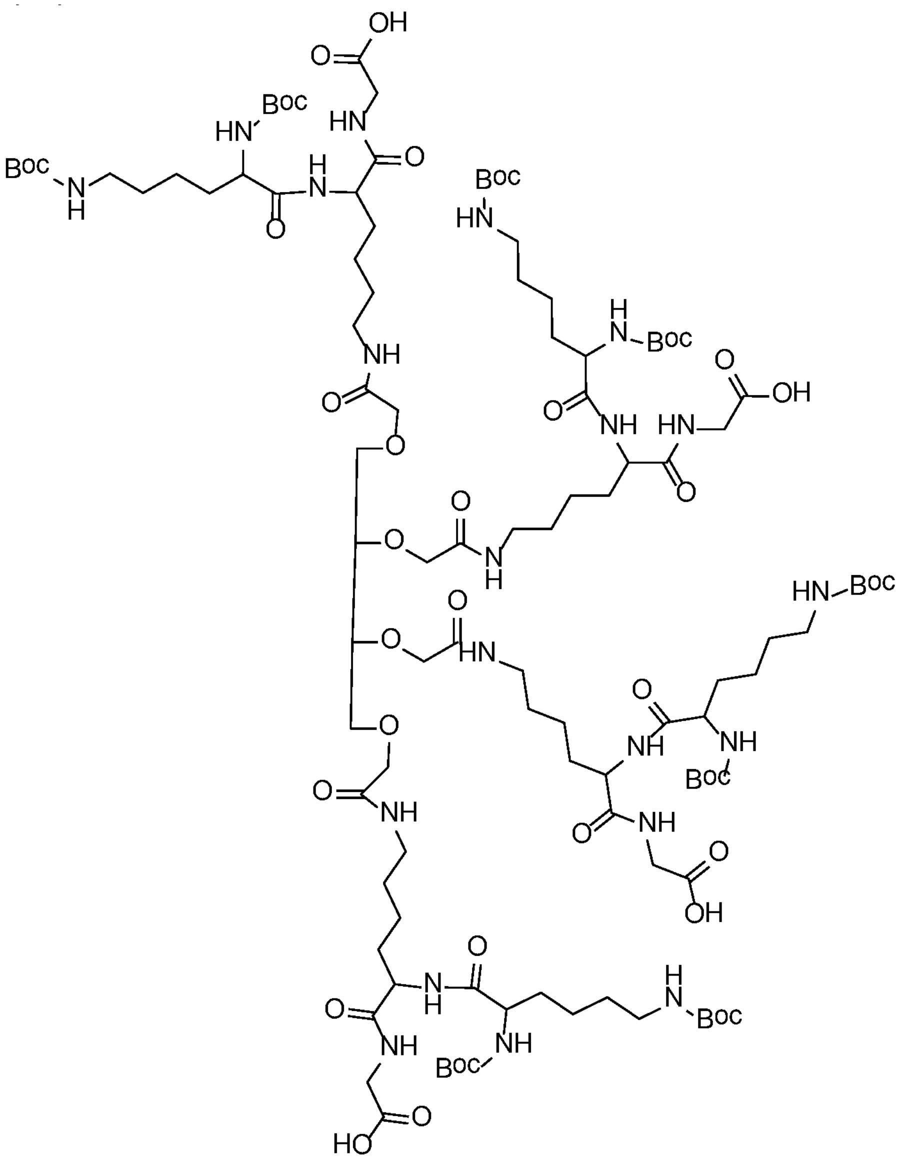

29-248 (0.52g, 0.1878mmol) and 10% Pd/C (0.0, 5 g) were added in a hydrogenation reactor, and dissolved with DMF (30 mL). The hydrogenation reactor was then sealed to perform the "three pumping and three charging" operation so that the pressure on the hydrogenation reactor was read as 0.18 MPa, and then the obtained solution reacted at room temperature overnight. At the end of the reaction, the reaction solution was filtered with diatomaceous earth. The filter cake was washed with DMF (20 mLx3), thus obtaining the DMF solution of the product, as the raw material for the next reaction.

49-80

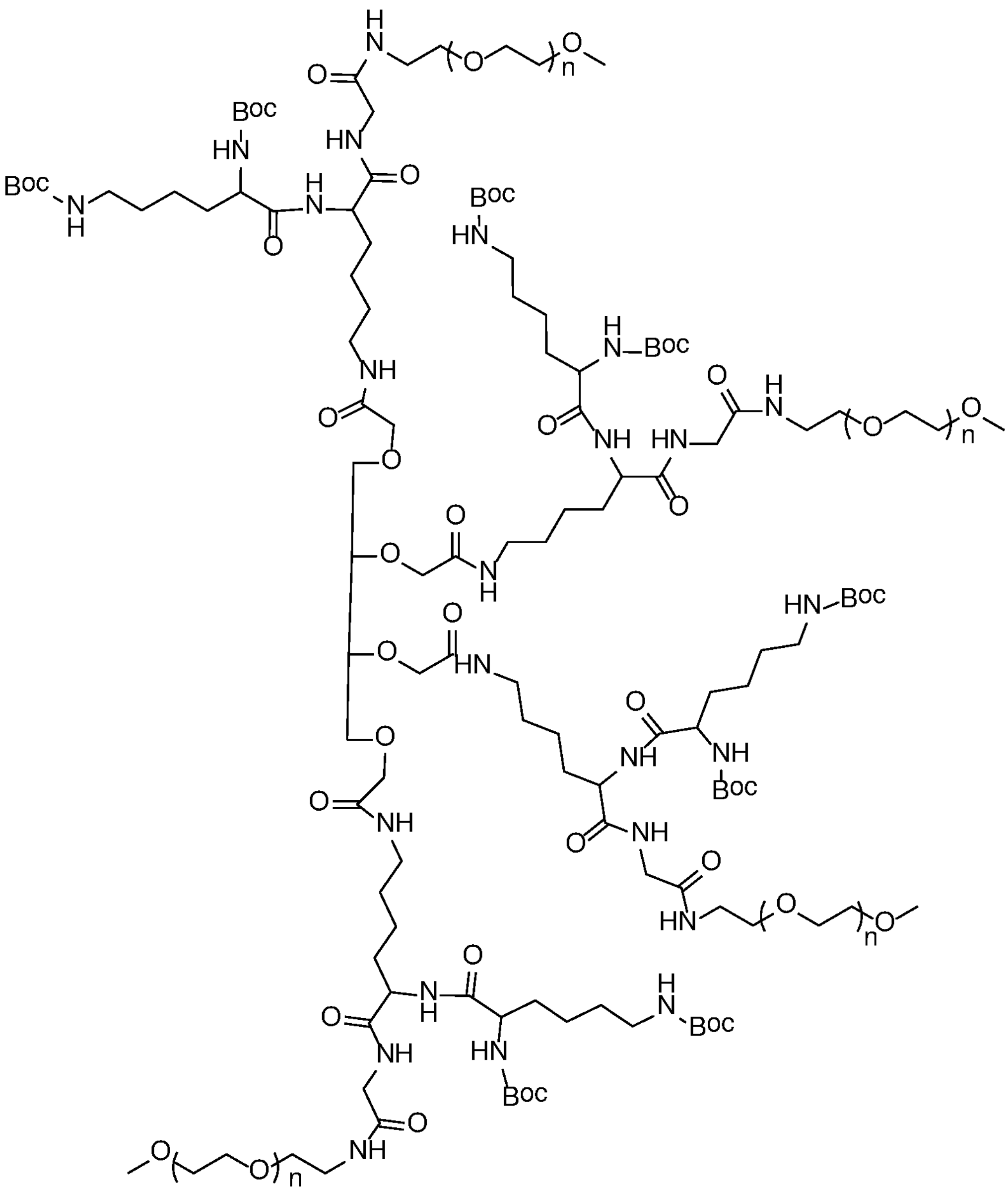

49-79 (0.45g, 0.1878mmol), M-$NH_2$HCl-5K (4.91g, 0.9389mmol, purchased from JenKem), HBTU (0.43g, 1.1237mmol), HOBT (0.15g, 1.1267mmol) were added in a 500 mL flask, and dissolved with DMF (60 mL), and then the mixed solution was stirred to react at -5°C for about 30 minutes. Then DIEA (0.56mL, 3.3780mmol) was slowly added dropwise, and the obtained solution continued to react at -5°C with stirring for 1 hour, and then reacted at room temperature in the dark for 3 days at a low speed of stirring. At the end of the reaction, the reaction solution was shaken with n-hexane (100 mL), and the supernatant was discarded. The above operations were repeated three times. The obtained solution was then shaken with methyl tert-butyl ether (80 mL) and a small amount of n-hexane (10 mL), and the supernatant was discarded. The above operations were repeated three times. A powdery solid was separated out of the reaction solution, and suction filtering was carried out. The filter cake was washed with methyl tert-butyl ether (40 mL × 3), the washed filter cakes were collected, and dissolved with a mixed solvent (100 mL) of methanol/ dichloromethane (1: 4), silica gel powder (20 g) was added, and the obtained mixture was then evaporated to dryness to obtain a powdery solid. The operations of dry sample loading, column chromatography and gradient elution with a dichloromethane mixed solution containing 1% ammonia water and 3%-7% methanol were carried out. The elution product was then collected, concentrated, and dried in a vacuum oven, thus obtaining the product (3.2g, 73.73%).

49-153

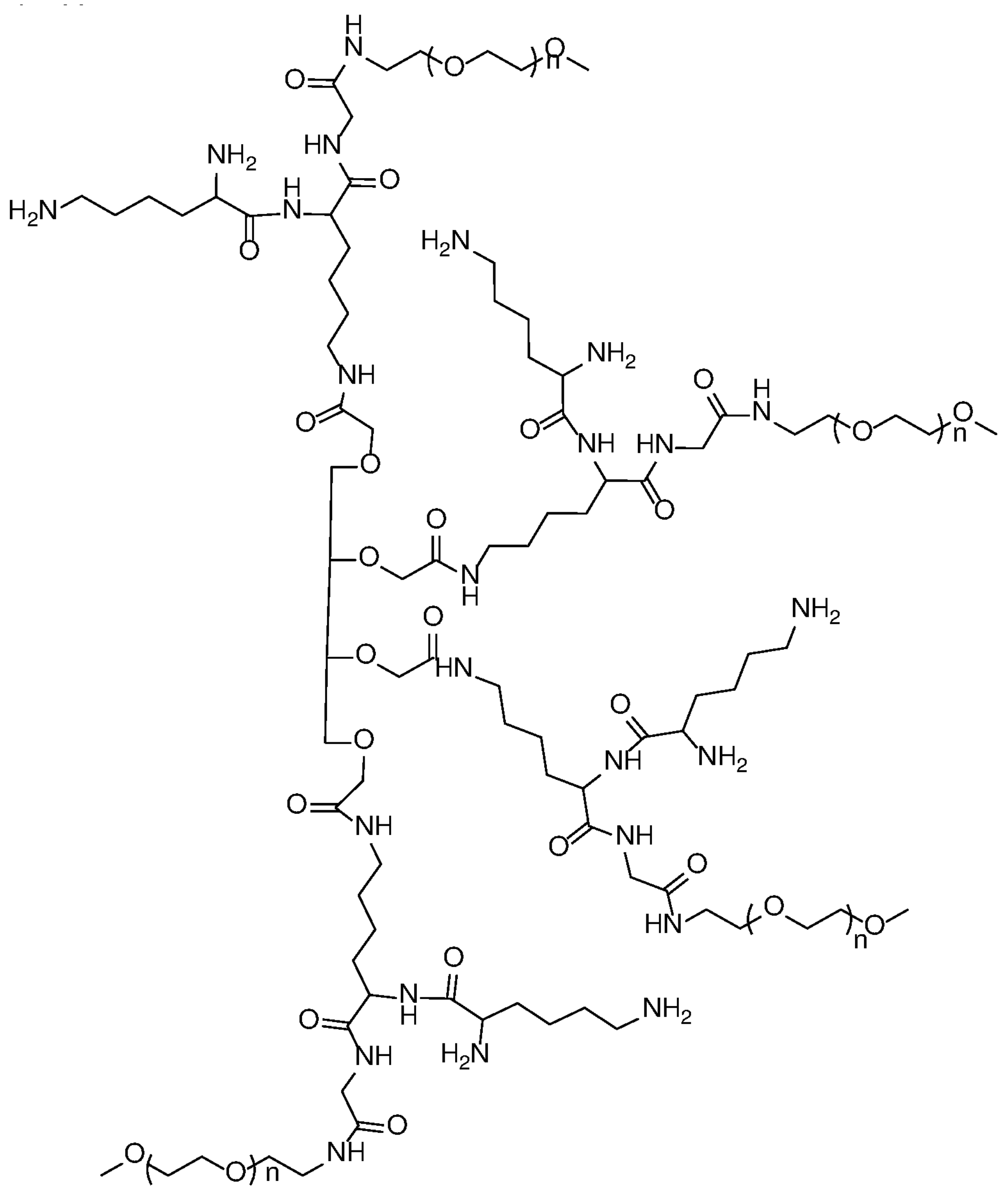

49-80 (3.2 g, 0.1383 mmol) was added in a 250 mL flask, and dissolved with dichloromethane (5 mL) and TFA (0.82 mL, 11.0640mmol), and then the mixed solution was stirred to react at room temperature in the dark overnight at a low speed. At the end of the reaction, the reaction solution was rotary evaporated to obtain an oily solution, and methyl tert-butyl ether (60 mL) was then added to the oily solution. A powdery solid was separated out of the obtained solution, and suction filtering was carried out. The filter cake was washed with methyl tert-butyl ether (40 mL × 3), the washed filter cakes were collected, and dissolved with a mixed solvent (100 mL) of methanol/ dichloromethane (1: 4), silica gel powder (20 g) was added, and the obtained mixture was then evaporated to dryness to obtain a powdery solid. The operations of dry sample loading, column chromatography and gradient elution with a dichloromethane mixed solution containing 3%-12% methanol were carried out. The elution product was then collected, concentrated, and dried in a vacuum oven, thus obtaining the product (1.74g, 56.35 %).

49-157

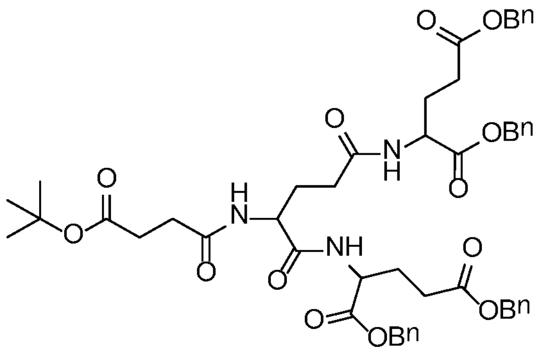

39-84 (5.16g, 6.7433 mmol), mono-tert-butyl succinate (1.40g, 8.0920 mmol, purchased from Accela), HBTU (3.84g, 10.1149mmol), HOBT (1.36g, 10.1149 mmol) were added in a 500 mL flask, and dissolved with DMF (50 mL), and then the mixed solution was stirred to react at -5°C for about 30 minutes. Then DIEA (10.03mL, 60.6897mmol) was slowly added dropwise, the obtained solution continued to react at -5°C with stirring for 1 hour, and was then moved to room temperature and stirred to react overnight. At the end of the reaction, deionized water (200 mL) was added to the reaction solution, the obtained solution was extracted with ethyl acetate (100 mL×3), and the obtained organic phases were combined. The organic phase was washed two times with saturated sodium chloride solution (200 mL), and concentrated, silica gel powder was added, and the obtained mixture was then evaporated to dryness to obtain a powdery product. The operations of column chromatography and gradient elution (with 50%-90% ethyl acetate, the rest of petroleum ether) were carried out. The elution product was then collected, concentrated, thus obtaining the product (5.66g, 90.99%).

49-158

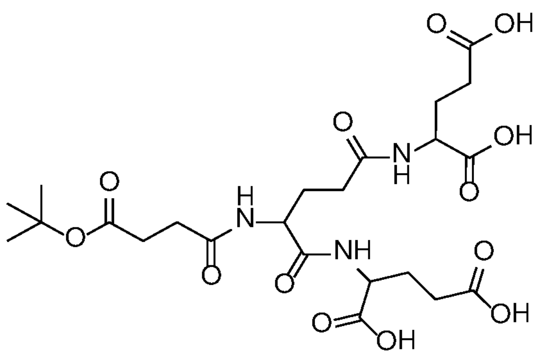

49-157 (2.8g, 3.0367mmol) and 10% Pd/C (0.08 g) were added in a hydrogenation reactor, and dissolved with DMF (30 mL). The hydrogenation reactor was then sealed to perform the "three pumping and three charging" operation so that the pressure on the hydrogenation reactor was read as 0.18 MPa, and then the obtained solution reacted at room temperature overnight. At the end of the reaction, the reaction solution was filtered with diatomaceous earth. The filter cake was washed with DMF (20 mLx3), thus obtaining the DMF solution of the product as raw material for the next reaction.

49-159

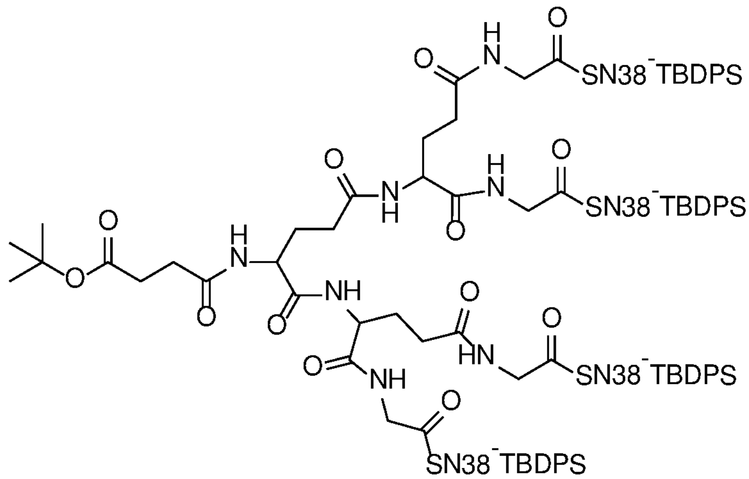

49-158 (1.71g, 3.0367mmol), 47-98 (8.77g, 12.7541mmol), HBTU (6.90g, 18.2202mmol), HOBT (2.46g, 18.2202mmol) were added in a 500 mL flask, and dissolved with DMF (60 mL), and then the mixed solution was stirred to react at -5°C for about 30 minutes. Then DIEA (13mL, 78.9542mmol) was slowly added dropwise, and the obtained solution continued to react at -5°C with stirring for 1 hour, and was then moved to room temperature and stirred to react overnight. At the end of the reaction, the reaction solution was shaken with n-hexane (100 mL), and the supernatant was discarded. The above operations were repeated three times. The obtained solution was then shaken with methyl tert-butyl ether (80 mL) and a small amount of n-hexane (10 mL), and the supernatant was discarded. The above operations were repeated three times. A powdery solid was separated out of the obtained solution, and suction filtering was carried out. The filter cake was washed with methyl tert-butyl ether (40 mL × 3), the washed filter cakes were collected, and dissolved with a mixed solvent (100 mL) of methanol/ dichloromethane (1: 4), silica gel powder (60 g) was added, and the obtained mixture was then evaporated to dryness to obtain a powdery solid. The operations of dry sample loading, column chromatography, and gradient elution with a dichloromethane mixed solution containing 3%-7% methanol were carried out. The elution product was then collected, concentrated, and dried in a vacuum oven, thus obtaining the product (11.3g, extra-quota).

49-161

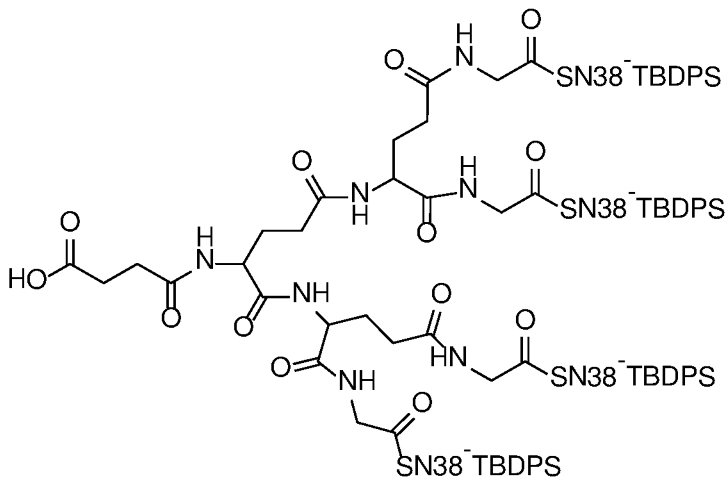

49-159 (9.84g, 3.0367mmol) was added in a 250 mL flask, and dissolved with dichloromethane (8 mL), TFA (8mL) was added, and then the mixed solution was stirred to react at room temperature overnight. At the end of the reaction, the reaction solution was rotary evaporated to obtain an oily solution, and methyl tert-butyl ether (60 mL) was then added to the oily solution. A powdery solid was separated out of the obtained solution, and suction filtering was carried out. The filter cake was washed with methyl tert-butyl ether (40 mL × 3), the washed filter cakes were collected, and dissolved with a mixed solvent (100 mL) of methanol/ dichloromethane (1: 4), silica gel powder (60 g) was added, and the obtained mixture was then evaporated to dryness to obtain a powdery solid. The operations of dry sample loading, column chromatography and gradient elution with a dichloromethane mixed solution containing

1%-4% methanol were carried out. The elution product was then collected, concentrated, and dried in a vacuum oven, thus obtaining the product (2.7g, 27.92%).

49-162

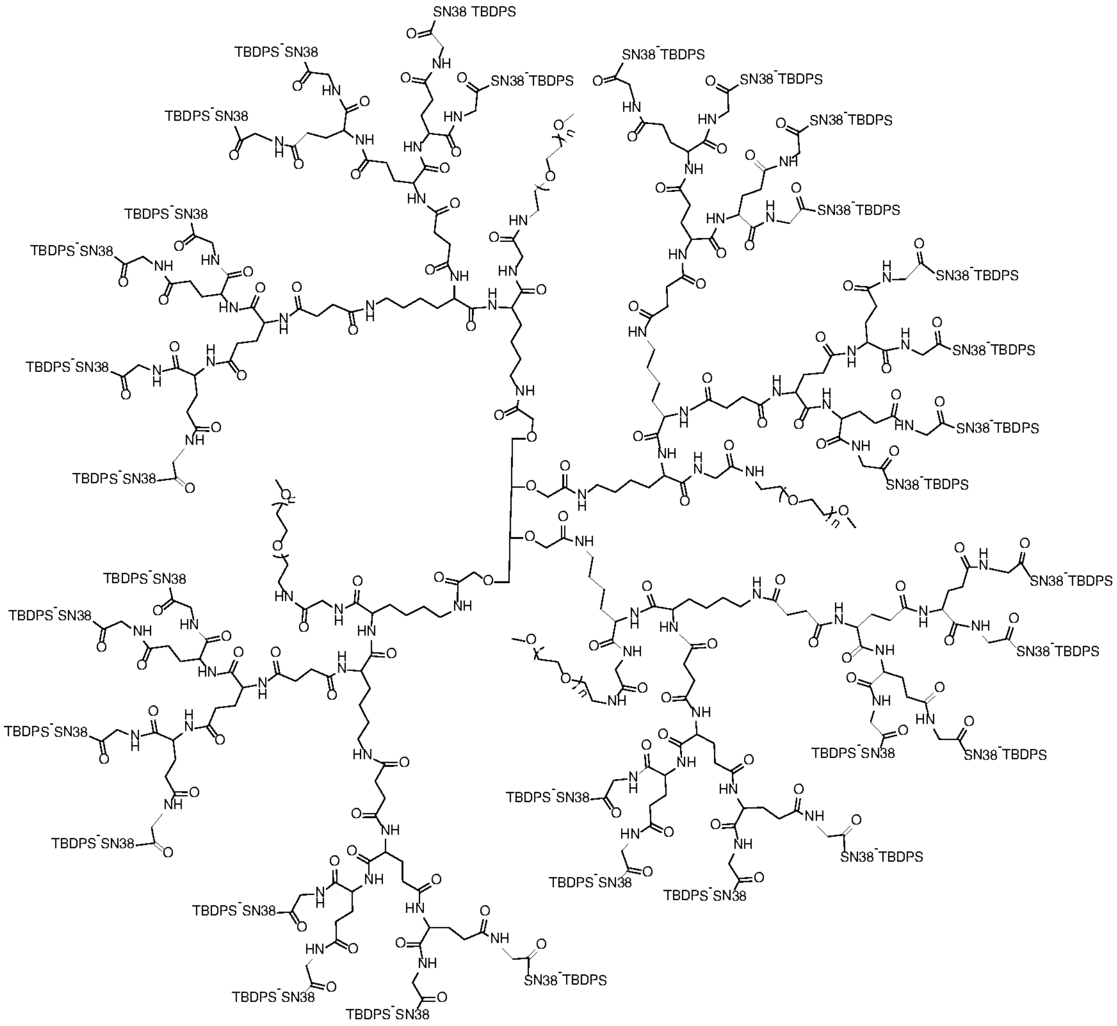

49-153 (1.19g, 0.0535mmol), 49-161 (1.5g, 0.4710mmol), HBTU (0.24g, 0.6422mmol), HOBT (0.08g, 0.6422mmol) were added in a 500 mL flask, and dissolved with DMF (60 mL), and then the mixed solution was stirred to react at -5°C for 30 minutes. Then DIEA (0.60mL, 3.6395mmol) was slowly added dropwise, and the obtained solution continued to react at -5°C with stirring for 1 hour, and then reacted at room temperature in the dark overnight at a low speed of stirring. At the end of the reaction, the reaction solution was shaken with n-hexane (100 mL), and the supernatant was discarded. The above operations were repeated three times. The obtained solution was then shaken with methyl tert-butyl ether (80 mL) and a small amount of n-hexane (10 mL), and the supernatant was discarded. The above operations were repeated three times. A powdery solid was separated out of the obtained solution, and suction filtering was carried out. The filter cake was washed with methyl tert-butyl ether (40 mL × 3), the washed filter cakes were collected, and dissolved with a mixed solvent (100 mL) of methanol/ dichloromethane (1: 4), silica gel powder (20 g) was added, and the obtained mixture was then evaporated to dryness to obtain a powdery solid. The operations of dry sample loading, column chromatography and gradient elution with a dichloromethane mixed solution containing 3%-15% methanol were carried out. The elution product was then collected, concentrated, and dried in a vacuum oven, thus obtaining the product (0.95g, 37.25%).

49-166

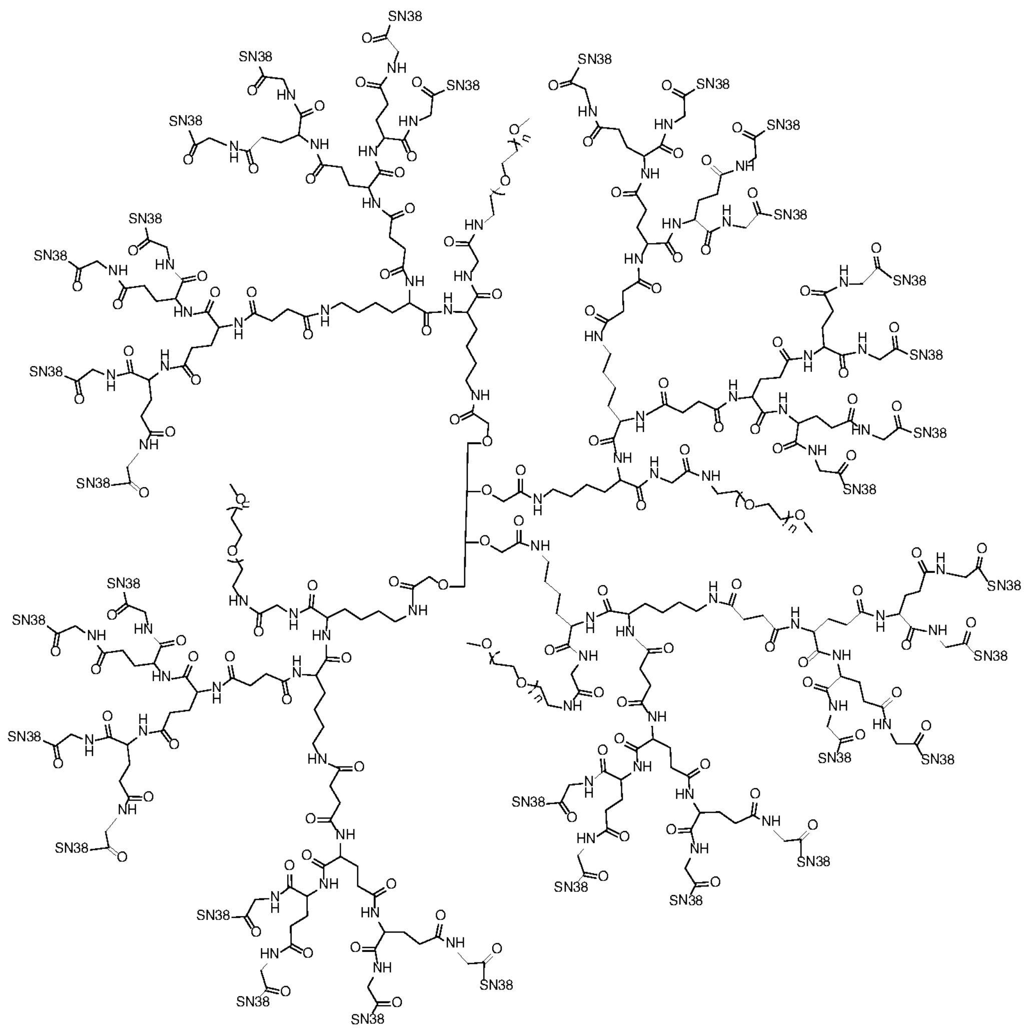

49-162 (0.95g, 0.0199mmol) was added in a 500ml flask, and dissolved with THF (10ml) and diluted hydrochloric acid (10ml, 0.05mmol/L) by ultrasonic, TBAF (0.5g, 1.9133mmol) was added, and then the mixed solution was stirred to react at room temperature in the dark for 3 hours. At the end of the reaction, the reaction solution was evaporated to dryness. The obtained dry product was dissolved with DMF (5ml), and the obtained solution was precipitated with isopropanol. Such operations were repeated three times. The precipitate was dissolved with anhydrous ethanol and a small amount of dichloromethane, and the obtained solution was precipitated with methyl tert-butyl ether. Such operations were repeated three times. The obtained solid was then collected, and dried in a vacuum oven, thus obtaining the product (0.75g, 93.75%).

$^1$H-NMR (600 MHz, DMSO-$d_6$) δ 8.44 - 8.22 (m, 24H), 8.05 - 7.89 (m, 46H), 7.44 - 7.27 (m, 78H), 5.53 - 5.34 (m, 50H), 5.31 - 5.08 (m, 52H), 4.55 - 4.44 (m, 15H), 4.29 - 4.13 (m, 31H), 4.11 - 3.89 (m, 71H), 3.89 - 3.80 (m, 23H), 3.78 - 3.60 (m, 82H), 3.55 -3.44(m, 1892H), 3.11 - 2.75 (m, 100H), 2.36 - 2.21 (m, 28H), 2.20 - 1.97 (m, 91H), 1.61 - 1.49 (m, 34H), 1.31 - 1.16(m, 136H), 0.97 - 0.91(m, 30H), 0.90-0.76(m, 79H).

Synthesis of 38-161

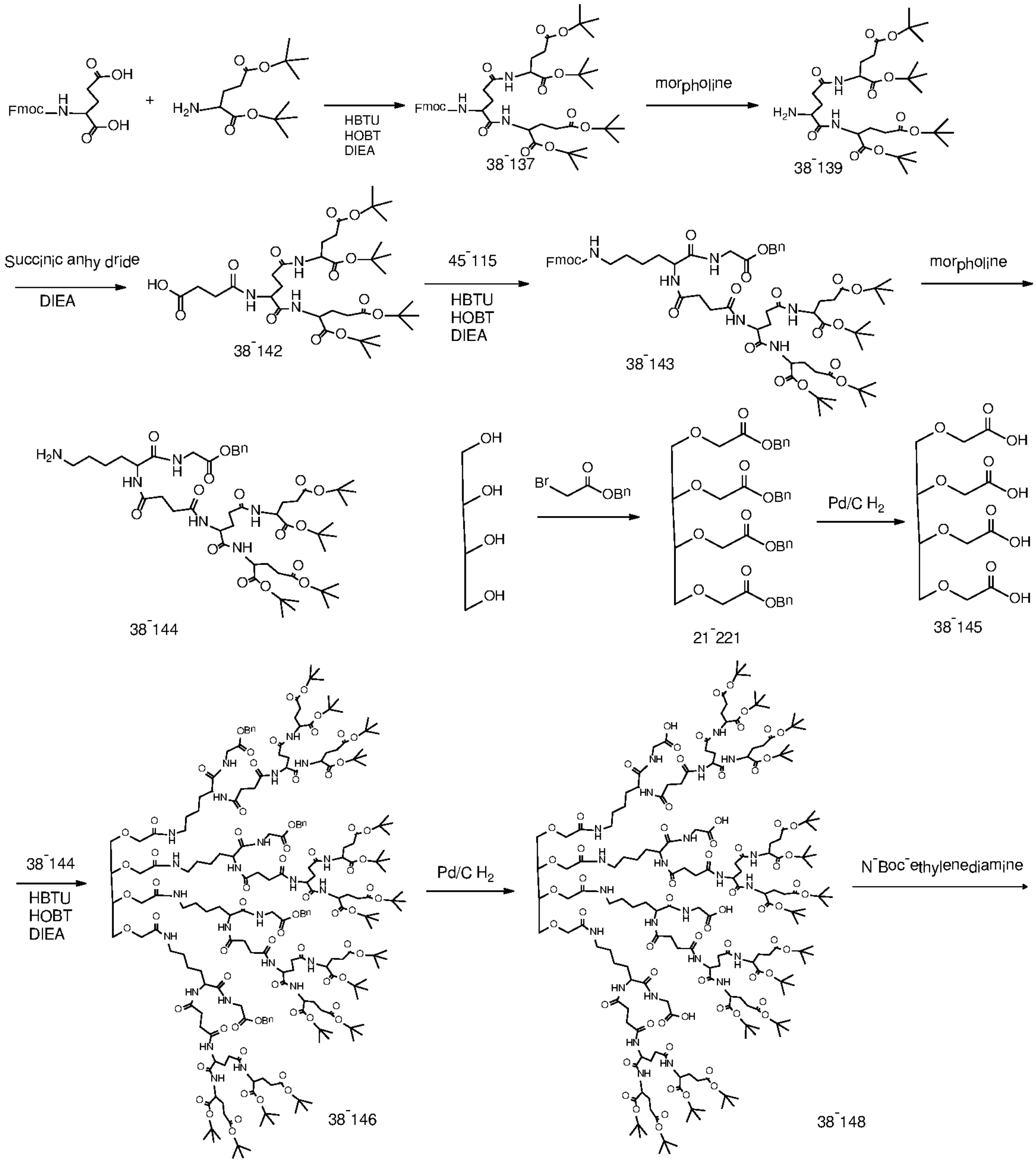


38-137

38-139

38-142

38-143

38-144

21-221

38-145

38-146

38-148

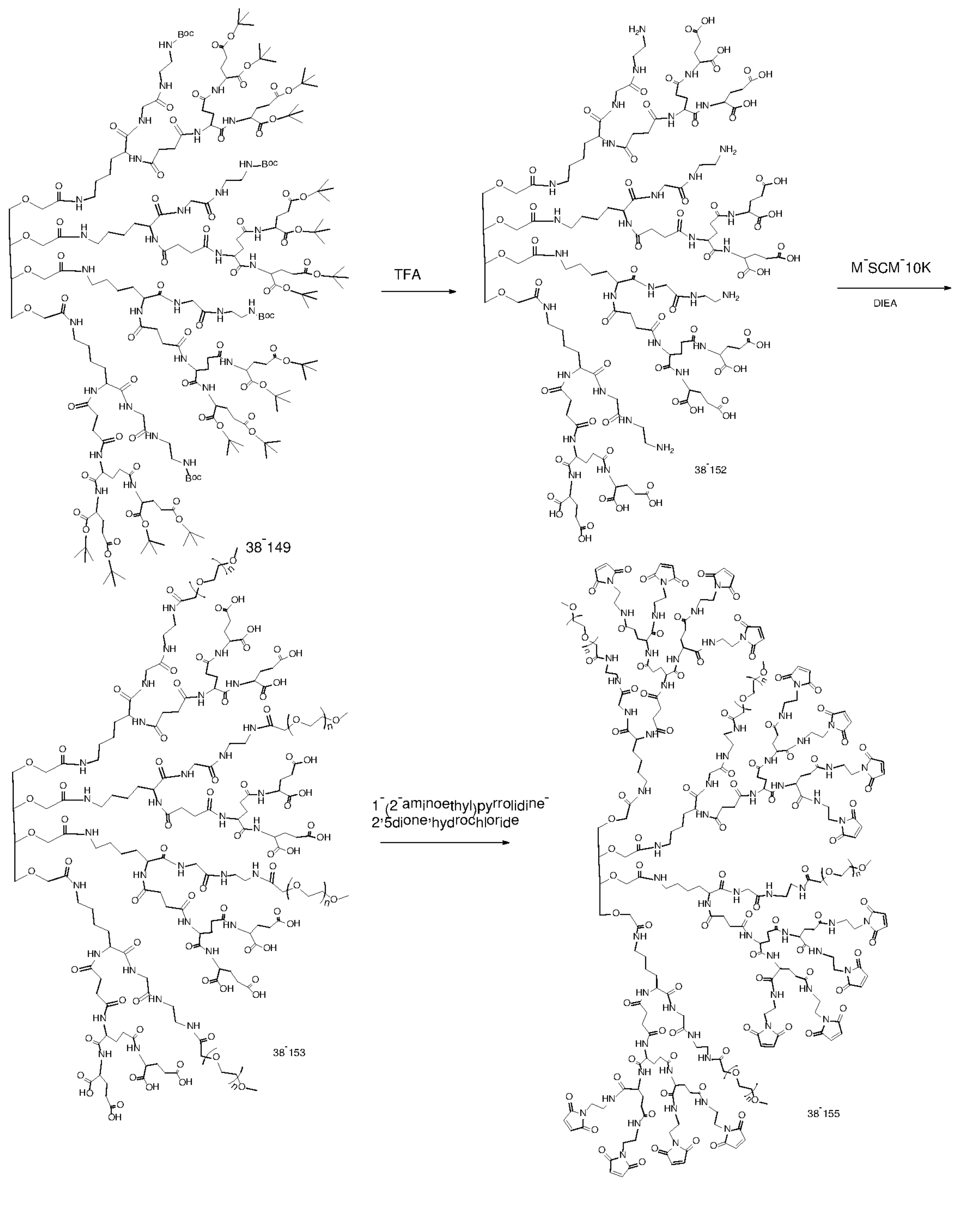


38-149

38-152

38-153

38-155

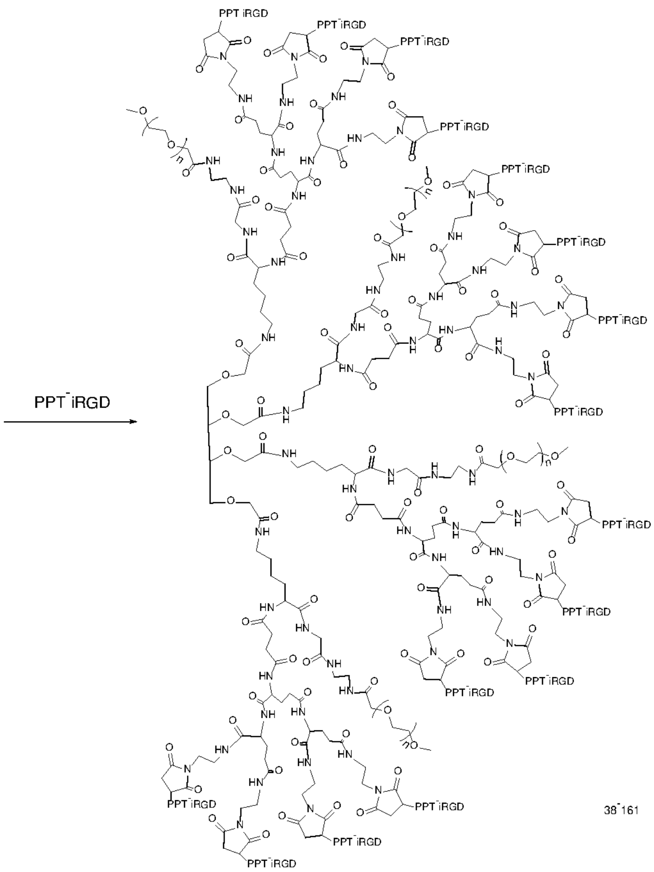


38-161

38-137

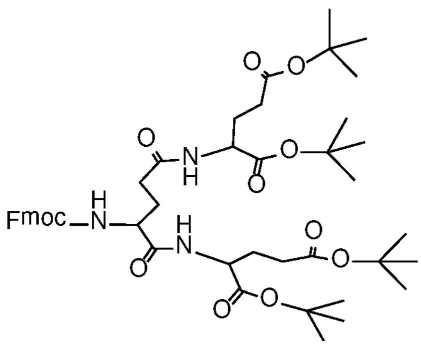

Fmoc-Glu-OH (2.974g, 8.05mmol, purchased from Aladdin), $NH_2$-Glu-OtBu (5g, 16.90mmol, purchased from innochem), HBTU (9.16g, 24.15mmol), HOBT (3.26g, 24.15mmol) were added in a 500 mL flask, and dissolved with DMF (10mL), and the mixed solution was stirred at -5°C for 30 minutes. DIEA (11.97mL, 72.45mmol) was slowly added dropwise, and then the mixed solution reacted at -5°C overnight. At the end of the reaction, the reaction solution was extracted with pure water and ethyl acetate, washed with saturated saline solution, and dried over anhydrous sodium sulfate, and then suction filtering was carried out. The organic phase was concentrated, and evaporated to dryness, thus obtaining the product 7 g.

38-139

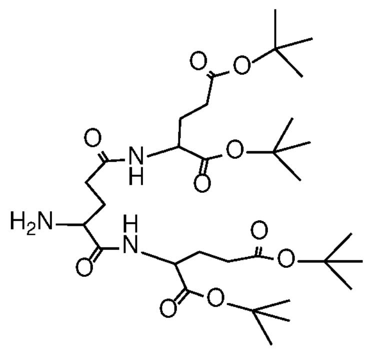

Reactant 38-137 (6.9g, 8.05mmol) was added in a 250 mL flask, and dissolved with DMF (50mL), morpholine (14mL, 161mmoL) was added, and then the mixed solution was stirred to react at room temperature for 3 hours. At the end of the reaction, saturated saline solution (150mL) and ethyl acetate (200mL) were added to the reaction solution, and the organic phase was separated. The aqueous phase was extracted three times with ethyl acetate (150mL×3) until there was no product in the aqueous phase. The obtained organic phases were combined, washed two times with saturated saline solution (150mL×2), and evaporated to dryness, thus obtaining the product 5 g.

38-142

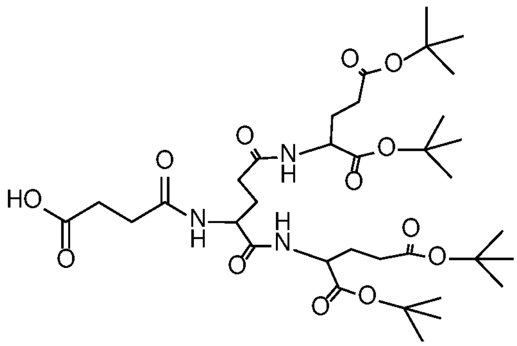

38-139 (5g, 8.05mmol) was added in a 250 mL flask, and dissolved with DMF (50mL). Under the condition of stirring at -5°C, DIEA (6.6526mL, 40.25mmol) was added, and after reacting for 30 minutes, succinic anhydride (2.4137g, 24.15, mmol) was quickly added, and the obtained solution reacted at the low temperature until the reaction ended. At the end of the reaction, the reaction solution was extracted with deionized water and ethyl acetate. The organic phase was washed twice with saturated saline solution, concentrated, and evaporated to dryness, thus obtaining the product 5g, yield 86%.

38-143

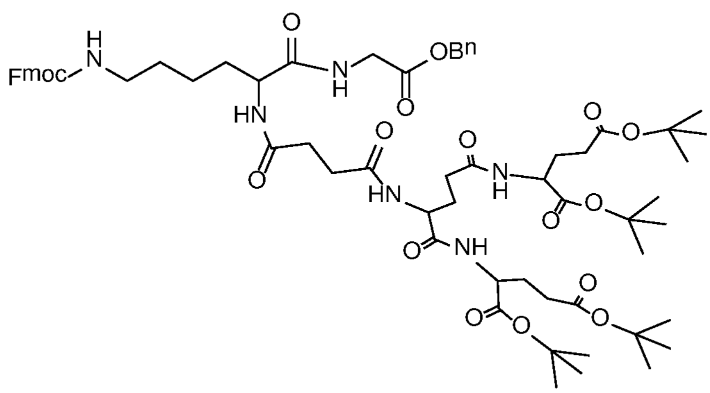

38-142 (5g, 6.8506mmol), 45-115 (3.53g, 6.8506mmol), HBTU (3.897g, 10.2759mmol), HOBT (1.3885g, 10.2759mmol) were added in a 500 mL flask, and dissolved with DMF (10mL), and the mixed solution was stirred at -5°C for 30 minutes. DIEA (5.0952mL, 30.8277mmol) was slowly added dropwise, and then the mixed solution

reacted at -5°C overnight. At the end of the reaction, the reaction solution was extracted with pure water and ethyl acetate. The organic phase was washed with saturated saline solution, concentrated and evaporated to dryness, thus obtaining the product 7 g, yield 83.3%.

38-144

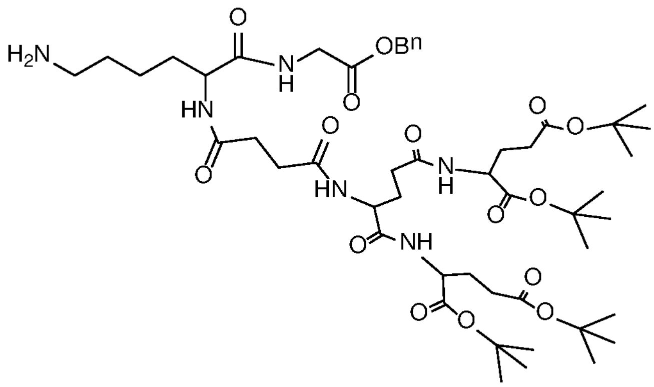

Reactant 38-143 (7g, 5.7029mmol) was added in a 250 mL flask, and dissolved with DMF (50mL), morpholine (9.9368mL, 114.0585mmol) was added, and then the mixed solution was stirred to react at room temperature for 3 hours. At the end of the reaction, saturated saline solution (150mL) and ethyl acetate (200mL) were added to the reaction solution, and the organic phase was separated. The aqueous phase was extracted three times with ethyl acetate (150mL×3) until there was no product in the aqueous phase. The obtained organic phases were combined, washed twice with saturated saline solution (150mL×2), and evaporated to dryness. The operations of column chromatography and gradient elution with 1%-4% methanol/dichloromethane were carried out, thus obtaining the product 4 g, yield 70%.

21-221

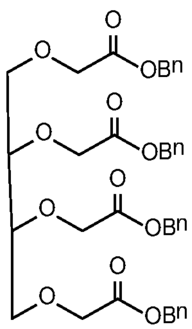

Erythritol (4.5g, 36.849mmol) was added in a 500 mL two-neck flask, and dissolved with THF (60mL) in a condition of ultrasonic, and then the obtained solution was stirred at 0°C. After introducing nitrogen for protective purpose, potassium tert-butoxide (200mL, 184.245mmol) was added, and the obtained solution was stirred at 0°C for 2 hours. Then, benzyl bromoacetate (29.187mL, 184.245mmol) was added, and the obtained solution was stirred for 3 hours and then moved to react at room temperature. At the end of the reaction, the reaction solution was extracted with pure water and ethyl acetate, and the organic phase was concentrated. The operations of dry sample loading, column chromatography and gradient elution with 1%-2% ethyl acetate/petroleum ether were carried out, thus obtaining the product 5g, yield 20%.

38-145

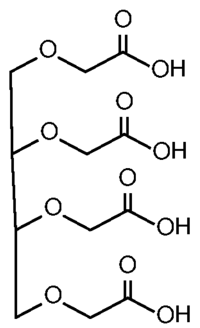

21-221 (0.64g, 0.9793mmol) and 10%Pd/C (100mg) were added in a hydrogenation reactor, DMF (30mL) was slowly added to dissolve the reactant with stirring, hydrogen was introduced to a pressure of 300 psi, and then the mixed solution was stirred to react at room temperature overnight. Next day, the reaction solution was filtered by suction through a sand core funnel filled with diatomaceous earth to remove the Pd/C, thus obtaining the DMF solution of the product, directly used for next reaction.

38-146

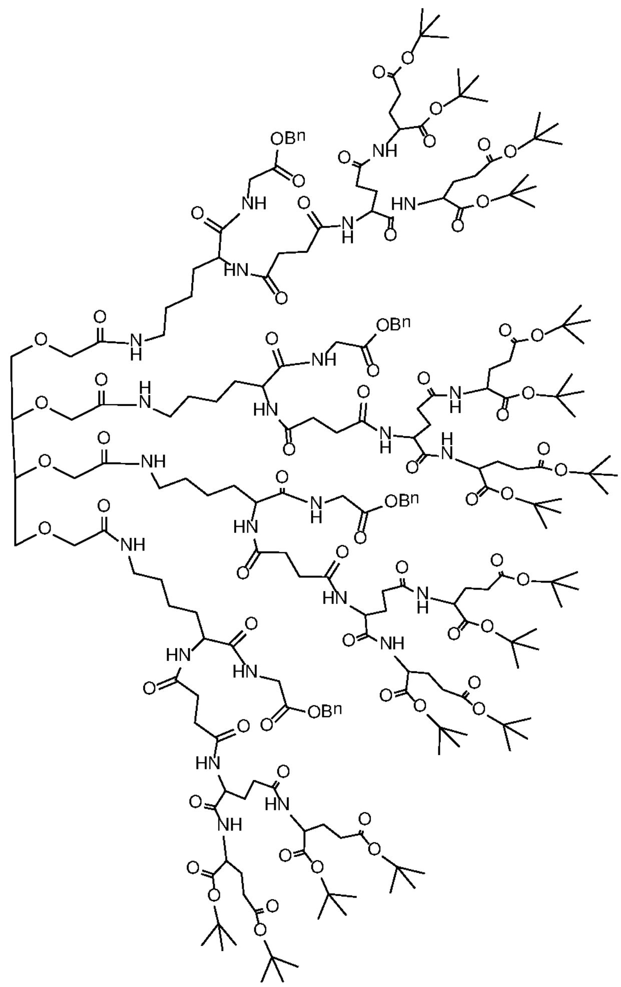

38-144 (4g, 3.9793mmol), HBTU (9.0546g, 23.8758mmol), HOBT (3.2261g, 23.8758mmol) were added in a 250 mL flask, and dissolved with the DMF solution of 38-145 (0.9043mmol), and the mixed solution was stirred at -5°C for 30 minutes. DIEA (11.8354mL, 71.6274mmol) was slowly added dropwise, and then the mixed solution reacted at -5°C overnight. At the end of the reaction, pure water was added to the reaction solution, and suction filtering was carried out. The operations of dry sample loading, column chromatography and elution with 1%-5% methanol/dichloromethane were carried out, thus obtaining the product 2.5g, yield 74.34%.

38-148

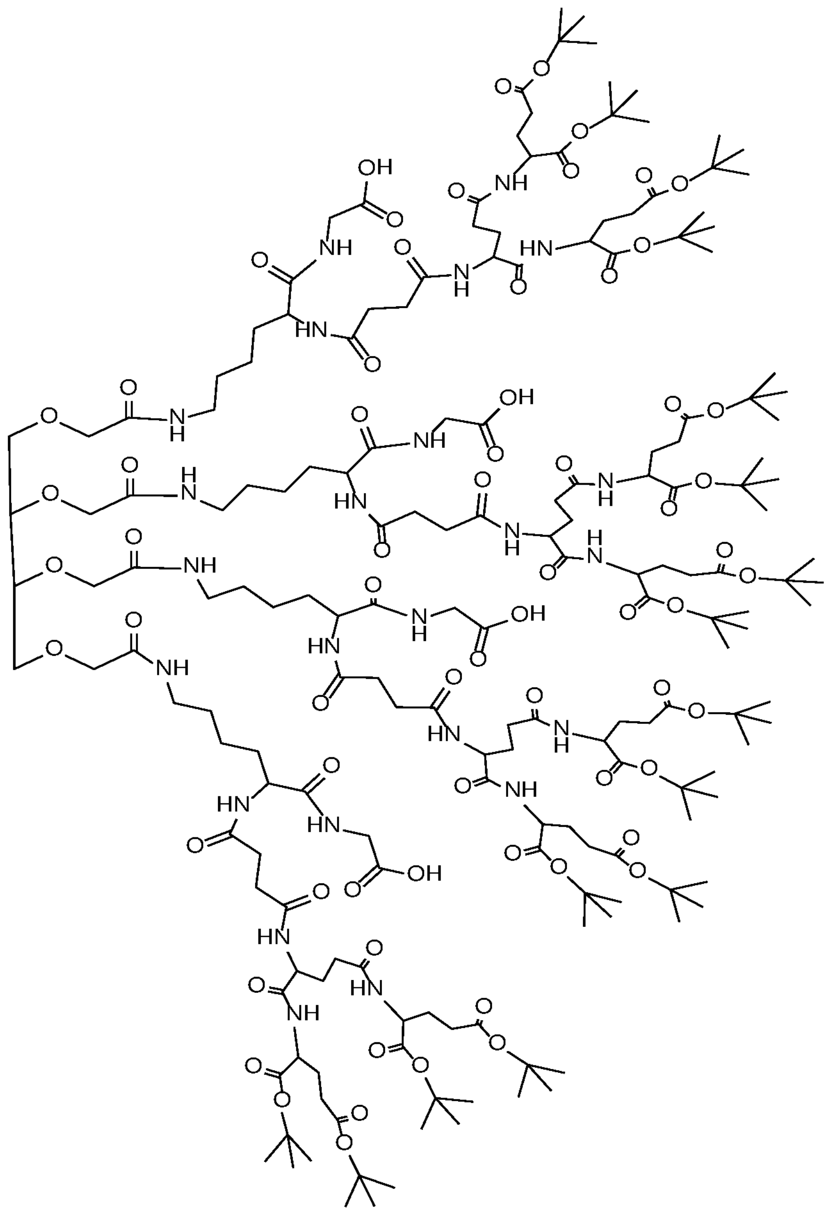

38-146 (0.29g, 0.0676mmol) and 10%Pd/C (50mg) were added in a hydrogenation reactor, DMF (30mL) was slowly added to dissolve the reactant with stirring, hydrogen was introduced to a pressure of 300 psi, and then the mixed solution was stirred to react at room temperature overnight. Next day, the reaction solution was filtered by suction through a sand core funnel filled with diatomaceous earth to remove the Pd/C, thus obtaining the DMF solution of the product, directly used for next reaction.

38-149

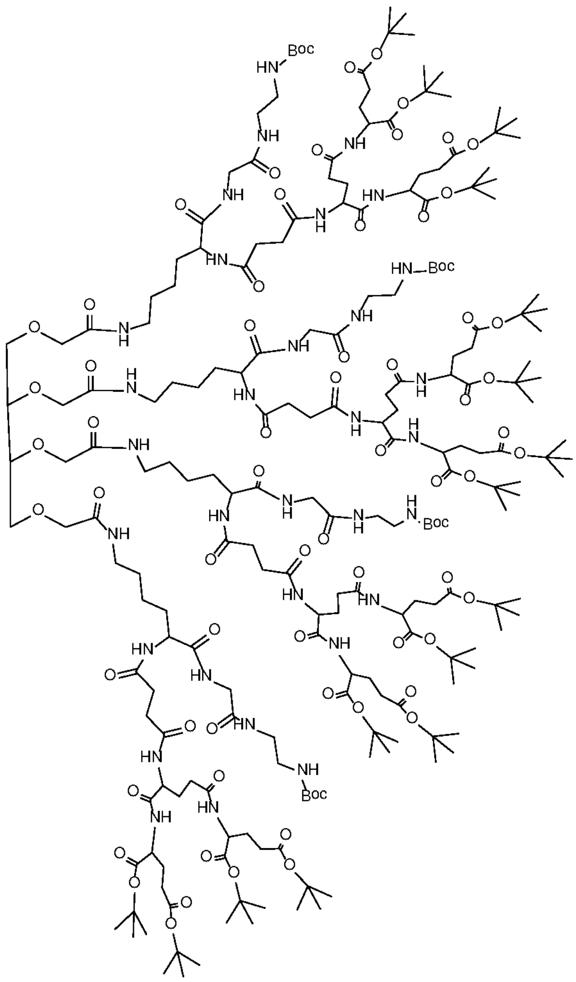

N-Boc-ethylenediamine (0.0656g, 0.4092mmol), HBTU (0.2116g, 0.5580mmol), HOBT (0.0754g, 0.5580mmol) were added in a 250 mL flask, and dissolved with the DMF solution of 38-148 (0.0930mmol), and the mixed solution was stirred at -5°C for 30 minutes. DIEA (0.27mL, 1.67mmol) was slowly added dropwise, and then the mixed solution reacted at -5°C overnight. At the end of the reaction, pure water was added to the reaction solution, and suction filtering was carried out. The operations of dry sample loading, column chromatography and elution with 1%-5% methanol/dichloromethane were carried out, thus obtaining the product 0.2g, yield 50%.

38-152

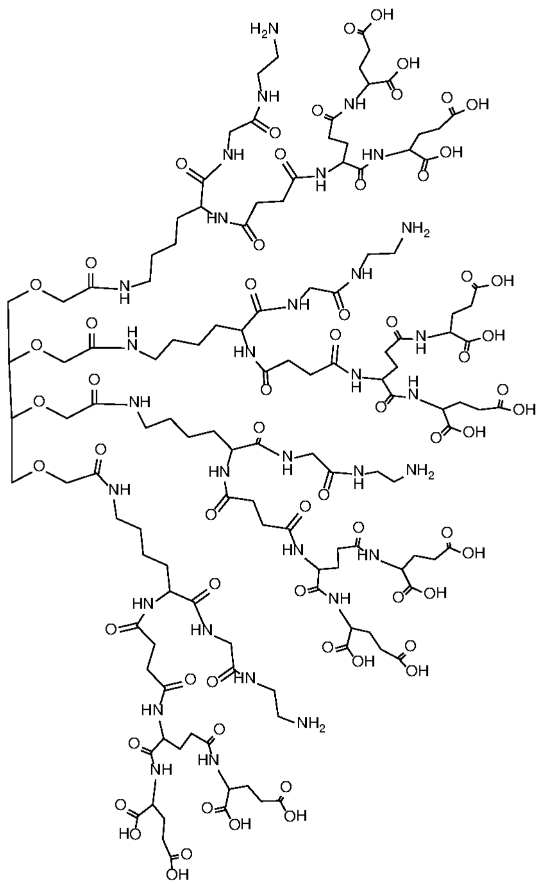

Compound 38-149 (0.2g, 0.0443mmol) was dissolved with dichloromethane (20mL) and TFA (2mL) in a condition of ultrasonic, and then the mixed solution was stirred to react. At the end of the reaction, methyl tert-butyl ether and n-hexane were added, and suction filtering was carried out, thus obtaining the product 0.14g, yield 100%.

38-153

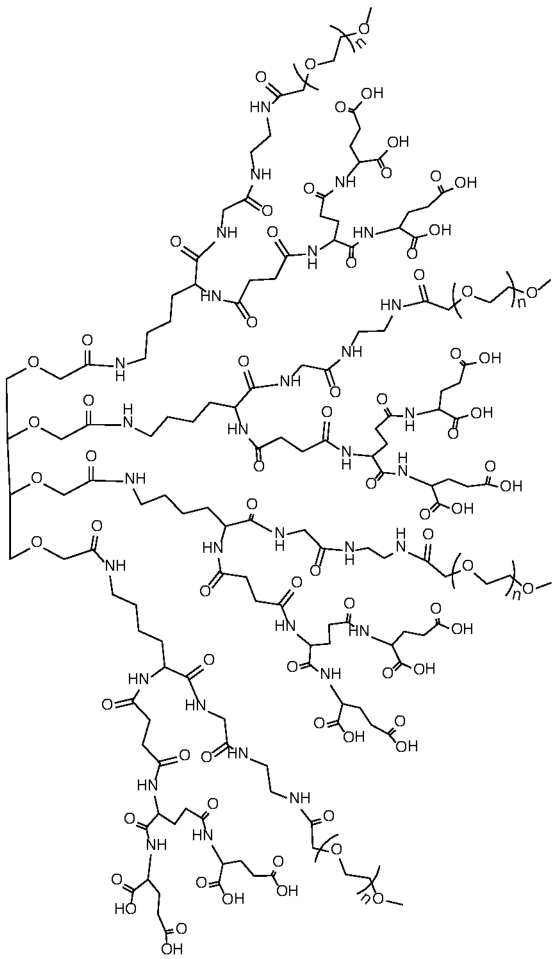

Compound 38-152 (0.14g, 0.1443mmol) was dissolved with DMF (20mL) in a condition of ultrasonic, and the obtained solution was stirred under the condition of - 5°C for 20 minutes. DIEA (0.29mL, 1.772mol) was slowly added, the mixed solution was stirred for 20 minutes, and then M-SCM-10K (2.11g, 0.1950mmol) was added. After 1 hour, the mixed solution was moved to room temperature and stirred to react in the dark for 7 days at a low speed. At the end of the reaction, methyl tert-butyl ether and n-hexane were added, and suction filtering was carried out to obtain a filter cake. The operations of dry sample loading, column chromatography and gradient elution with 1% ammonia water + 5%-25% methanol/dichloromethane were carried out, thus obtaining the product 1.5g, yield 75%.

38-155

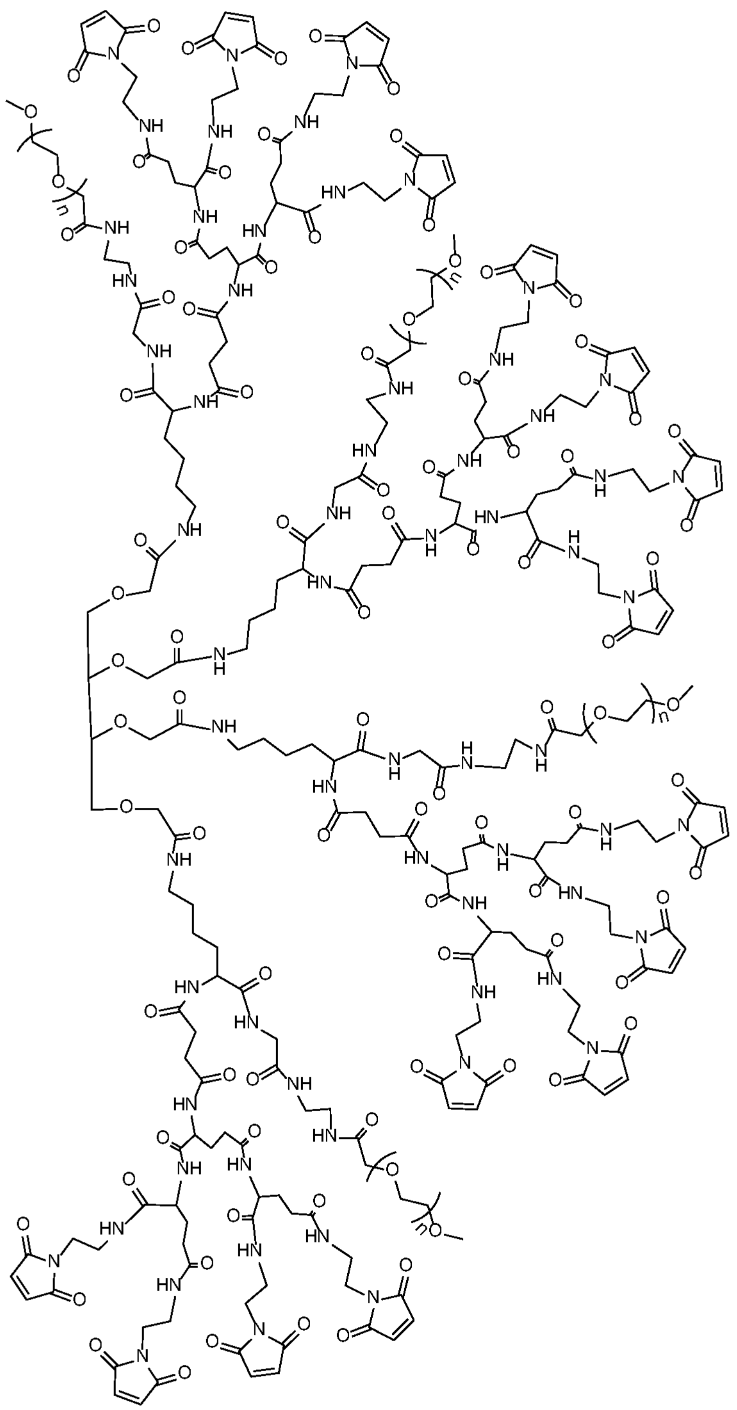

38-153 (1.3g, 0.0288mmol), 1-(2-aminoethyl)-1H-pyrrole-2,5-dione hydrochloride (0.0808g, 0.5767mmol, purchased from Shanghai Chemicalbook Biology), HBTU (0.2621g, 0.6912mmol), HOBT (0.0934g, 0.6912mmol) were added in a 500 mL flask, and dissolved with DMF (10mL), and the mixed solution was stirred under the condition of -5°C for 30 minutes. DIEA (0.3427mL, 2.0736mmol) was slowly added dropwise, and then the mixed solution reacted under the condition of -5°C overnight. At the end of the reaction, the reaction solution was extracted with pure water and ethyl acetate. The organic phase was washed with saturated saline solution, concentrated and evaporated to dryness, thus obtaining the product 1.3 g.

38-161

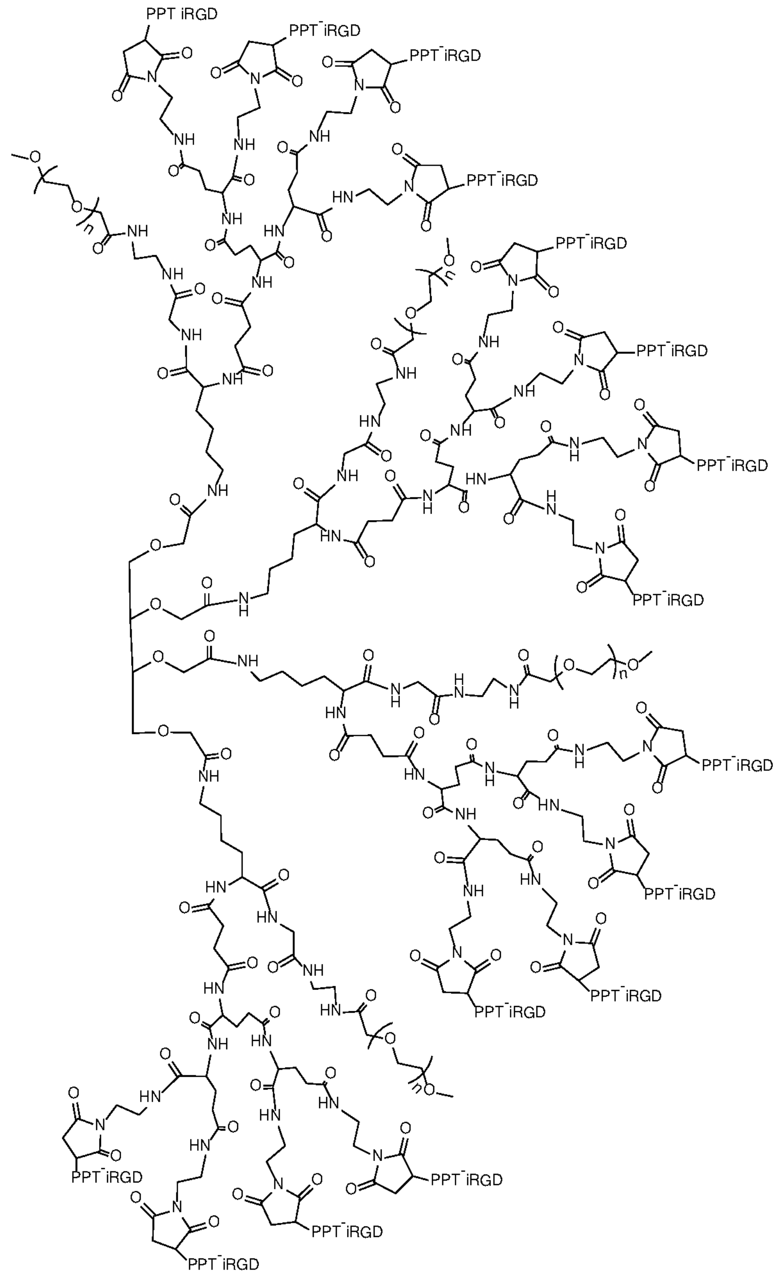

38-155 (1.3g, 0.0276mmol), PPT-iRGD (0.8847g, 0.5527mmol, purchased from Dangang Bio), were added in a 250 mL flask, and dissolved with DMSO (50mL), and the obtained solution was stirred in the dark under the condition of 40°C for 2 days, and then stirred at room temperature for 3 days. At the end of the reaction, methyl tert-butyl ether was added to the reaction solution, the upper layer solution was discarded, and the precipitated solid was dissolved with 10% methanol/dichloromethane, and then silica gel powder (5g) was added. The mixture was dried, loaded on silica gel column, and eluted with 5%-10% methanol/dichloromethane. 1.2g of the product was obtained with a yield of 57.14%.

$^1$H-NMR (600 MHz, DMSO-$d_6$) δ 9.57-9.13 (m, 31H), 9.09-8.63 (m, 10H), 8.02-7.91 (m, 50H), 7.91-7.80 (m, 75H), 7.79-7.70 (m, 4H), 7.69-7.54 (m, 2H), 7.53-7-48 (s, 1H), 7.45-7.38 (s, 1H), 7.37-7.27 (m, 2H), 7.26-7.19 (s, 1H), 3.82-3.77(m, 15H), 3.76-3.72 (m, 13H), 3.67-3.63 (m, 5H), 3.62-3.52 (m, 63H), 3.55-3.42(m, 3934H), 3.15-3.07 (m, 58H), 3.06-3.01 (m, 44H), 2.99-2.95(m, 13H), 2.94-2.85 (m, 625H), 2.78-2.76 (m, 6H), 2.75-2.70(m, 155H), 2.69-2.66(m, 11H), 2.63-2.59(m, 4H), 2.58-2.53(m, 132H), 2.52-2.45(m, 134H), 1.69-1.62(m, 11H), 1.59-1.49 (m, 8H), 1.42-1.34(m, 13H), 1.31-1.22(m, 460H), 1.20-1.10(m, 27H).

Synthesis of 38-192:

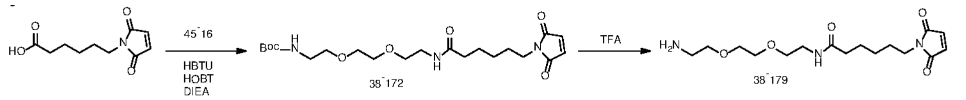


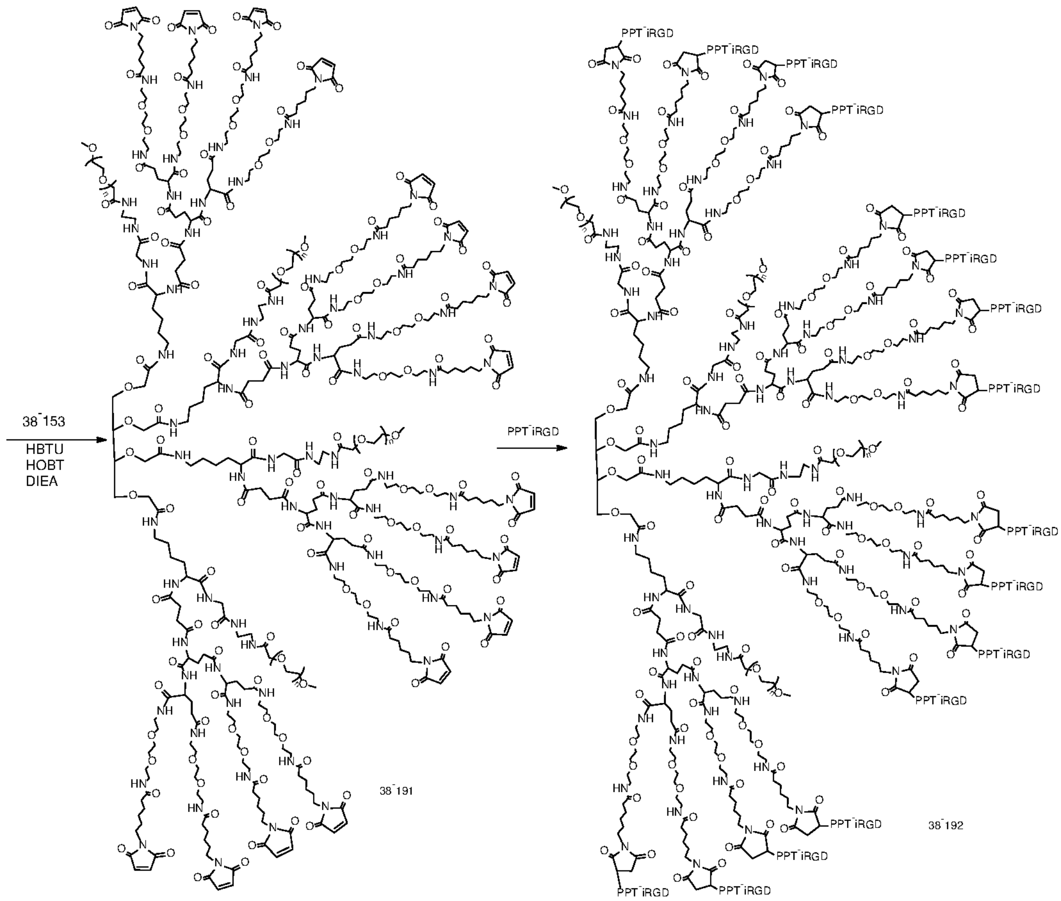


45-16

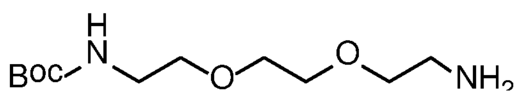

1,2-Bis (2-aminoethoxy) ethane (50mL, 340.7327mmol, purchased from Tcl) was added in a 500 mL flask, and dissolved with dichloromethane (150mL), triethylamine (94.9828 mL, 681.4654mmol) was added, (Boc) $_2$O (74.3751g, 340.7327mmol) was slowly added in batches under stirring, and then the mixed solution was stirred to react at room temperature overnight. Next day, silica gel powder was directly added to the reaction solution, followed by evaporation to dryness. The operations of sample loading, column chromatography and gradient elution with 2%-5% methanol/dichloromethane were carried out, thus obtaining the product 9.3g, yield 10%.

38-172

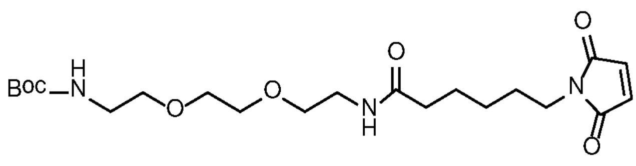

45-16 (8g, 11.9225mmol), 6-maleimidohexanoic acid (2.7701g, 13.1147mmol), HBTU (6.782g, 17.8838mmol), HOBT (2.4166g, 17mmol) were added in a 500 mL flask, and dissolved with a proper amount of DMF(50mL), and the obtained solution was stirried in the dark at -5°C for 30 minutes. Then DIEA (8.8676mL, 53.6512mmol) was slowly added dropwise, and the mixed solution reacted in the dark at -5°C with stirring for 1 hour, and then moved to room temperature and reacted with stirring in the dark overnight. At the end of the reaction, the reaction solution was extracted with ethyl acetate and saturated saline solution. The organic phase was concentrated, and evaporated to dryness, thus obtaining the product 10g.

38-179

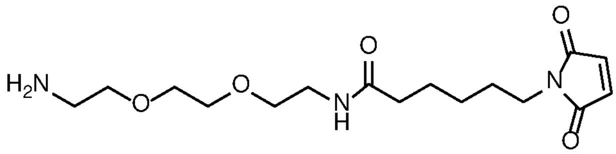

38-172 (10g, 22.649 mmol) was added in a 250 mL flask, and dissolved with dichloromethane (15 mL) and TFA ( 25ml, 339.7325mmol), and the obtained solution was stirred to react at room temperature in the dark overnight. At the end of the reaction, the reaction solution was rotary evaporated to obtain an oily solution, and methyl tert-butyl ether (60 mL) was then added to the oily solution. A powdery solid was separated out of the obtained solution, and suction filtering was carried out. The filter cake was collected, and dissolved with a mixed solvent (100 mL) of methanol/ dichloromethane (1: 4), silica gel powder (20 g) was added, and the obtained mixture was then evaporated to dryness to obtain a powdery solid. The operations of dry sample loading, column chromatography and gradient elution with a dichloromethane mixed solution containing 3%-10% methanol were carried out. The elution product was then collected, concentrated, and dried in a vacuum oven, thus obtaining the product 3g, yield 38.96%.

38-191

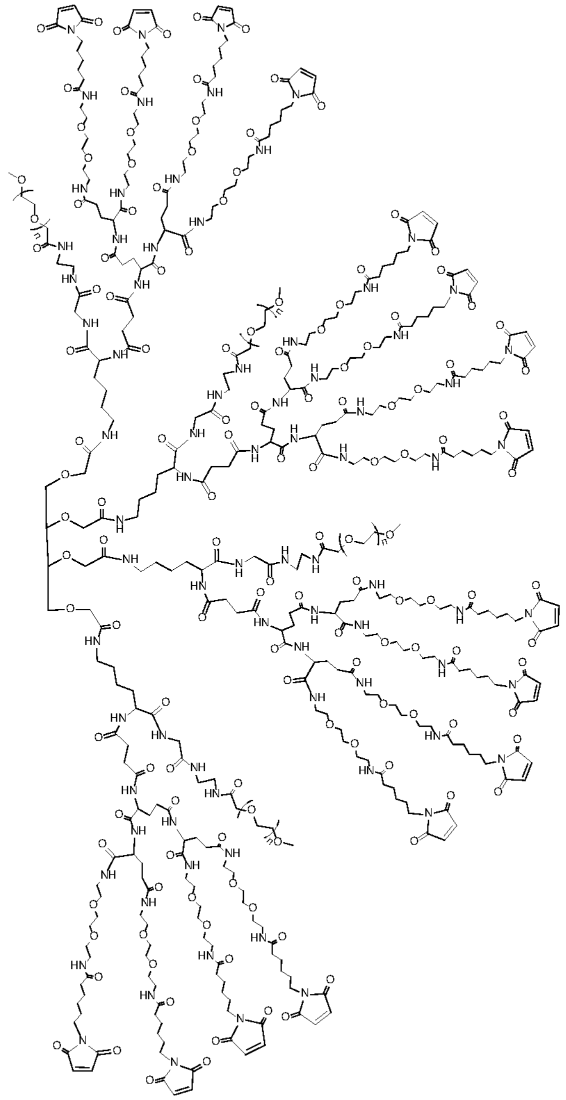

38-153 (1g, 0.0221mmol), 38-179 (0.1508g, 0.442mmol), HBTU (0.2012g, 0.5304mmol), HOBT (0.0717g, 1.5912mmol) were added in a 500 mL flask, and dissolved with DMF (10mL), and the mixed solution was stirred at -5°C for 30 minutes. DIEA (0.2630mL, 1.5912mmol) was slowly added dropwise, and then the mixed solution reacted at -5°C overnight. At the end of the reaction, the reaction solution was extracted with pure water and ethyl acetate. The organic phase was washed with saturated saline solution, concentrated and evaporated to dryness, thus obtaining the product 1 g.

38-192

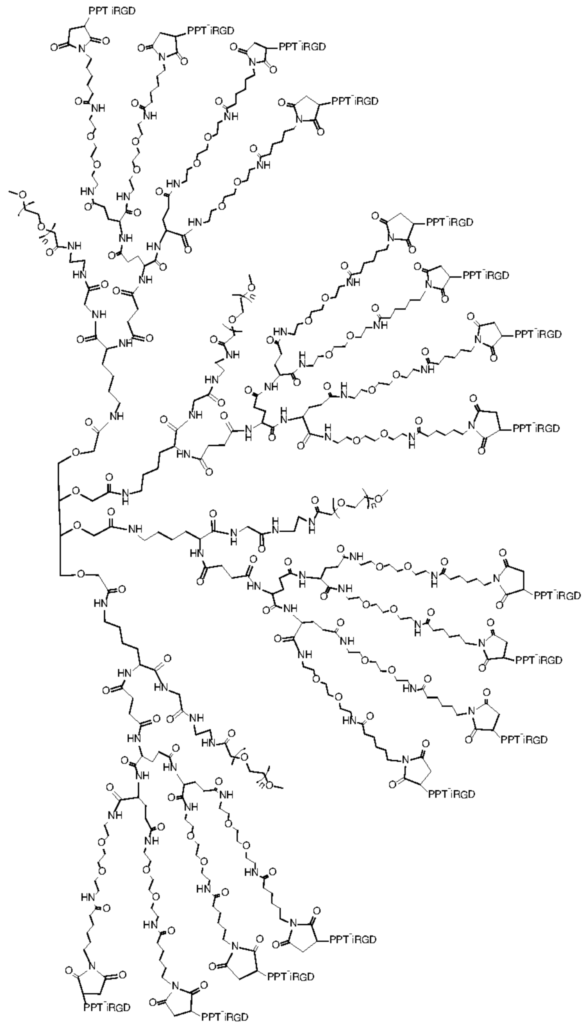

38-191 (1g, 0.0199mmol), PPT-iRGD (0.6370g, 0.3979mmol), were added in a 250 mL flask, and dissolved with DMSO (50mL), and the obtained solution was stirred in the dark under the condition of 40°C for 2 days, and then stirred at room temperature for 3 days. At the end of the reaction, methyl tert-butyl ether was added to the reaction solution, the upper layer solution was discarded, and the precipitated solid was dissolved with 10% methanol/dichloromethane, and then silica gel powder (5g) was added. The mixture was dried, loaded on silica gel column, and eluted with 5%-10% methanol/dichloromethane. 0.8g of the product was obtained with a yield of 50%.

$^1$H-NMR (600 MHz, DMSO-$d_6$) $\delta$ 8.44-8.33 (m, 64H), 8.25-8.18 (m, 47H), 7.29-7.22( m, 144H), 4.90-4.79 (m, 8H), 4.66-4.45 (m, 101H), 4.38-4.19 (m, 194H), 3.80-3.71 (m, 83H), 3.55-3.45 (m, 3843H), 3.20-3.04 (m, 208H), 2.94-2.86(m, 72H), 2.83-2.72 (m, 153H), 2.35-2.31(m, 20H), 2.06-2.02(m, 29H), 1.92-1.83(m, 189H), 1.68-1.42(m, 435H), 1.30-1.21( m, 155H), 0.88-0.83(m, 192H).

Synthesis of 52-95:

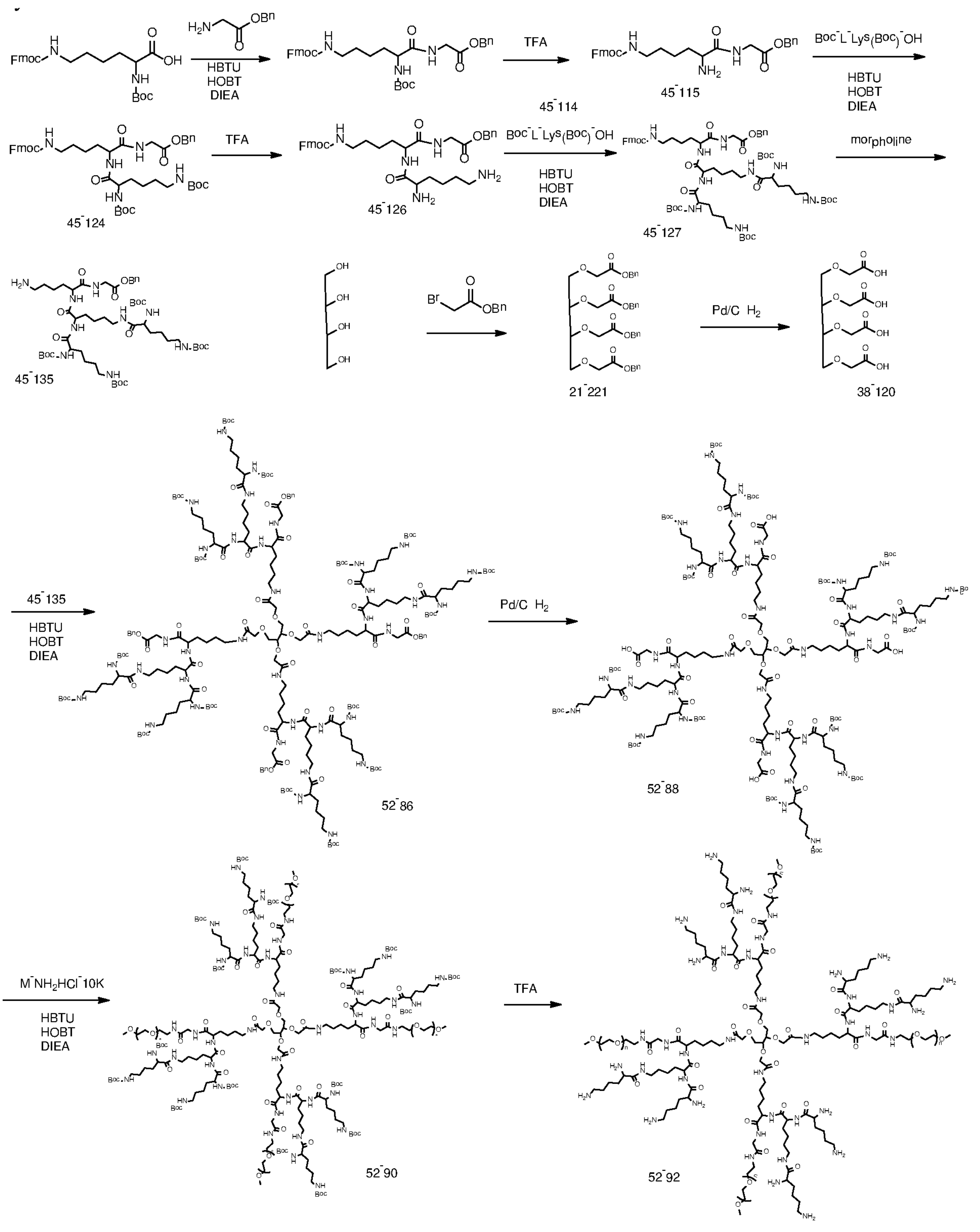
HBTU
HOBT
DIEA
45-114
TFA
45-115
Boc-L-Lys(Boc)-OH
HBTU
HOBT
DIEA
45-124
TFA
45-126
Boc-L-Lys(Boc)-OH
HBTU
HOBT
DIEA
45-127
morpholine
45-135
21-221
Pd/C $H_2$
38-120
45-135
HBTU
HOBT
DIEA
52-86
Pd/C $H_2$
52-88
M-$NH_2$HCl-10K
HBTU
HOBT
DIEA
52-90
TFA
52-92

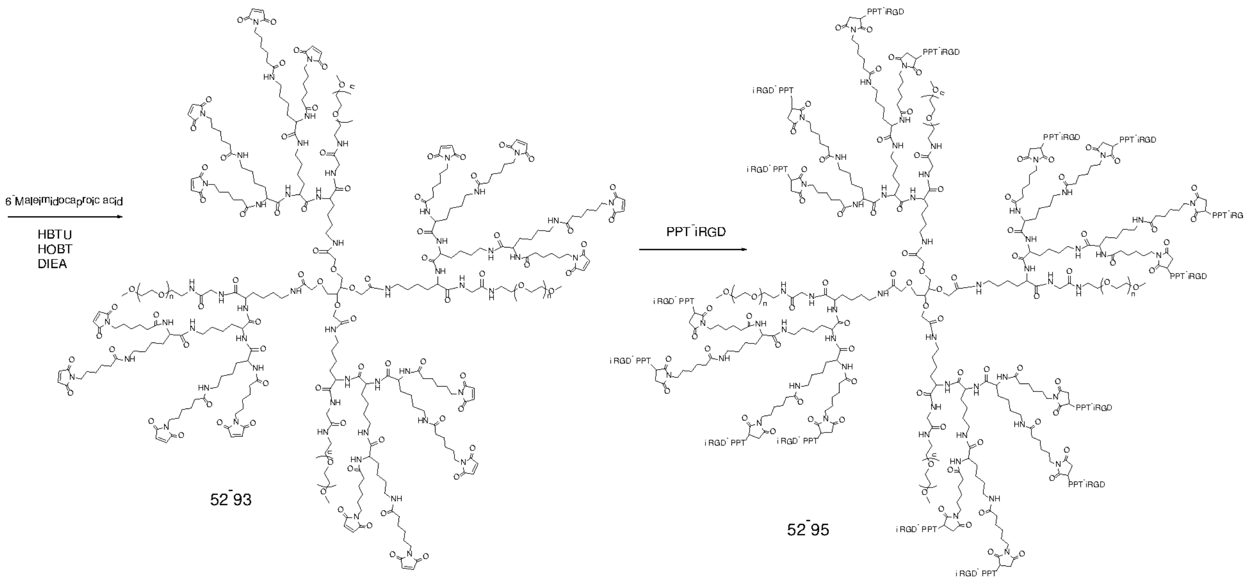


52-93

52-95

45-114

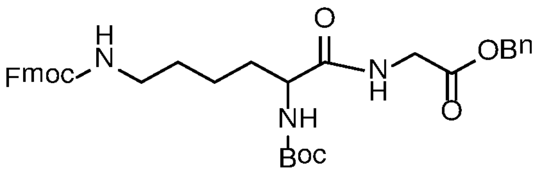

Boc-L-Lys(Fmoc)-OH (20g, 42.6857mmol), Gly-OBn (14.4g, 42.6857mmol), HBTU (24g, 64.0286mmol), HOBT (8.65g, 64.0286mmol) were added in a 500 mL flask, and dissolved with DMF (40 mL), and the mixed solution was stirred at -5°C for 30 minutes. DIEA (31.7mL, 192.085mmol) was slowly added dropwise, and then the mixed solution reacted at -5°C overnight. At the end of the reaction, the reaction solution was extracted with pure water and ethyl acetate. The organic phase was washed with saturated saline solution, concentrated and evaporated to dryness, thus obtaining the product 26 g.

45-115

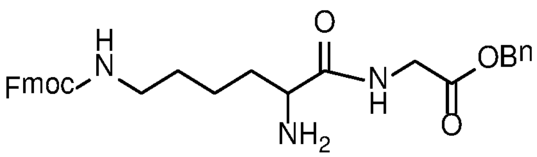

Compound 45-114 (26g, 42.6857mmol) was dissolved with dichloromethane (20mL) and TFA (14mL, 190.2855mmol) in a condition of ultrasonic, and then the mixed solution was stirred to react. At the end of the reaction, the reaction solution was concentrated. The concentrated product was dissolved with ethyl acetate (100 mL), and a saturated sodium bicarbonate solution was added until the aqueous phase became alkaline. The organic phase was separated, and the aqueous phase was extracted three times with ethyl acetate (50mL×3) until there was no product in the aqueous phase. The obtained organic phases were combined, then washed once with saturated saline solution (50mL), and evaporated to dryness, thus obtaining the product 22g.

45-124

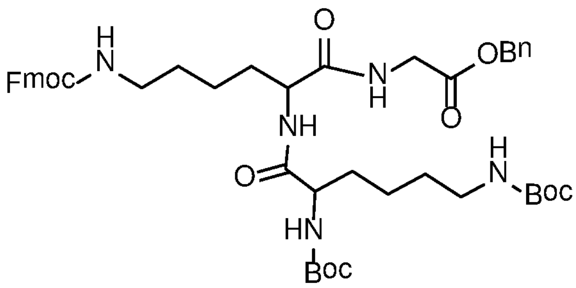

Boc-L-Lys(Boc)-OH (7.3g, 21.334mmol), 45-115 (11g, 21.334mmol), HBTU (12.1363g, 32.0016mmol), HOBT (4.3241g, 32.0016mmol) were added in a 500 mL flask, and dissolved with DMF (80 mL), and the mixed solution was stirred at -5°C for 30 minutes. DIEA (15mL, 96.003mmol) was slowly added dropwise, and then the mixed solution reacted at -5°C overnight. At the end of the reaction, the reaction solution was extracted with pure water and ethyl acetate. The organic phase was washed with saturated saline solution, and concentrated. The operations of dry sample loading, column chromatography, and gradient elution with 50%-100% ethyl acetate/petroleum ether were carried out, thus obtaining the product 15 g, yield 83%.

45-126

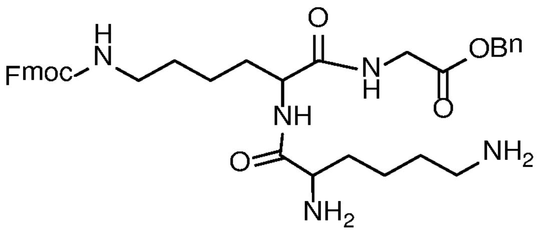

Compound 45-124 (15g, 17.7725mmol) was dissolved with dichloromethane (30mL) and TFA (39mL, 533.1754mmol) in a condition of ultrasonic, and then the mixed solution was stirred to react. At the end of the reaction, the reaction solution was concentrated. The concentrated product was dissolved with ethyl acetate (100 mL), and a saturated sodium bicarbonate solution was added until the aqueous phase became alkaline. Then, the organic phase was separated, and the aqueous phase was extracted three times with ethyl acetate (150mL×3) until there was no product in the aqueous phase. The obtained organic phases were combined, then washed once with saturated saline solution (150mL), and evaporated to dryness, thus obtaining the product 11.44g.

45-127

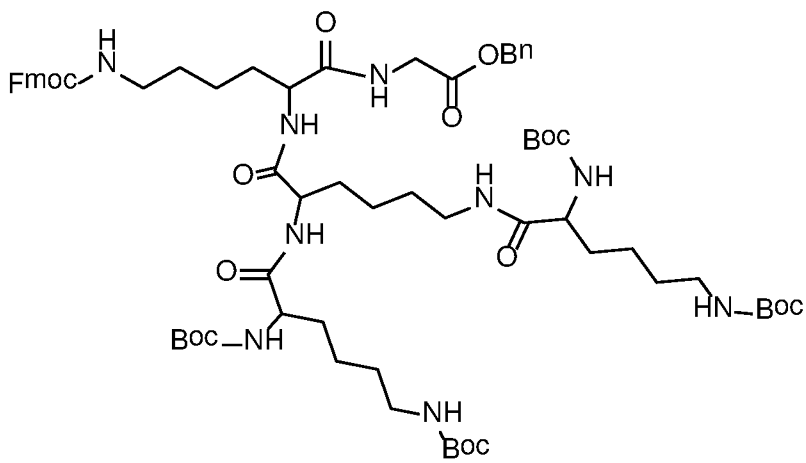

Boc-L-Lys(Boc)-OH (13.022g, 37.5911mmo1), 45-126 (11.4g, 17.0868mmol), HBTU (19.5538g, 51.5604mmol), HOBT (6.9668g, 51.5604mmol) were added in a 500 mL flask, and dissolved with DMF (80 mL), and the mixed solution was stirred at -5°C for 30 minutes. DIEA (25.4mL, 153.7812mmol) was slowly added dropwise, and then the mixed solution reacted at -5°C overnight. At the end of the reaction, the reaction solution was extracted with

pure water and ethyl acetate. The organic phase was washed with saturated saline solution, and concentrated. The operations of dry sample loading, column chromatography, and gradient elution with 1%-55% methanol/ dichloromethane were carried out, thus obtaining the product 18 g, yield 81%.

45-135

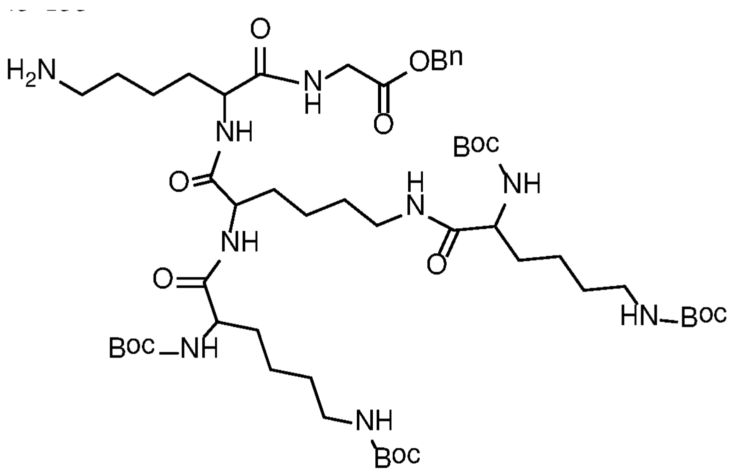

Reactant 45-127 (1.1g, 0.8458mmol) was added in a 250 mL flask, and dissolved with DMF (50mL), morpholine (1.4745mL, 16.9155mmoL) was added, and then the mixed solution was stirred to react at room temperature for 3 hours. At the end of the reaction, saturated saline solution (150mL) and ethyl acetate (200mL) were added to the reaction solution, and the organic phase was separated. The aqueous phase was extracted three times with ethyl acetate (150mL×3) until there was no product in the aqueous phase. The obtained organic phases were combined, washed two times with saturated saline solution (150mLX2), and evaporated to dryness, thus obtaining the product 0.912 g.

21-221

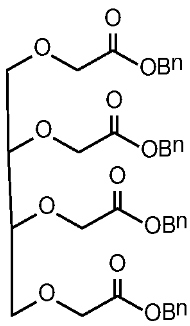

Erythritol (4.5g, 36.849mmol) was added in a 500 mL two-neck flask, and dissolved with THF (60mL) in a condition of ultrasonic, and then the obtained solution was stirred at 0°C. After introducing nitrogen for protective purpose, potassium tert-butoxide (200mL, 184.245mmol) was added, and the obtained solution was stirred at 0°C for 2 hours. Then, benzyl bromoacetate (29.187mL, 184.245mmol) was added, and the obtained solution was stirred for 3 hours and then reacted at room temperature. At the end of the reaction, the reaction solution was extracted with pure water and ethyl acetate, and the organic phase was concentrated. The operations of dry sample loading, column chromatography and gradient elution with 1%-2% ethyl acetate/petroleum ether were carried out, thus obtaining the product 5g, yield 20%.

38-120

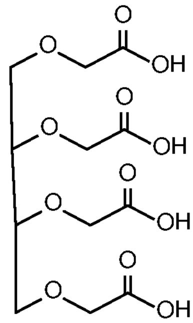

21-221 (0.64g, 0.9793mmol) and 10%Pd/C (100mg) were added in a hydrogenation reactor, DMF (30mL) was slowly added to dissolve the reactant with stirring, hydrogen was introduced to a pressure of 300 psi, and then the mixed solution was stirred to react at room temperature overnight. Next day, the reaction solution was filtered by suction through a sand core funnel filled with diatomaceous earth to remove the Pd/C, thus obtaining the DMF solution of the product, directly used for next reaction.
52-86

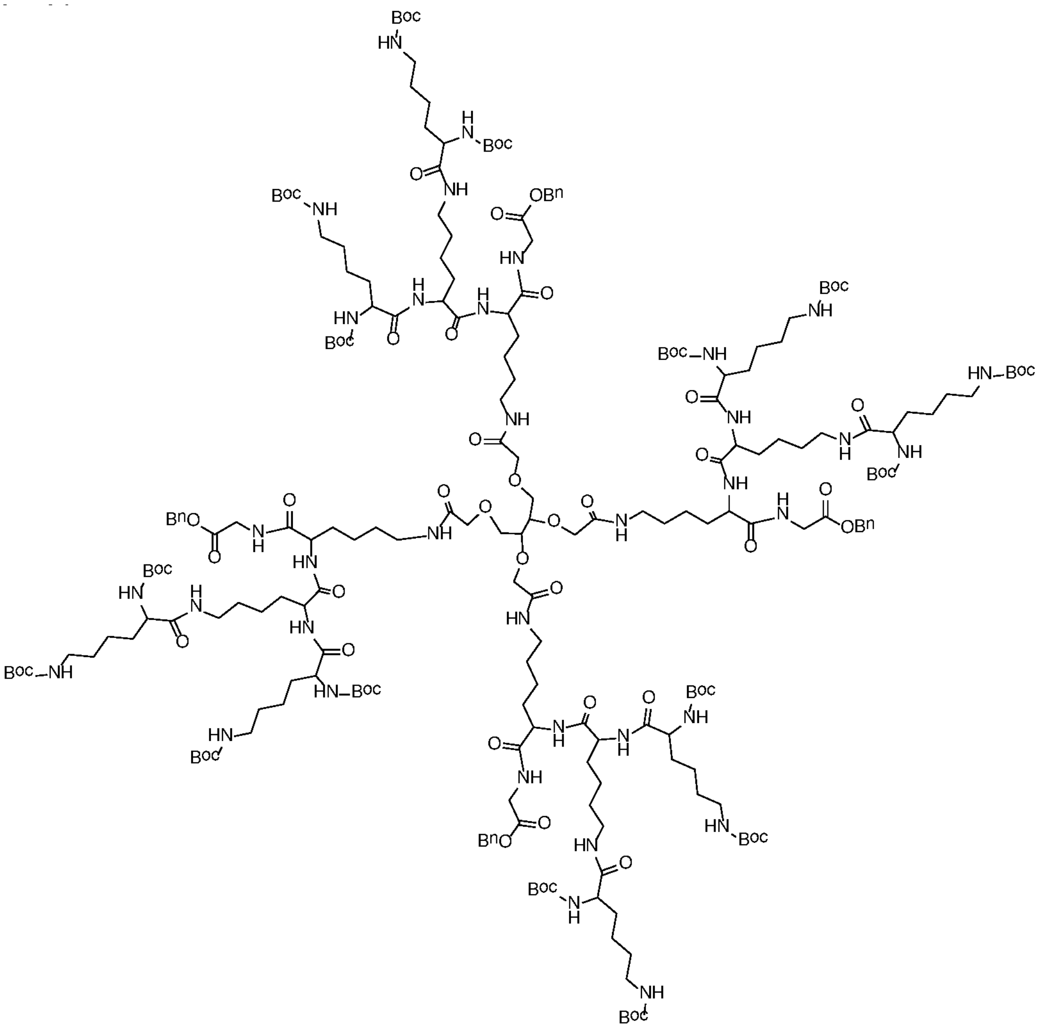

45-135 (0.5g, 0.46mmol), HBTU (0.22g, 0.576mmol), HOBT (0.08g, 0.576mmol) were added in a 250 mL flask, and dissolved with the DMF solution of 38-120 (0.096mmol), and the mixed solution was stirred at -5°C for 30 minutes. DIEA (0.3mL, 1.728mmol) was slowly added dropwise, and then the mixed solution reacted at -5°C overnight. At the end of the reaction, pure water was added to the reaction solution, and suction filtering was carried out. The operations of dry sample loading, column chromatography and elution with 2% methanol/dichloromethane were

carried out, thus obtaining the product 0.32g, yield 50%.

52-88

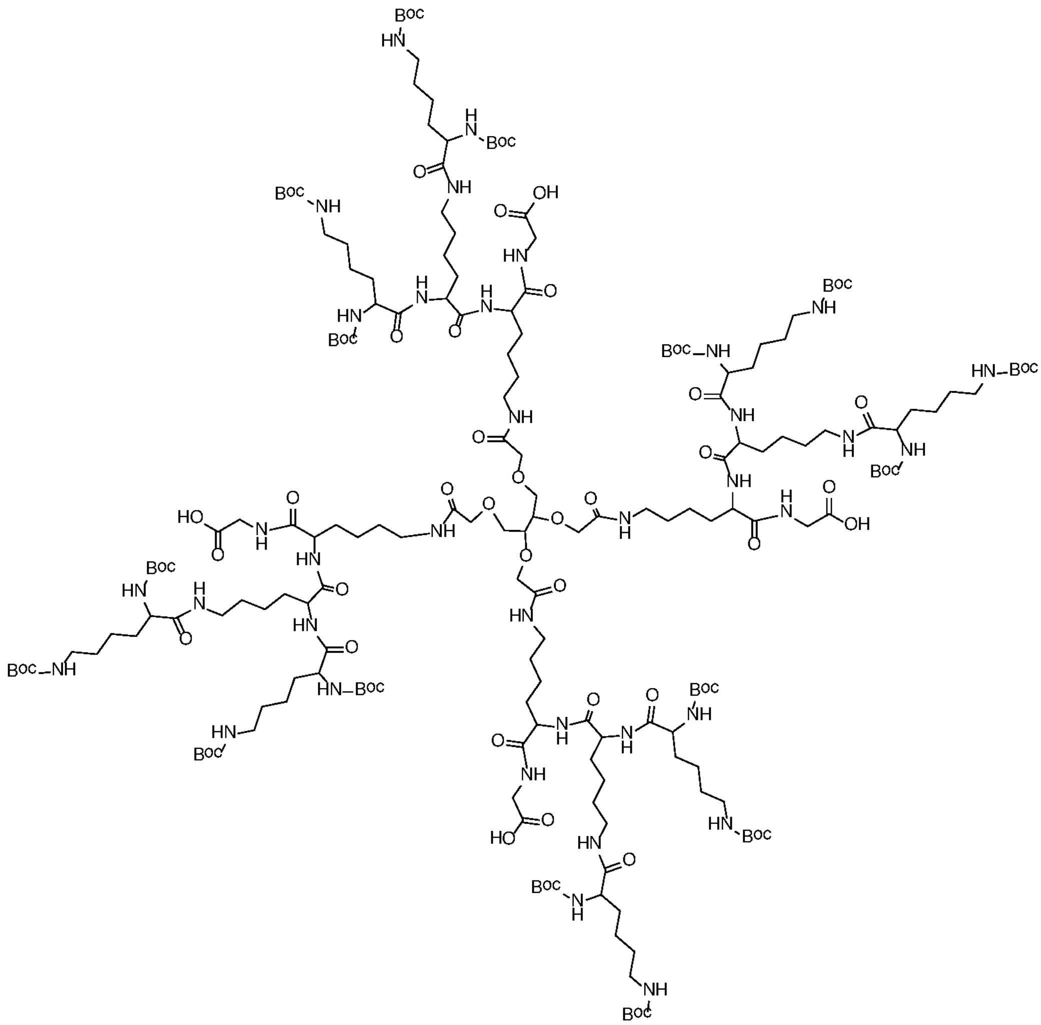

52-86 (0.32g, 0.0694mmol) and 10%Pd/C (75mg) were added in a hydrogenation reactor, DMF (50mL) was slowly added to dissolve the reactant with stirring, hydrogen was introduced to a pressure of 2 MPa, and then the mixed solution was stirred to react at room temperature overnight. Next day, the reaction solution was filtered by suction through a sand core funnel filled with diatomaceous earth to remove the Pd/C, thus obtaining the DMF solution of the product, directly used for next reaction.

52-90

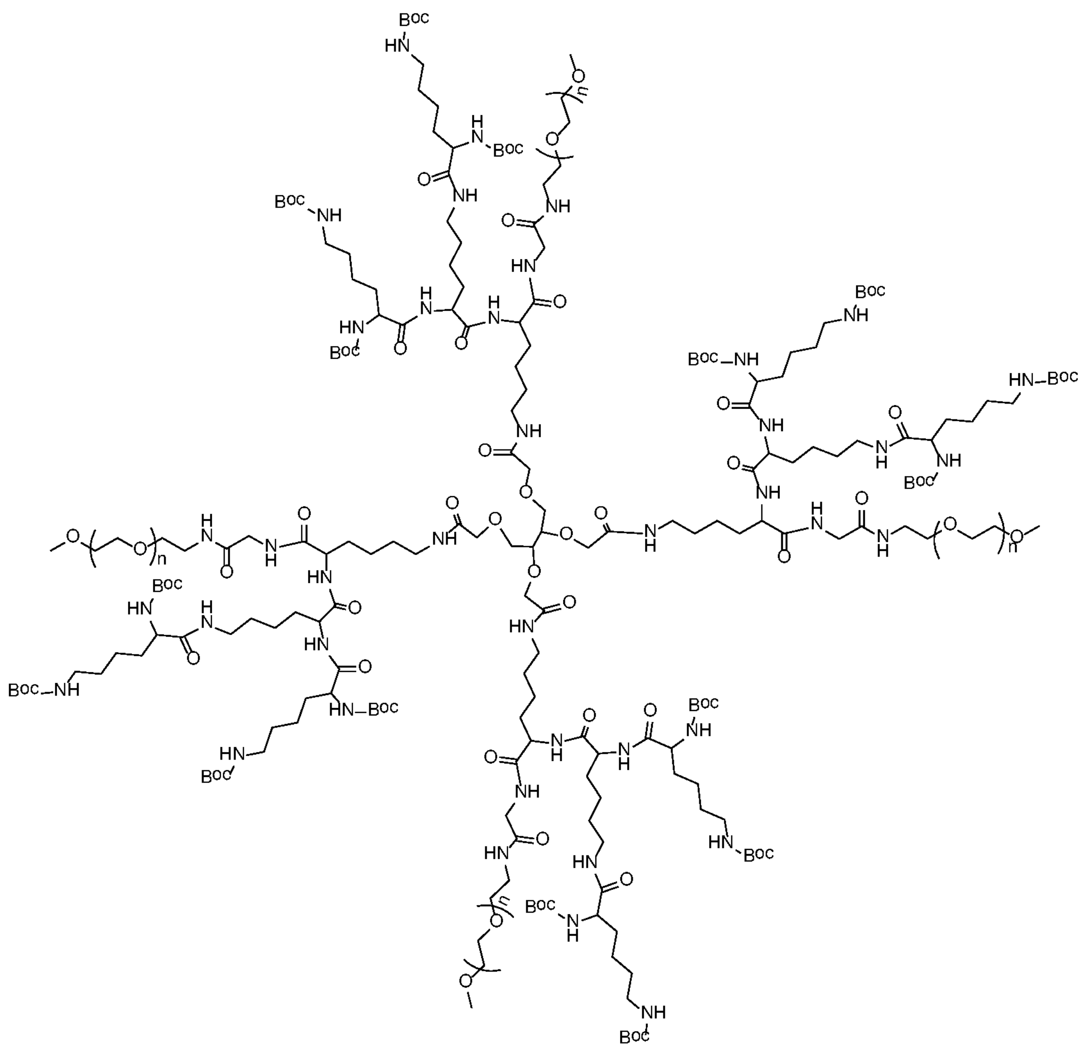

M-$NH_2$HCl-10K (3.065g, 0.2914mmol), HBTU (0.1579g, 0.4164mmol), HOBT (0.0562g, 0.4164mmol) were added in a 250 mL flask, and dissolved with the DMF solution of 52-88 (0.0667mmol), and the mixed solution was stirred at -5°C for 30 minutes. Then DIEA (0.257mL, 1.5545mmol) was slowly added dropwise, and the mixed solution reacted under the condition of -5°C for 1 hour and then moved to room temperature and reacted in the dark at a low speed of stirring. At the end of the reaction, methyl tert-butyl ether was added to the reaction solution, and suction filtering was carried out. The operations of dry sample loading, column chromatography and elution with 5%-6% methanol/dichloromethane were carried out, thus obtaining the product 1.2g.

52-92

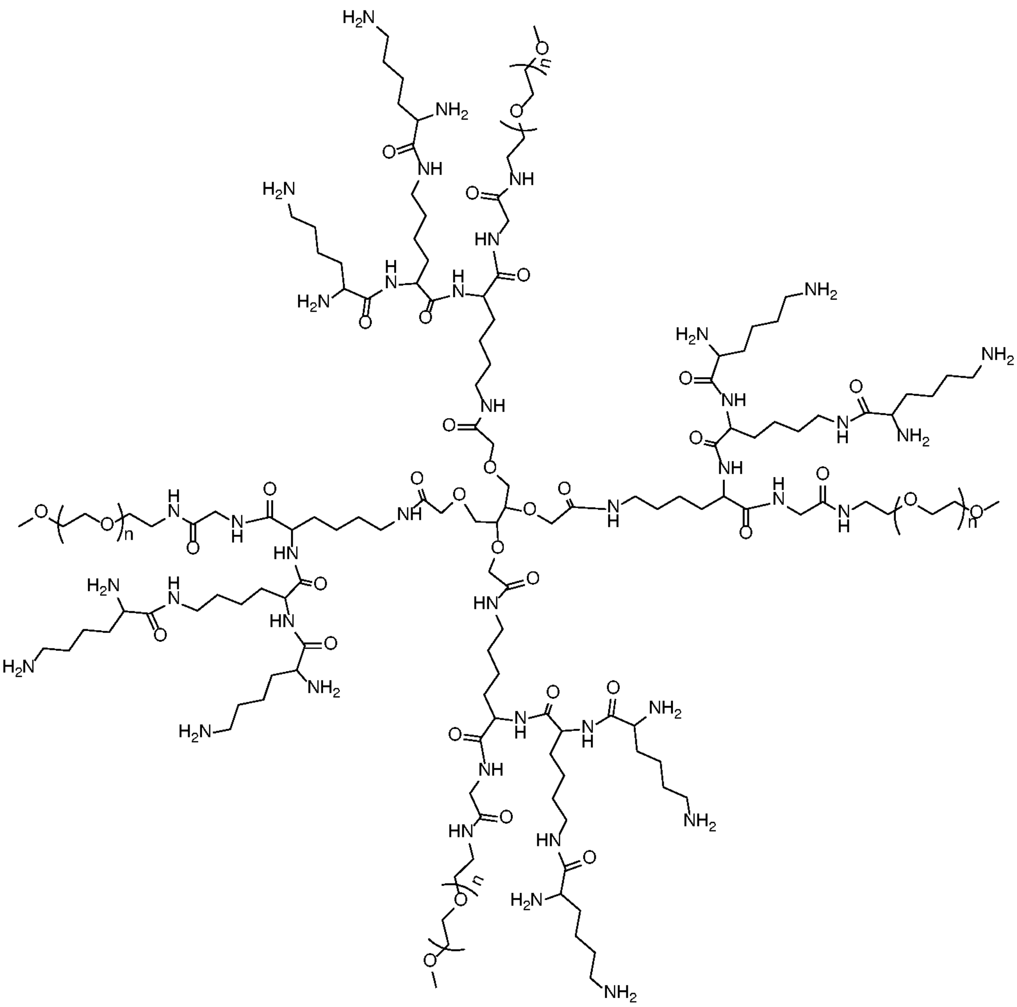

Compound 52-90 (1.5g, 0.0325mmol) was dissolved with dichloromethane (10mL) and TFA (0.78mL, 10.4mol) in a condition of ultrasonic, and then the mixed solution was stirred to react. At the end of the reaction, methanol (30mL) and an excess amount of potassium carbonate were added to the reaction solution, and the mixed solution was stirred for 30 minutes. The operations of suction filtering and drying were carried out, thus obtaining the product 2g.
52-93

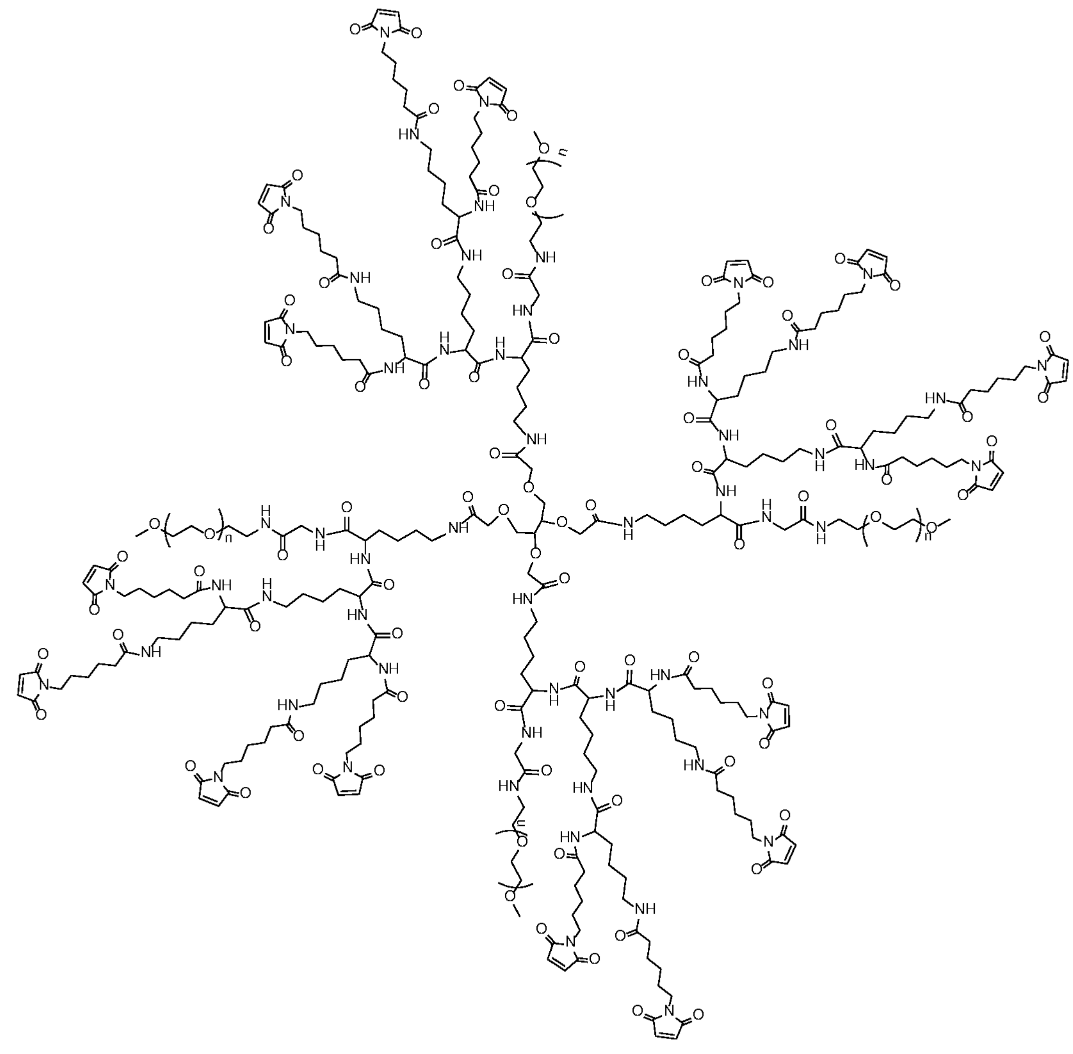

52-92(0.0325mmol), 6-maleimidohexanoic acid (0.137g, 0.65mmol), HBTU (0.295g, 0.78mmol), HOBT (0.105g, 0.78mmol) were added in a 250 mL flask, and dissolved with DMF, and the obtained solution was stirred in the dark under the condition of -5°C for 30 minutes. Then DIEA (0.4mL, 2.34mmol) was slowly added dropwise, and the mixed solution reacted under the condition of -5°C for 1 hour and then moved to room temperature and reacted in the dark. At the end of the reaction, methyl tert-butyl ether was added to the reaction solution to make the product precipitated. The precipitated product was dissolved and then precipitated several times to provide 1.5g of the product.
52-95

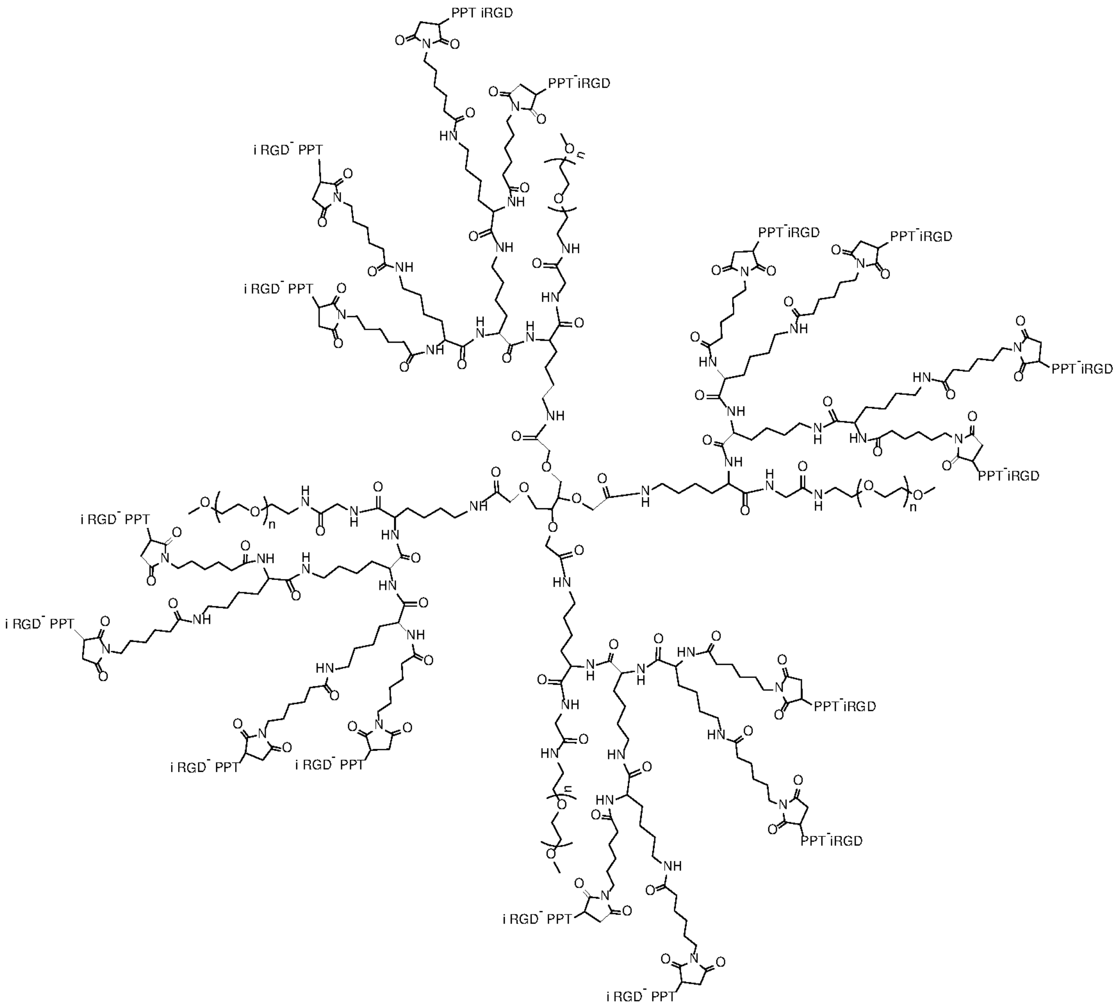

52-93 (0.0325mmol), PEPTIDE-iRGD (1.04g, 0.65mmol), were added in a 250 mL flask, and dissolved with DMSO (50mL), and the obtained solution was stirred in the dark under the condition of 40°C for 2 days, and then stirred at room temperature for 3 days. At the end of the reaction, methyl tert-butyl ether was added to the reaction solution, the upper layer solution was discarded, and the precipitated solid was dissolved with 10% methanol/dichloromethane, and then silica gel powder (5g) was added. The mixture was dried, loaded on silica gel column, and eluted with 5%-25% methanol/dichloromethane. 0.3g of the product was obtained with a yield of 44%. Molecular weight range determined by MALDI-TOF MS was 72500-74070.

Synthesis of 56-89:

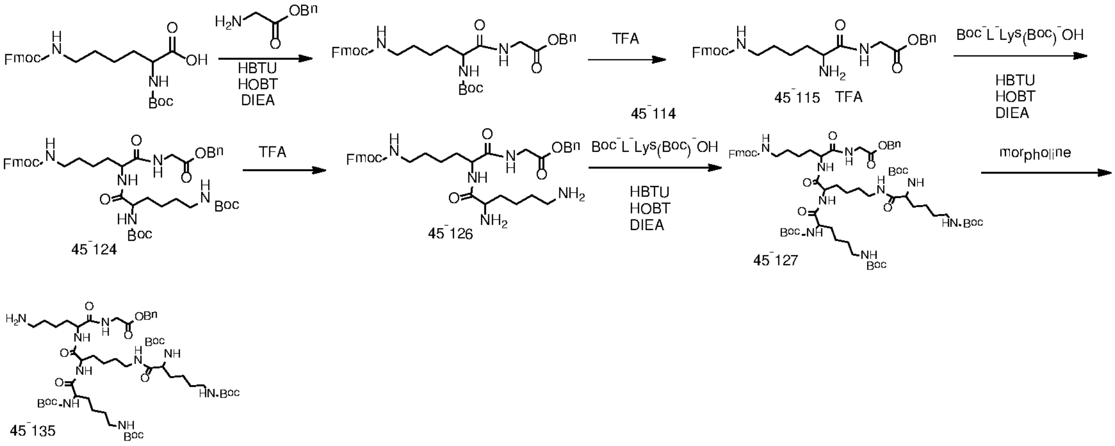
HBTU
HOBT
DIEA
TFA
45-114
45-115 TFA
Boc-L-Lys(Boc)-OH
HBTU
HOBT
DIEA
45-124
TFA
45-126
Boc-L-Lys(Boc)-OH
HBTU
HOBT
DIEA
45-127
morpholine
45-135

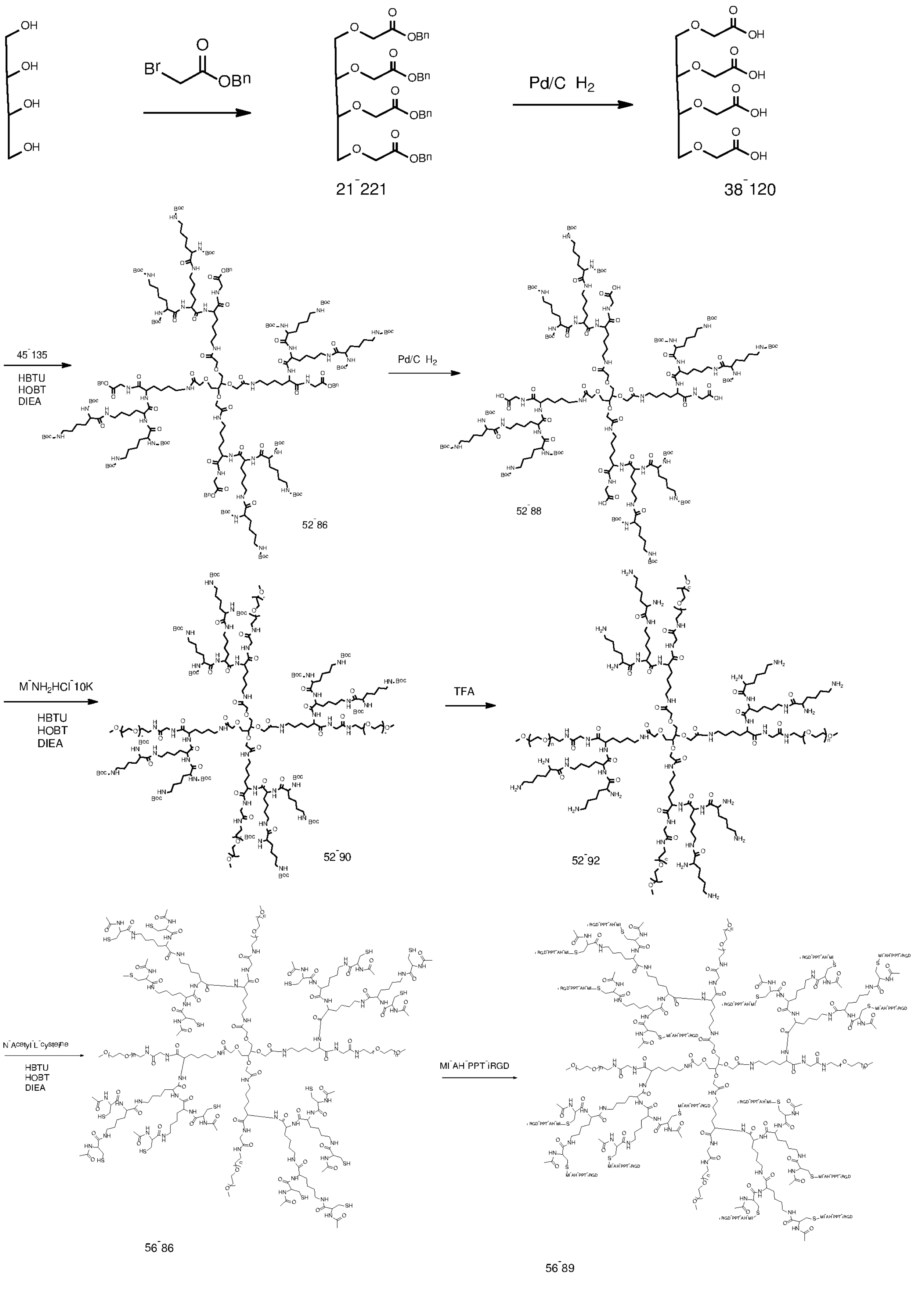


21-221

38-120

52-86

52-88

52-90

52-92

56-86

56-89

45-114

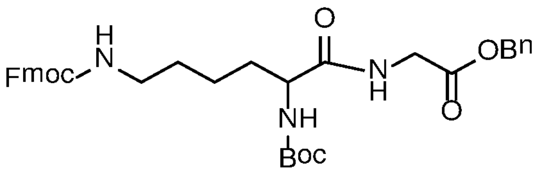

Boc-L-Lys(Fmoc)-OH (20g, 42.6857mmol), Gly-OBn (14.4g, 42.6857mmol), HBTU (24g, 64.0286mmol), HOBT (8.65g, 64.0286mmol) were added in a 500 mL flask, and dissolved with DMF (40 mL), and the mixed solution was stirred at -5°C for 30 minutes. DIEA (31.7mL, 192.085mmol) was slowly added dropwise, and then the mixed solution reacted at -5°C overnight. At the end of the reaction, the reaction solution was extracted with pure water and ethyl acetate. The organic phase was washed with saturated saline solution, concentrated and evaporated to dryness, thus obtaining the product 26 g.

45-115

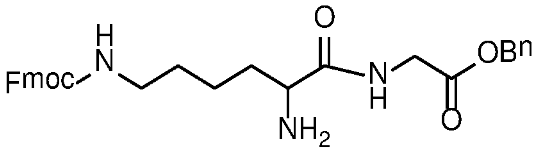

Compound 45-114 (26g, 42.6857mmol) was dissolved with dichloromethane (20mL) and TFA (14mL, 190.2855mmol) in a condition of ultrasonic, and then the mixed solution was stirred to react. At the end of the reaction, the reaction solution was concentrated. The concentrated product was dissolved with ethyl acetate (100 mL), saturated sodium chloride solution was added, the organic phase was separated, and the aqueous phase was extracted three times with ethyl acetate (50mL×3) until there was no product in the aqueous phase. The obtained organic phases were combined, then washed once with saturated saline solution (50mL), and evaporated to dryness, thus obtaining the product 22g.

45-124

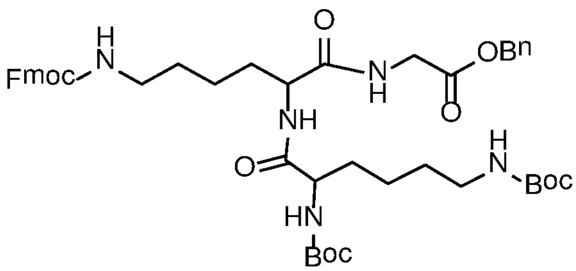

Boc-L-Lys(Boc)-OH (7.3g, 21.334mmol), 45-115 (11g, 21.334mmol), HBTU (12.1363g, 32.0016mmol), HOBT (4.3241g, 32.0016mmol) were added in a 500 mL flask, and dissolved with DMF (80 mL), and the mixed solution was stirred at -5°C for 30 minutes. DIEA (15mL, 96.003mmol) was slowly added dropwise, and then the mixed solution reacted at -5°C overnight. At the end of the reaction, the reaction solution was extracted with pure water and ethyl acetate. The organic phase was washed with saturated saline solution, and concentrated. The operations of dry sample loading, column chromatography, and gradient elution with 50%-100% ethyl acetate/petroleum ether were carried out, thus obtaining the product 15 g, yield 83%.

45-126

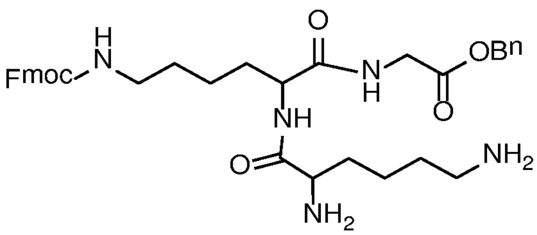

Compound 45-124 (15g, 17.7725mmol) was dissolved with dichloromethane (30mL) and TFA (39mL, 533.1754mmol) in a condition of ultrasonic, and then the mixed solution was stirred to react. At the end of the reaction, the reaction solution was concentrated. The concentrated product was dissolved with ethyl acetate (100 mL), and a saturated sodium bicarbonate solution was added until the aqueous phase became alkaline. Then, the organic phase was separated, and the aqueous phase was extracted three times with ethyl acetate (150mL×3) until there was no product in the aqueous phase. The obtained organic phases were combined, then washed once with saturated saline solution (150mL), and evaporated to dryness, thus obtaining the product 11.44g.

45-127

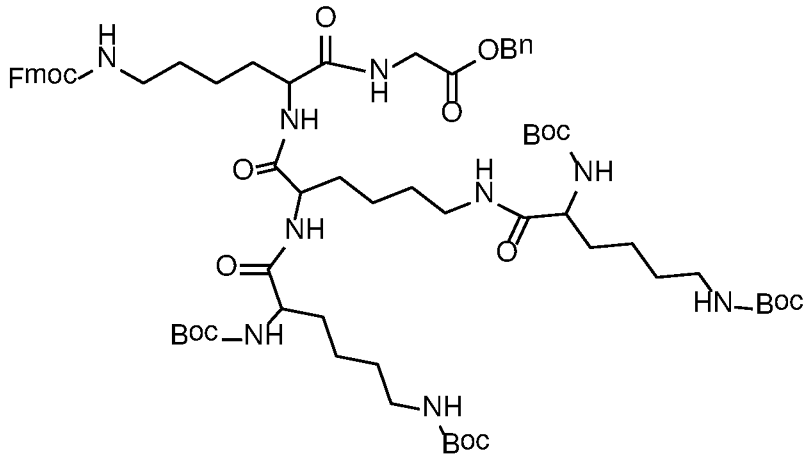

Boc-L-Lys(Boc)-OH (13.022g, 37.5911mmol), 45-126 (11.4g, 17.0868mmol), HBTU (19.5538g, 51.5604mmol), HOBT (6.9668g, 51.5604mmol) were added in a 500 mL flask, and dissolved with DMF (80 mL), and the mixed solution was stirred at -5°C for 30 minutes. DIEA (25.4mL, 153.7812mmol) was slowly added dropwise, and then the mixed solution reacted at -5°C overnight. At the end of the reaction, the reaction solution was extracted with pure water and ethyl acetate. The organic phase was washed with saturated saline solution, and concentrated. The operations of dry sample loading, column chromatography, and gradient elution with 1%-55% methanol/ dichloromethane were carried out, thus obtaining the product 18 g, yield 81%.

45-135

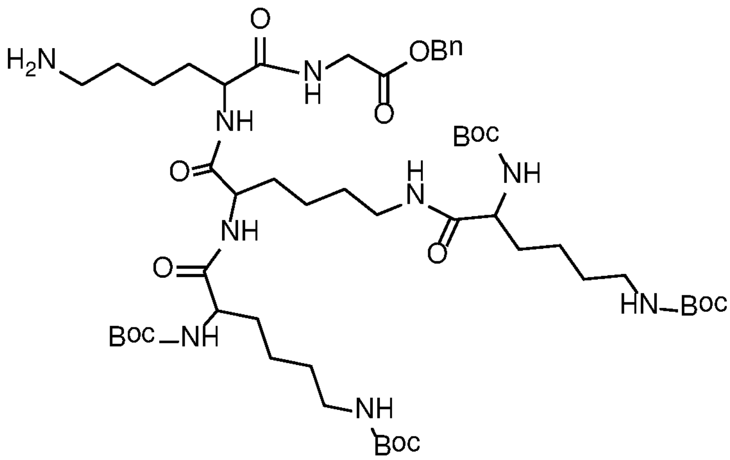

Reactant 45-127 (1.1g, 0.8458mmol) was added in a 250 mL flask, and dissolved with DMF (50mL), morpholine (1.4745mL, 16.9155mmoL) was added, and then the mixed solution was stirred to react at room temperature for 3 hours. At the end of the reaction, saturated saline solution (150mL) and ethyl acetate (200mL) were added to the reaction solution, and the organic phase was separated. The aqueous phase was extracted three times with ethyl acetate (150mL×3) until there was no product in the aqueous phase. The obtained organic phases were combined, washed two times with saturated saline solution (150mLX2), and evaporated to dryness, thus obtaining the product 0.912 g.

21-221

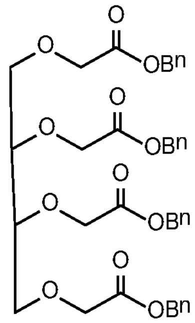

Erythritol (4.5g, 36.849mmol) was added in a 500 mL two-neck flask, and dissolved with THF (60mL) in a condition of ultrasonic, and then the obtained solution was stirred at 0°C. After introducing nitrogen for protective purpose, potassium tert-butoxide (200mL, 184.245mmol) was added, and the obtained solution was stirred at 0°C for 2 hours. Then, benzyl bromoacetate (29.187mL, 184.245mmol) was added, and the obtained solution was stirred for 3 hours and then reacted at room temperature. At the end of the reaction, the reaction solution was extracted with pure water and ethyl acetate, and the organic phase was concentrated. The operations of dry sample loading, column chromatography and gradient elution with 1%-2% ethyl acetate/petroleum ether were carried out, thus obtaining the product 5g, yield 20%.

38-120

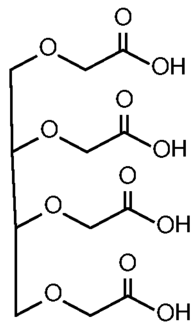

21-221 (0.64g, 0.9793mmol) and 10%Pd/C (100mg) were added in a hydrogenation reactor, DMF (30mL) was slowly added to dissolve the reactant with stirring, hydrogen was introduced to a pressure of 300 psi, and then the mixed solution was stirred to react at room temperature overnight. Next day, the reaction solution was filtered by suction through a sand core funnel filled with diatomaceous earth to remove the Pd/C, thus obtaining the DMF solution of the product, directly used for next reaction.

52-86

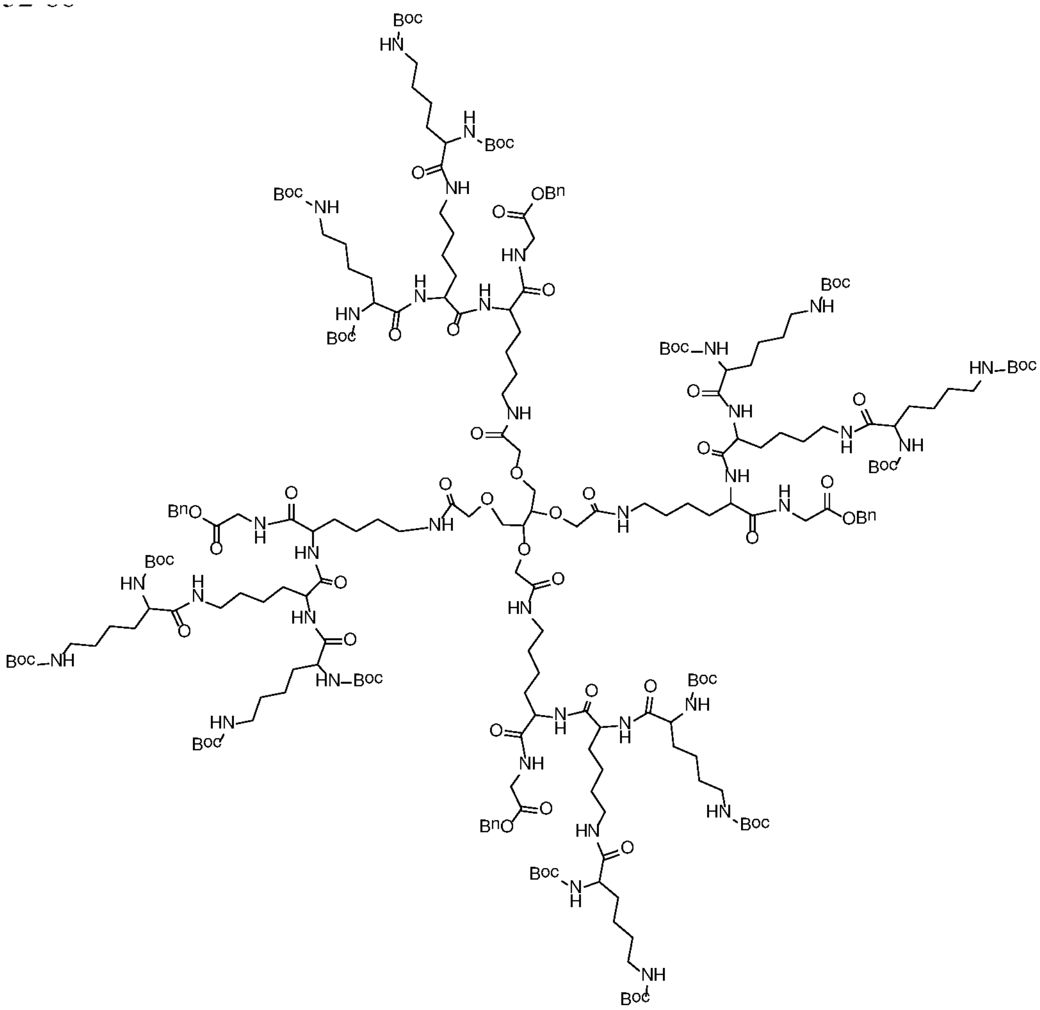

45-135 (0.5g, 0.46mmol), HBTU (0.22g, 0.576mmol), HOBT (0.08g, 0.576mmol) were added in a 250 mL flask, and dissolved with the DMF solution of 38-120 (0.096mmol), and the mixed solution was stirred at -5°C for 30 minutes. DIEA (0.3mL, 1.728mmol) was slowly added dropwise, and then the mixed solution reacted at -5°C overnight. At the end of the reaction, pure water was added to the reaction solution, and suction filtering was carried out. The operations of dry sample loading, column chromatography and elution with 2% methanol/dichloromethane were carried out, thus obtaining the product 0.32g, yield 50%.

52-88

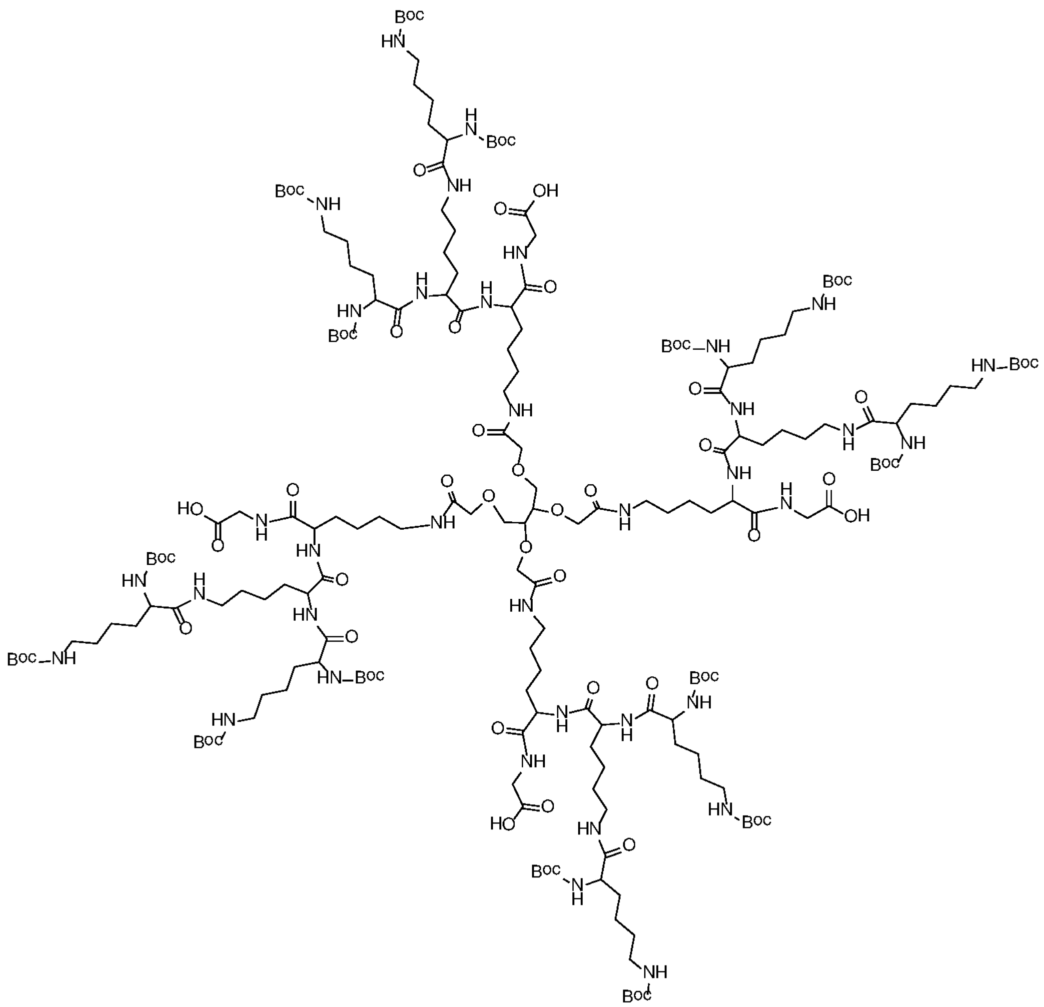

52-86 (0.32g, 0.0694mmol) and 10%Pd/C (75mg) were added in a hydrogenation reactor, DMF (50mL) was slowly added to dissolve the reactant with stirring, hydrogen was introduced to a pressure of 2 MPa, and then the mixed solution was stirred to react at room temperature overnight. Next day, the reaction solution was filtered by suction through a sand core funnel filled with diatomaceous earth to remove the Pd/C, thus obtaining the DMF solution of the product, directly used for next reaction.

52-90

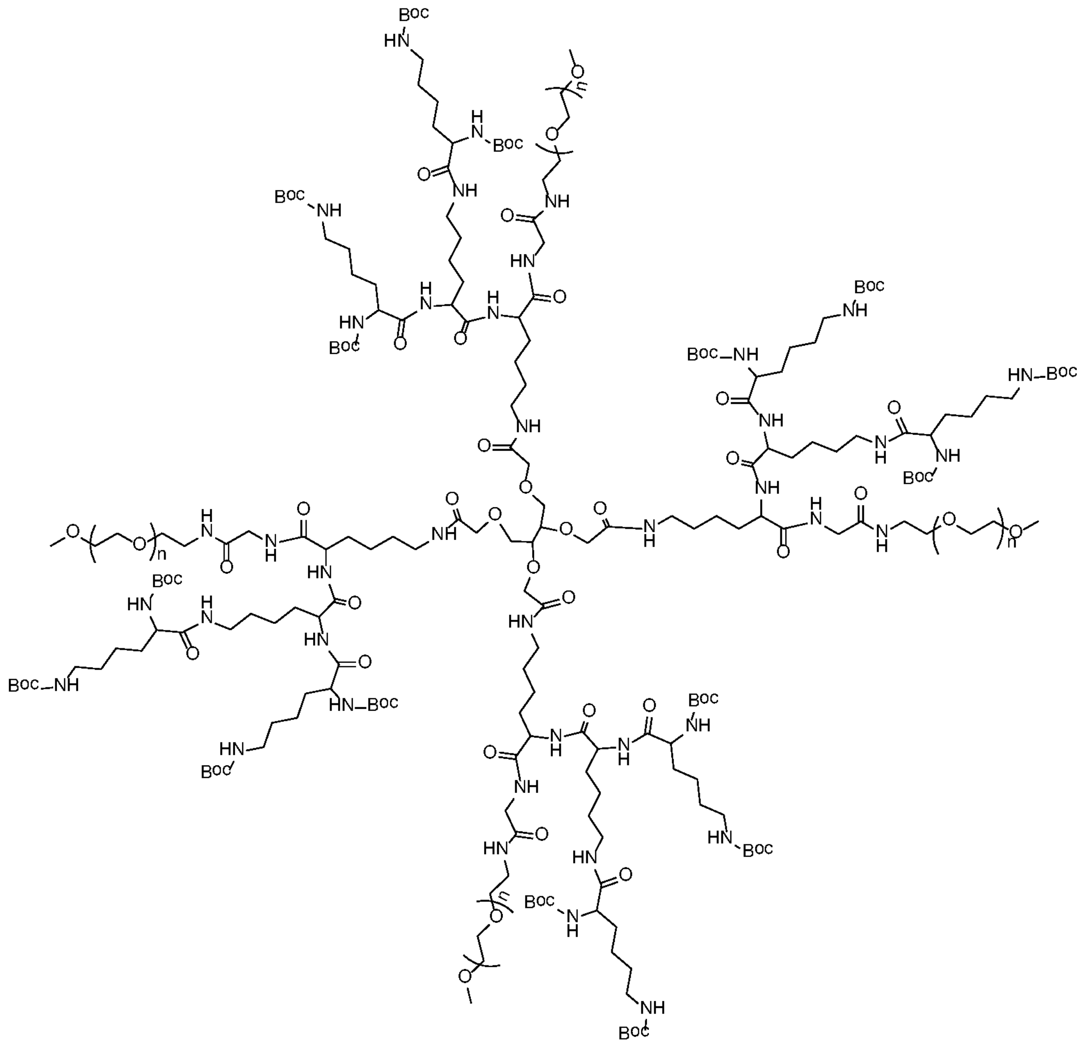

M-$NH_2$HCl-10K (3.065g, 0.2914mmol), HBTU (0.1579g, 0.4164mmol), HOBT (0.0562g, 0.4164mmol) were added in a 250 mL flask, and dissolved with the DMF solution of 52-88 (0.0667mmol), and the mixed solution was stirred at -5°C for 30 minutes. DIEA (0.257mL, 1.5545mmol) was slowly added dropwise, and the mixed solution reacted under the condition of -5°C for 1 hour and then moved to room temperature and reacted in the dark at a low speed of stirring. At the end of the reaction, methyl tert-butyl ether was added to the reaction solution, and suction filtering was carried out. The operations of dry sample loading, column chromatography and elution with 5%-6% methanol/dichloromethane were carried out, thus obtaining the product 1.2g.

$^1$H-NMR (400 MHz, DMSO-$d_6$) δ 7.98-7.88 (m, 14H), 7.71-7.60 (m, 15H), 7.58-7.47 (m, 16H), 7.45-7.31(m, 6H), 5.76 (s, 28H), 3.8-3.35 (m, 3810H), 3.20-3.12(m, 13H), 2.91-2.80 (m, 10H), 2.71-2.60 (m, 14H), 2.33-2.30 (m, 13H), 2.25-2.09 (m, 22H), 1.36-1.28 (m, 51H), 1.20-1.11 (m, 49H), 1.05-1.00 (m, 96H),

52-92

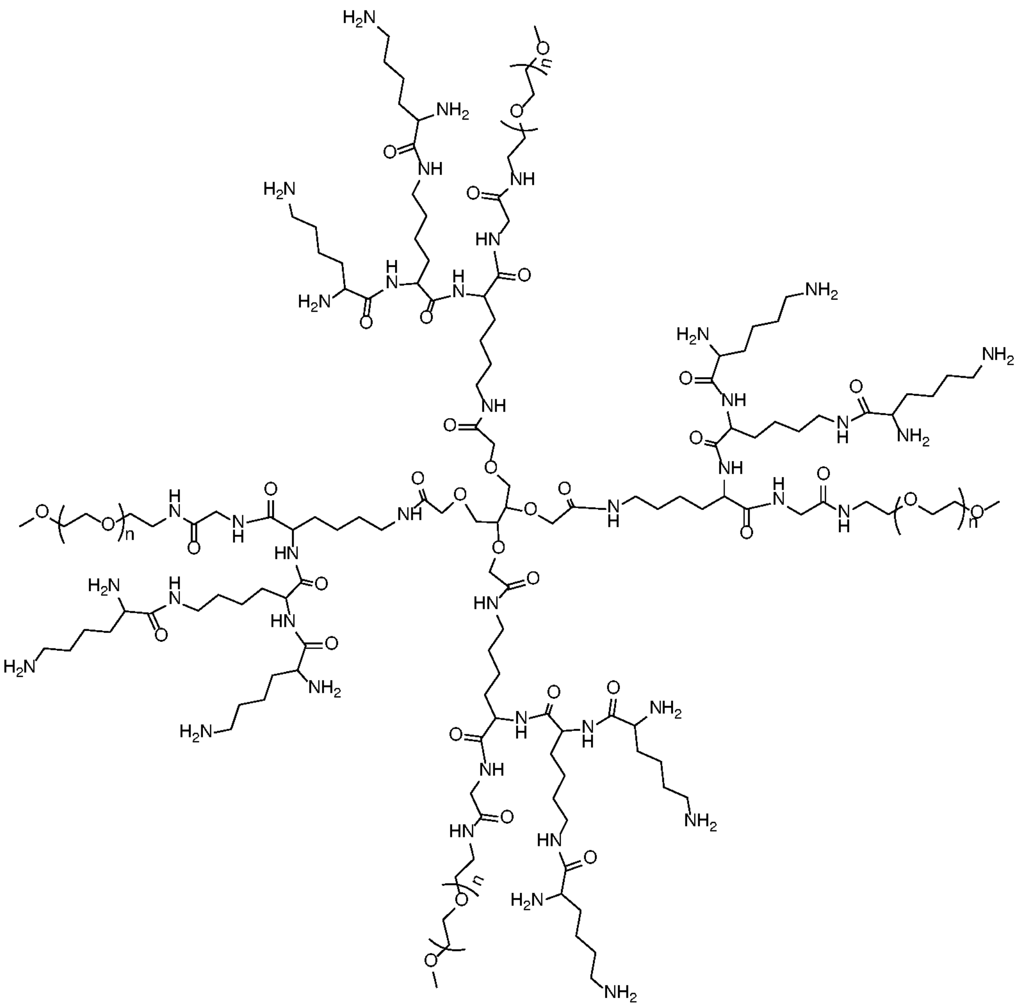

Compound 52-90 (1.5g, 0.0325mmol) was dissolved with dichloromethane (10mL) and TFA (0.78mL, 10.4mol) in a condition of ultrasonic, and then the mixed solution was stirred to react. At the end of the reaction, methanol (30mL) and an excess amount of potassium carbonate were added to the reaction solution, and the mixed solution was stirred for 30 minutes. The operations of suction filtering and drying were carried out, thus obtaining the product 2g. $^1$H-NMR (400 MHz, DMSO-$d_6$) δ 8.25-8.02 (m, 12H), 7.92-7.80 (m, 11H), 7.71-7.65 (m, 2H), 5.76-5.60 (m, 8H), 5.58-5.50 (m, 23H), 3.75-3.70 (m, 27H), 3.60-3.56(m, 35H), 3.51-3.40 (m, 3810H), 2.71-2.65 (m, 12H), 2.60-2.55 (m, 16H), 1.71-1.65 (m, 15H), 1.60-1.55 (m, 28H), 1.52-1.47(m, 17H), 1.16-1.00 (m, 18H)

56-86

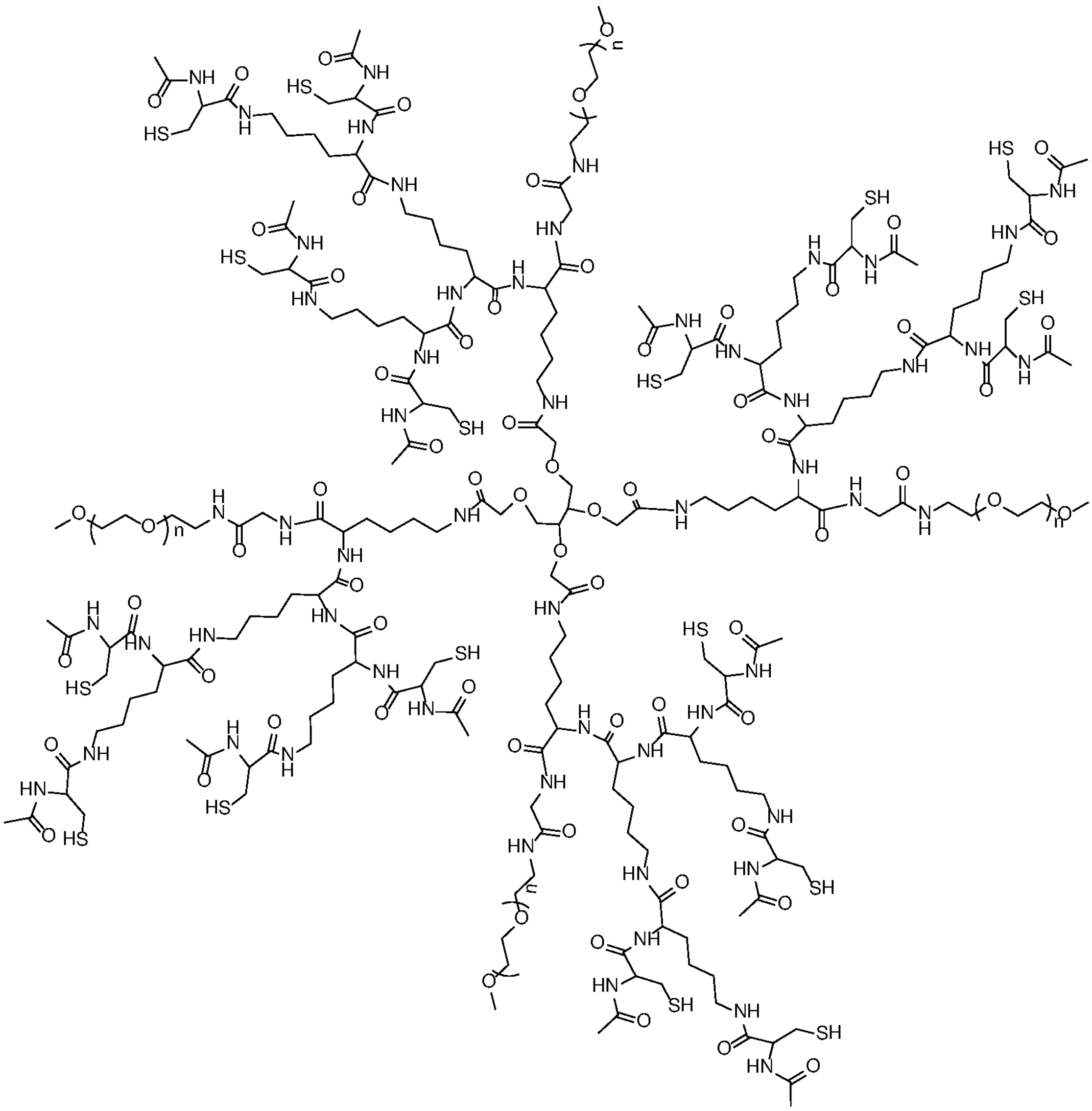

52-92(0.0325mmol), N-acetyl-L-cysteine (0.046g, 0.286mmol, purchased from InnoChem), HBTU (0.147g, 0.39mmol), HOBT (0.052g, 0.39mmol) were added in a 250 mL flask, and dissolved with DMF, and the obtained solution was stirred in the dark under the condition of -5°C for 30 minutes. Then DIEA (0.193mL, 1. 17mmol) was slowly added dropwise, and the mixed solution reacted under the condition of -5°C for 1 hour and then moved to room temperature and reacted in the dark. At the end of the reaction, methyl tert-butyl ether was added to the reaction solution to make the product precipitated. The precipitated product was dissolved and precipitated several times to provide 1.5g of the product.

$^1$H-NMR (400 MHz, DMSO-$d_6$) δ 8.03-8.00 (m, 56H), 5.75-5.68 (m, 5H), 4.45-4.37 (m, 26H), 3.55-5.45 (m, 3810H), 1.81-1.75 (m, 80H), 1.51-1.45 (m, 26H), 1.40-1.33(m, 133H), 0.85-0.83 (m, 12H),

56-89

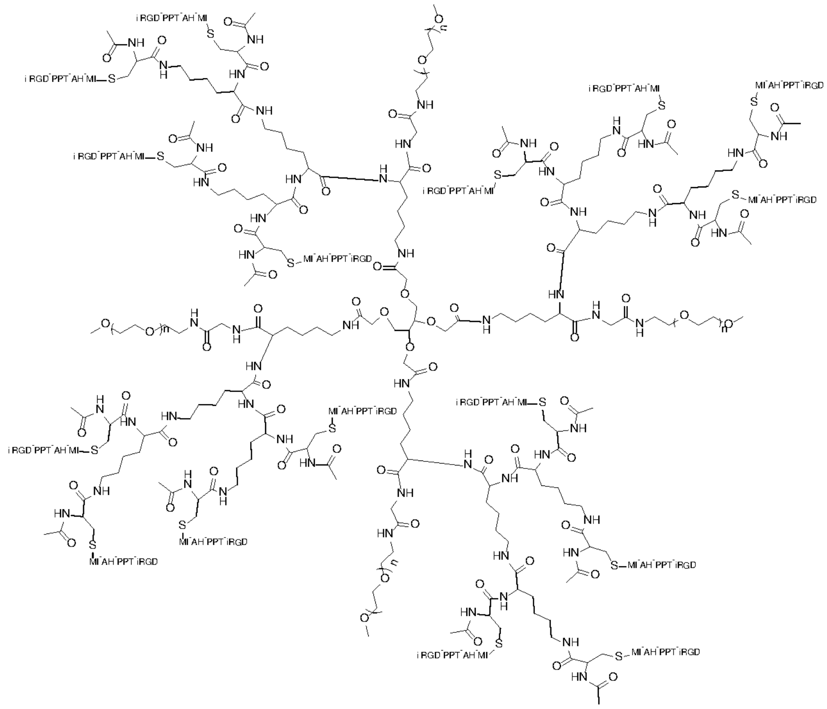

56-86 (0.0325mmol), MI-AH-PPT-iRGD (1.04g, 0.65mmol, purchased from Dangang Bio), were added in a 250 mL flask, and dissolved with DMSO (50mL), and the obtained solution was stirred in the dark under the condition of 40°C for 2 days, and then stirred at room temperature for 3 days. At the end of the reaction, methyl tert-butyl ether was added to the reaction solution, the upper layer solution was discarded, and the precipitated solid was dissolved with 10% methanol/dichloromethane, and then silica gel powder (5g) was added. The mixture was dried, loaded on silica gel column, and eluted with 5%-25% methanol/dichloromethane. 0.3g of the product was obtained with a yield of 44%.

$^1$H-NMR (400 MHz, DMSO-$d_6$) $\delta$ 8.35-8.30(m, 7H), 8.24-8.20 (m, 14H), 8.17-8.10 (m, 48H), 8.07-8.01 (m, 32H), 8.00-7.94(m, 20H), 7.86-7.80 (m, 24H), 7.78-7.71(m, 34H), 7.69-7.51 (m, 31H), 7.45-7.37 (m, 144H), 7.31-7.22 (m, 28H), 5.69-5.60 (m, 5H), 5.07-4.95 (m, 14H), 4.40-4.35(m, 61H), 4.23-4.06(m, 78H), 3.76-3.65 (m, 120H), 3.55-3.40 (m, 3810H), 2.82-2.76 (m, 35H), 2.65-2.60 (m, 16H), 2.11-2.05 (m, 25H), 1.78-1.70(m, 125H), 1.31-1.28 (m, 57H), 1.23-119 (m, 150H), 1.17-1.14 (m, 146H), 1.10-1.04 (m, 317H), 0.93-0.84 (m, 41H), 0.80-0.76(m, 240H)

**Example 2 Synergistic test of polyethylene glycol conjugated drug synergist on antitumor effect of anticancer drug on BALB/c nude mouse subcutaneous transplantation tumor models of human breast cancer MDA-MB-231 cells**

1. Preparation method:

Test sample:

1) a proper amount of anticancer drug (such as 49-166) was taken, and a proper amount of normal saline was added to prepare a solution with a proper concentration.
2) a proper amount of polyethylene glycol conjugated drug synergist to be tested (such as 45-164) + anticancer drug (such as 49-166) was taken, and a proper amount of normal saline was added to prepare a solution with a proper concentration.

Negative control: normal saline was directly used.

The prepared test samples and control sample were preserved at 2-8°C or in an ice box before administration, and the residual test samples and control sample after administration were treated as medical waste.

2. Cells and experimental animals

Human breast cancer cell MDA-MB-231: it was from the Cell Resource Center of Institute of Basic Medicine of Chinese Academy of Medical Sciences, cultured under the conditions of RPMI1640 + 10% FBS, 37°C, 5% $CO_2$.

Animal species & strain: BALB/c nude mice
Animal level: SPF level
Animal source: Beijing Vital River Laboratory Animal Technology Co., Ltd.
Animal age at tumor inoculation: about 4-5 weeks.

Animal weight at tumor inoculation: about 15-18 g. The weights of animals of the same sex were between 80-120% of the average weight.
Animal sex and number: female, 96 mice were purchased, 48 modeling animals were screened for final experiments, and the remaining animals were either handed over to veterinarian or euthanized.
Animals were reared in an independent ventilation system (IVC), at most 6 animals of the same group in each cage, and an SPF level animal house was provided, with the environmental conditions controlled as follows: room temperature 20-26°C, 40-70% of relative humidity and illumination with 12 hours light dark alternation. During quarantine domestication and testing period, qualified mouse feed (manufacturer: Beijing Keao Xieli Feed Co., Ltd.) was provided each day. The animals ate freely and drunk water freely.

3. Model establishment

MDA-MB-231 cells were revived, and cell passage amplification was carried out. When amplified to a sufficient number, the cells in the logarithmic growth phase were collected for cell inoculation.
According to the actual cell number, the cells were adjusted to have a concentration of $1\times10^8$/mL, and mixed with Matrigel (Matrix Basement Membrane Matrix, BD Co.) at a volume ratio of 1:1, to obtain a cell suspension with a concentration of $5\times10^7$/mL. The cell suspension was inoculated subcutaneously in the right armpit of 96 mice at 0.2 mL per mouse. The tumor growth was observed after inoculation, and 48 tumorigenic animals with the tumor volume of 77.30-292.27 $mm^3$ were finally screened and used for the test.

4. Animal grouping and dosing

The tumorigenic animals were randomly divided into 8 groups according to the tumor volume and the body weight, including: group 1 (negative control group, normal saline), group 6 (49-166, 48 mg/kg), group 8 (49-166+45-164, 48+176 mg/kg), 6 animals in each group. The animals were intravenously injected, for administration of each group at D1, D4, D7, D10, D13, D18, D21, D24, and at D27 the animals were euthanized.

5. Experimental results

5.1 General clinical observation

Observation frequency and time: during the experiment, all animals underwent gross observations twice daily.
Observation contents: including mental state, behavioral activities, food intake and the like of the animals.

5.2 Body weight

Test animal: all animals

Test time: after receiving, before inoculation, the day of grouping (i.e., D1, the day of first administration), 2 times per week after first administration, and before euthanasia, the animals were weighed. The animals were also weighed when they died accidentally or when they were dying or euthanized.

5.3 Measurement of tumor diameter:

Test animal: all animals

Test time: the day of grouping (i.e., D1, the day of first administration), 2 times per week after first administration, and before euthanasia, the long and short diameters of tumor were measured using a slide caliper and recorded, and

the tumor volume was calculated.

The tumor volume was calculated according to the following formula:

$$V=1/2\times\text{long diameter}\times\text{short diameter}^2$$

During the experiment, the general clinical symptoms of the animals were observed 2 times every day, and the body weight and tumor diameter were measured 10 times in total. The tumor was stripped after euthanization, and the tumor weight was weighed. The tumor volume, relative tumor volume RTV, relative tumor proliferation rate T/C% and tumor weight inhibition rate $IR_{TW}$% were calculated.

6. Analysis and evaluation of results

Statistical analysis: in this experiment, statistical software SPSS13.0 was used to process the data, and the measured data was expressed by mean value ± standard error. The specific analysis process was as follows

One-way analysis of variance (ANOVA) was used to carry out the statistical analysis. Comparative analysis between groups was performed by Tukey test if ANOVA had statistical significance ($P \leq 0.05$) and variance was homogeneous, and comparative analysis between groups was performed by Dunnett's T3 test if variance was inhomogeneous.

6.1 Evaluation of therapeutic efficacy based on the tumor volume

The relative tumor volume (RTV) and the relative tumor proliferation rate T/C% were calculated according to the following formula:

$$RTV=V_t/V_0$$

Vt: tumor volume obtained by measuring tumor every day

$V_0$: initial tumor volume (before administration)

$$T/C\%=\text{ average RTV of the administration group/average RTV of the control group}\times 100\ \%$$

If the relative tumor proliferation rate T/C% of the administration group was ≤ 40 %, and the RTV of the administration group was effective compared with the RTV of the negative control group ($P \leq 0.05$), tumor growth inhibition effect was achieved; on the other hand, if T/C% was > 40%, tumor growth was not inhibited.

6.2 Evaluation of therapeutic efficacy based on the tumor weight

After the experiment, tumor nodules were stripped and weighed, and the differences in tumor weight among the groups were compared to further calculate the tumor inhibition rate $IR_{TW}$. $IR_{TW}$>60% was taken as an effective reference indicator. The calculation was conducted according to the following formula:

$$IR_{TW}\ (\%) = (W_{\text{Control group}}-W_{\text{Administration group}})\ /W_{\text{Control group}}\times 100\ \%$$

6.3 Gross anatomy observation

The animals died during the experiment and the euthanized animals after observation period were subjected to gross anatomy for observation of their main organs, to see if there are obvious abnormalities visible to the naked eye.

6.4 Photograph recording

The pictures of the euthanized animals and tumors were taken.

The concrete results were as follows:

Throughout the experiment, no obvious abnormality was seen in each group of the animals. The body weight of each group of the animals slowly increased during the experiment, and no significant difference was seen between each test

sample group and the group 1 (P>0.05).

In the negative control group (group 1), the tumor gradually increased throughout the experiment, by the end of the experiment (D27), the group 1 had an average tumor volume of 2962.92±2176.59 $mm^3$ and an average RTV of 19.14±12.01; the average tumor volumes of the groups 6, 8 were 1690.27±785.04 $mm^3$, 1273.37±358.45 $mm^3$ respectively, and the average RTVs thereof were 13.03±5.43, 10.55±5.73 respectively.

The tumor growth trend of each group is shown in FIG. 1.

By the end of the experiment (D27), the T/C% values of the groups 6, 8 were 68.08%, 55.10% respectively, and the $IR_{TW}$% values thereof were 31.92%, 44.90% respectively.

At the end of the experiment, the tumors of the animals were weighed after euthanasia. The average tumor weights of the groups 1, 6, 8 were 2.555±2.207 g, 0.990±0.399 g, 0.684±0.165 g respectively. The $IR_{TW}$% of the groups 6, 8 were 61.24%, 73.21% respectively.

The schematic diagram of the tumor weight inhibition rate of each group is shown in FIG. 2.

Conclusion: under the experimental conditions, the test sample 49-166 at a dose of 48 mg/kg, and the test sample 49-166 at a dose of 48 mg/kg in combination with the test sample 45-164 at a dose of 176 mg/kg (49-166+45-164), which were respectively administered by tail vein injection, had an obvious inhibition effect on the growth of the BALB/c nude mouse subcutaneous transplantation tumor of human breast cancer MDA-MB-231 cells, and the tumor inhibition effect of 49-166+45-164 was obviously superior to that of 49-166.

The inventors found out through the experiments and the results that the polyethylene glycol conjugated drug synergist of the present invention could be used as a synergist of the anticancer effect of the anticancer drug on subcutaneous transplantation tumor (such as BALB/c nude mouse subcutaneous transplantation tumor of human breast cancer MDA-MB-231 cells), and the synergism was obvious.

## Claims

1. A polyethylene glycol conjugated drug synergist of formula (I) or a pharmaceutically acceptable salt thereof,

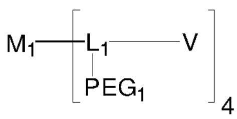

formula I

wherein:

$M_1$ is

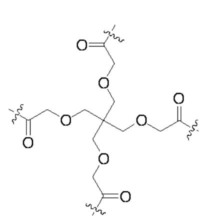

or

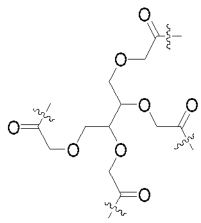

;

$PEG_1$ is single-arm polyethylene glycol segment, $PEG_1$ is connected to $L_1$ through carbonyl group or $PEG_1$ has amino group or activated amino group at its terminal, the number-average molecular weight of $PEG_1$ is 5k-40k, preferably 5k-10k or 10k-40k, and more preferably 10k;

$L_1$ is

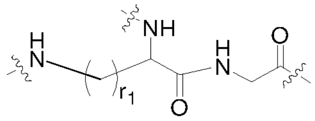

or

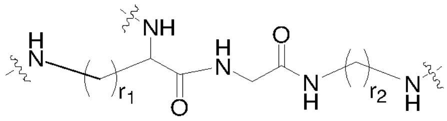

,

, each $r_1$ independently is 1, 2, 3, 4, 5 or 6, preferably 1, 2, 3 or 4, more preferably 3 or 4; $r_2$ is 1, 2, 3, 4, 5 or 6, preferably 1, 2, 3 or 4, more preferably 1 or 2;
preferably, $L_1$ is

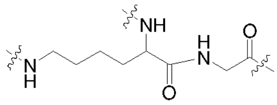 or 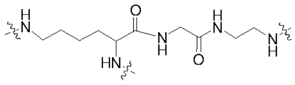 ;

V is

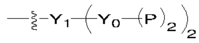 ;

Y1, Y0 are each independently selected from

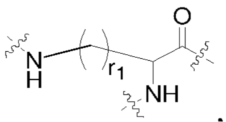 ,

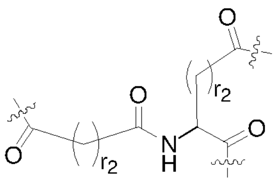 or 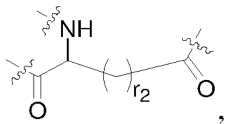 ,

$r_1$ is 1, 2, 3, 4, 5 or 6, $r_1$ is preferably 1, 2, 3 or 4, $r_1$ is more preferably 3 or 4, each $r_2$ independently is 1, 2, 3, 4, 5 or 6, each $r_2$ independently is preferably 1, 2, 3 or 4, each $r_2$ independently is more preferably 1 or 2;
preferably, Y1, Y0 are each independently selected from

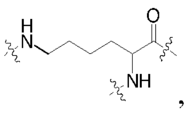 ,

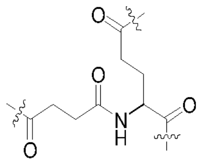 , 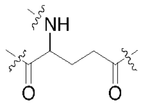 ;

or preferably, Y1 is selected from is 3 or 4, each $r_2$ independently is 1 or 2

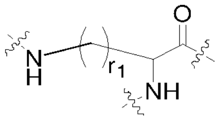 or 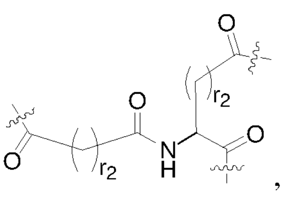 ,

$r_1$
more preferably, Y1 is selected from

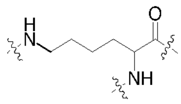 or 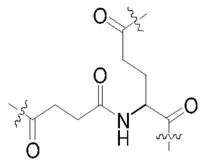 ;

or preferably, Y0 is selected from or 4, $r_2$ is 1 or 2;

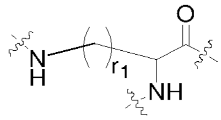 or 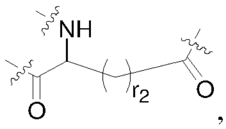 ,

$r_1$ is 3
more preferably, Y0 is selected from P is -Lv-T;

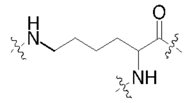 or 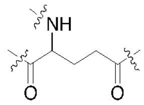 ;

$L_V$ is selected from

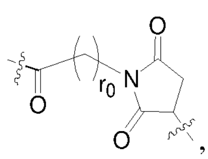 , 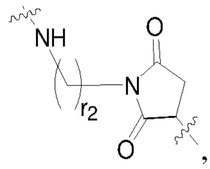 ,

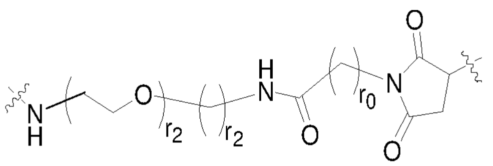 or 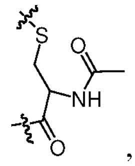 ,

each $r_0$ independently is 1 2, 3 4, 5 or 6, each $r_0$ independently is preferably 3, 4, 5 or 6, each $r_0$ independently is more preferably 5 or 6, each $r_2$ independently is 1, 2, 3, 4, 5 or 6, each $r_2$ independently is preferably 1, 2, 3 or 4, each $r_2$ independently is more preferably 1 or 2;
preferably, $L_V$ is selected from

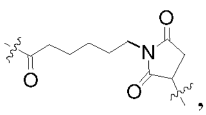 , 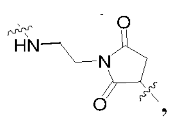 ,

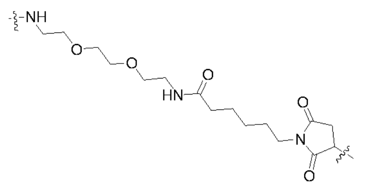 or 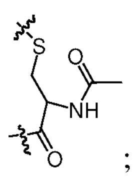 ;

Tis

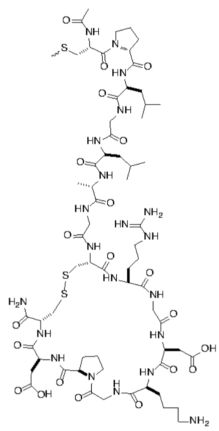 or 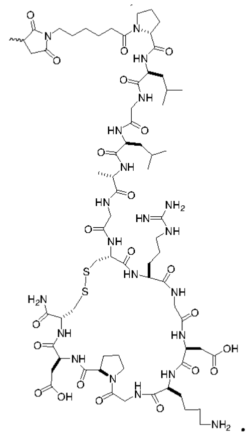.

2. The polyethylene glycol conjugated drug synergist or a pharmaceutically acceptable salt thereof according to claim 1, wherein, the polyethylene glycol conjugated drug synergist is selected from:

| No. | Structural formula |
|---|---|
| 45-164 | 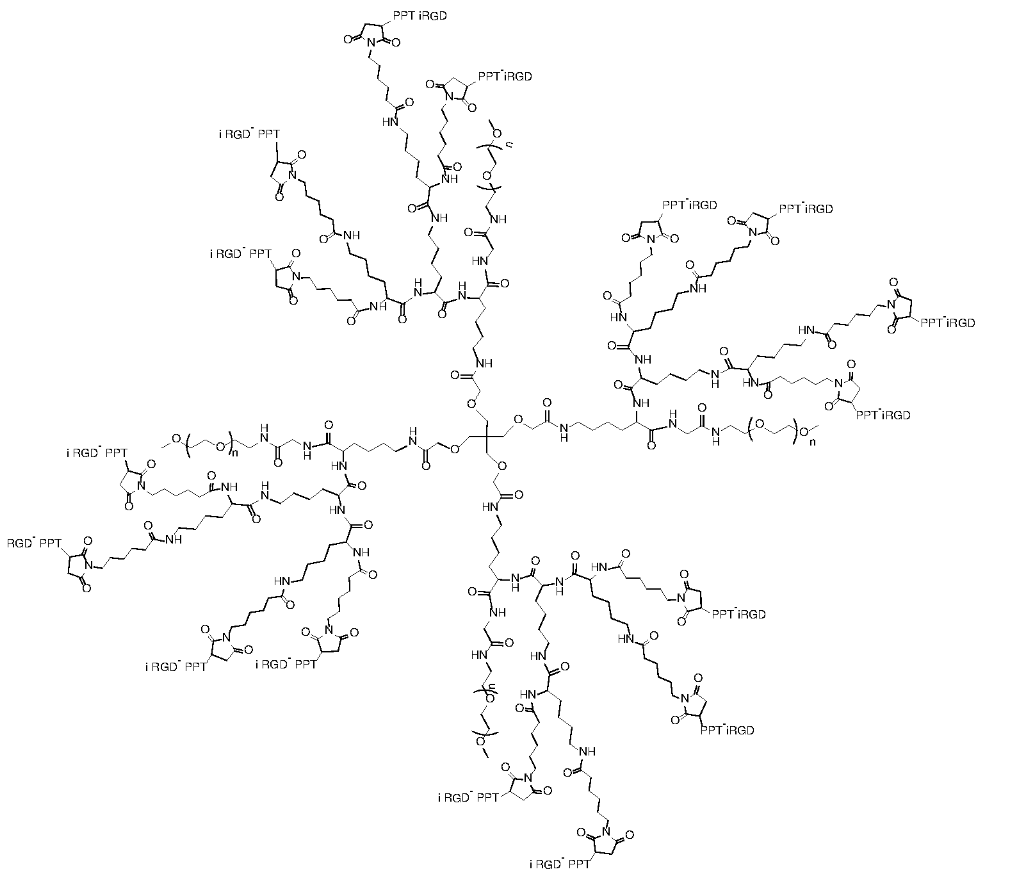 wherein, 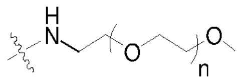 has a number-average molecular weight of 10k |

(continued)

| No. | Structural formula |
|---|---|
| 38-161 | 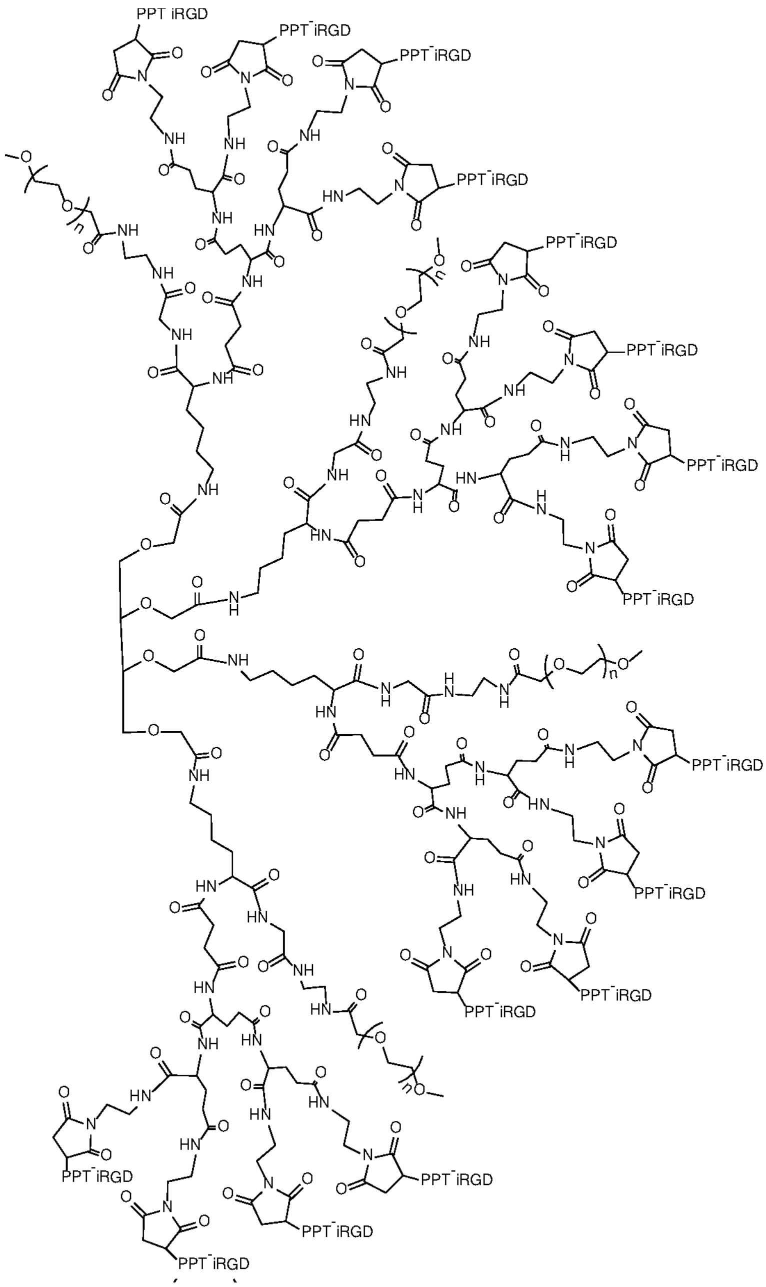 |
| | wherein, 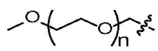 , has a number-average molecular weight of 10k |

(continued)

| No. | Structural formula | |
|---|---|---|
| 38-192 | 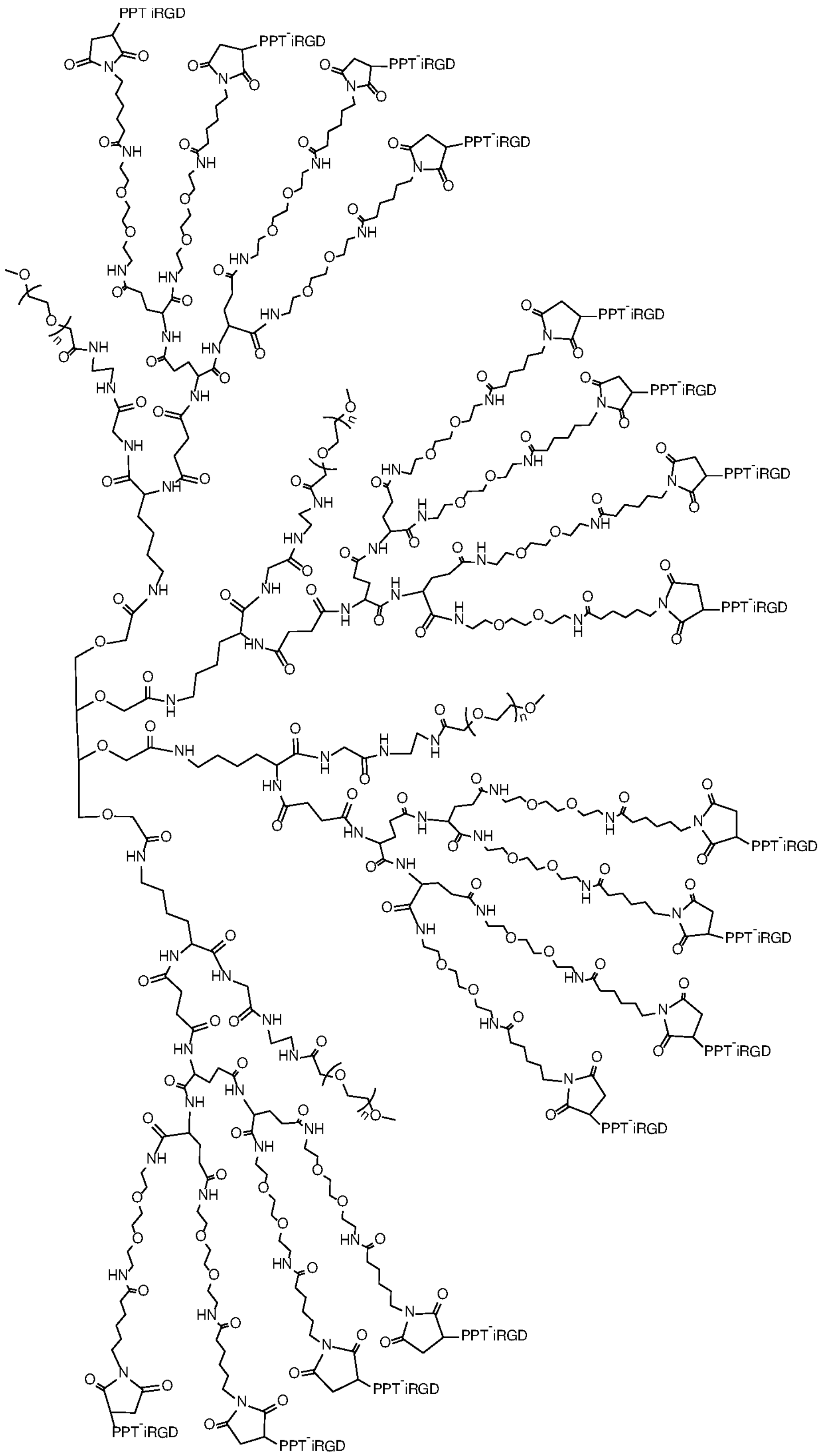 | |
| | wherein, 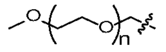 | has a number-average molecular weight of 10k |

(continued)

| No. | Structural formula |
| --- | --- |
| 52-95 | 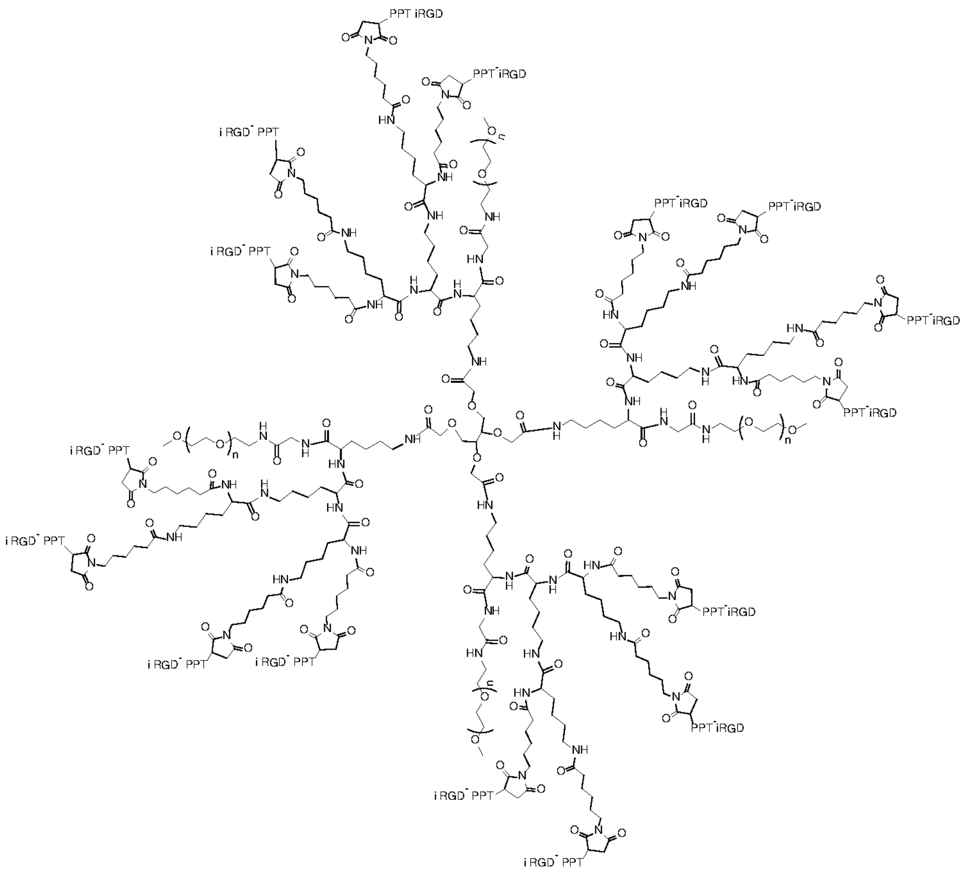 wherein, 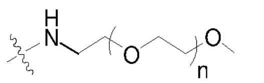, has a number-average molecular weight of 10k |

(continued)

| No. | Structural formula |
|---|---|
| 56-89 | 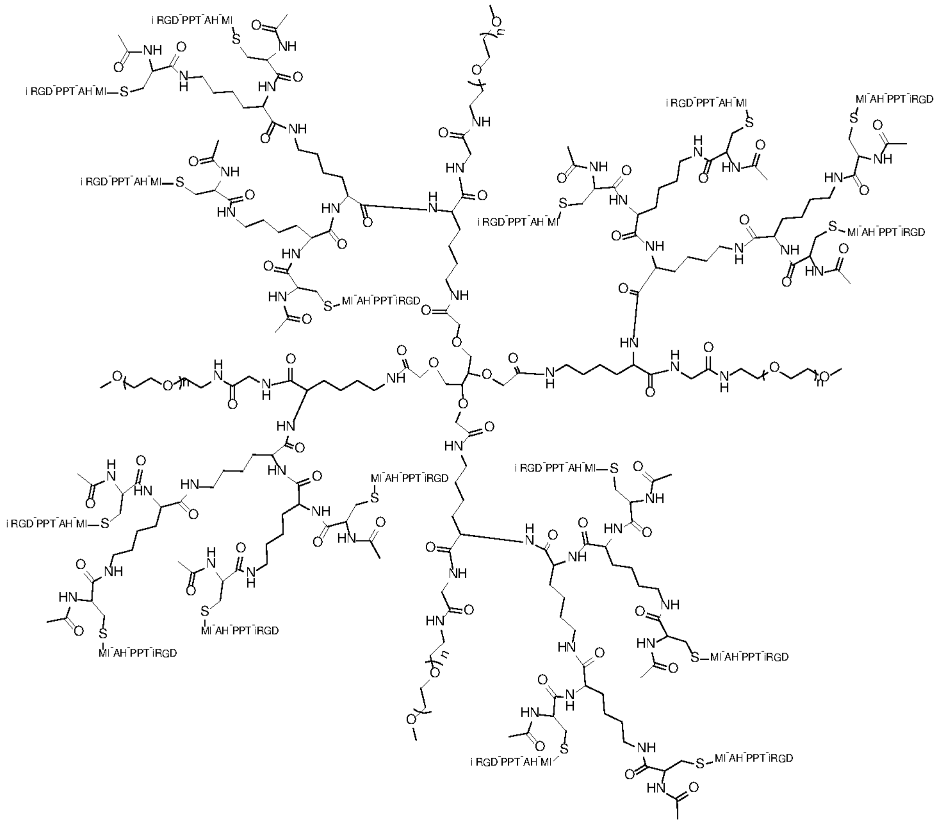<br>wherein, 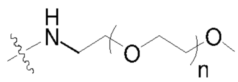 has a number-average molecular weight of 10k |

**3.** A method for preparing the polyethylene glycol conjugated drug synergist or a pharmaceutically acceptable salt thereof according to claim 1, comprising the following steps:

(1) preparing the intermediate

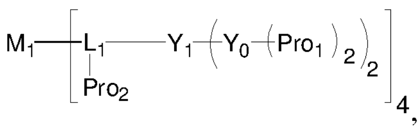

wherein:

$M_1$, $L_1$, $Y_1$, $Y_0$ are as defined in claim 1,
$Pro_2$ is a protecting group for carboxyl group, preferably, $Pro_2$ is benzyloxy group,
$Pro_1$ is a protecting group for amino group or carboxyl group, preferably, when $Pro_1$ is a protecting group for amino group, $Pro_1$ is tert-butoxy carbonyl group, preferably, when $Pro_1$ is a protecting group for carboxyl group, $Pro_1$ is tert-butoxy group;

(2) subjecting the intermediate

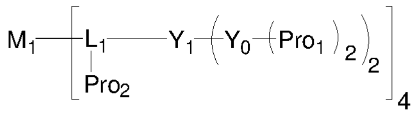

to the first deprotection, to obtain the intermediate

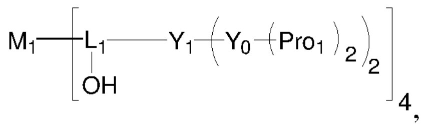

wherein,

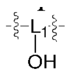

has carboxyl group at its terminal;
(3) allowing the intermediate

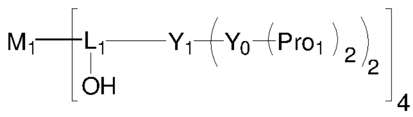

and $PEG_1$ to carry out amidation reaction, to obtain the intermediate

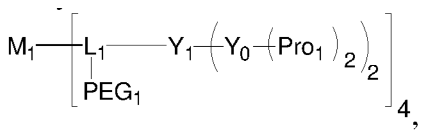

wherein, $PEG_1$ is as defined in claim 1;
(4) when $Pro_1$ is a protecting group for amino group, subjecting the intermediate

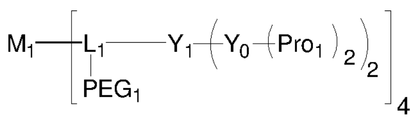

to the second deprotection, to obtain the intermediate

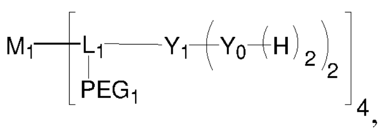

wherein,

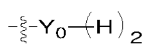

has amino group at its terminal; or, when $Pro_1$ is a protecting group for carboxyl group, subjecting the intermediate

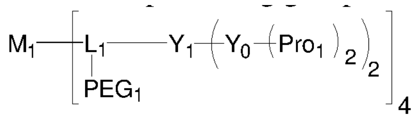

to the third deprotection, to obtain the intermediate

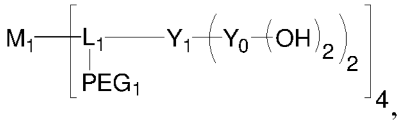

wherein,

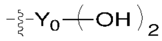

has carboxyl group at its terminal;
(5) allowing the intermediate

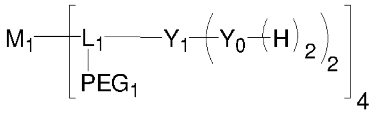

and

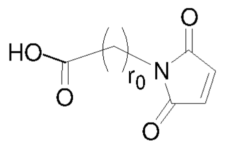

to carry out amidation reaction to obtain the intermediate

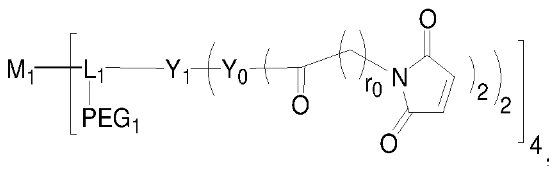

, wherein, $r_0$ is as defined in claim 1; or allowing the intermediate

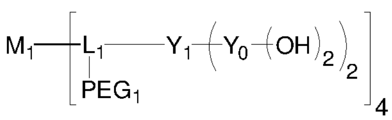

and

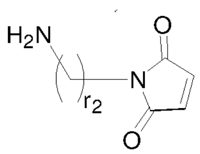

or

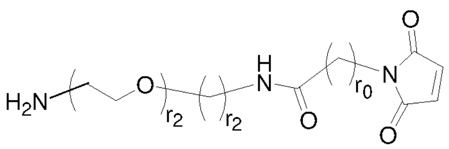

to carry out amidation reaction, to obtain the intermediate

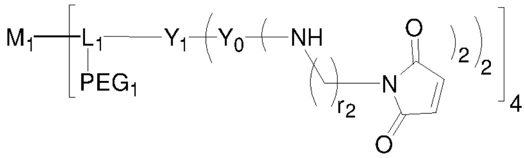

or

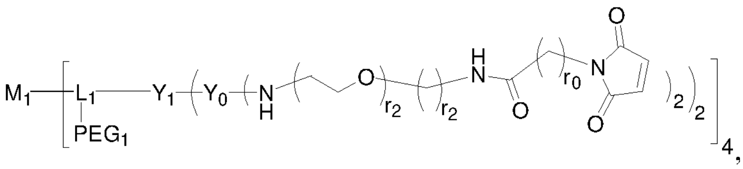

wherein, $r_0$, $r_2$ are as defined in claim 1;
(6) allowing the intermediate

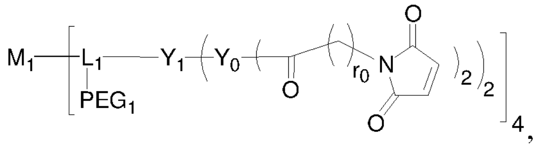

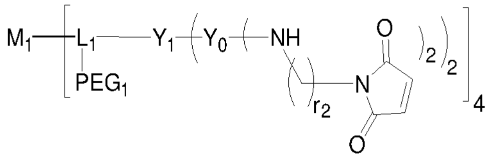

or

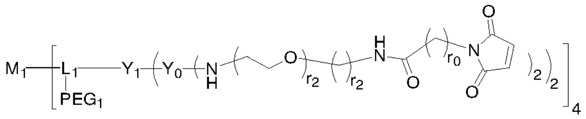 and 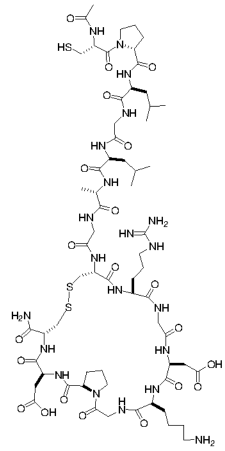

to carry out addition reaction, to obtain the polyethylene glycol conjugated drug synergist as defined in claim 1; preferably, the intermediate

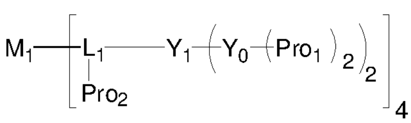

is selected from:

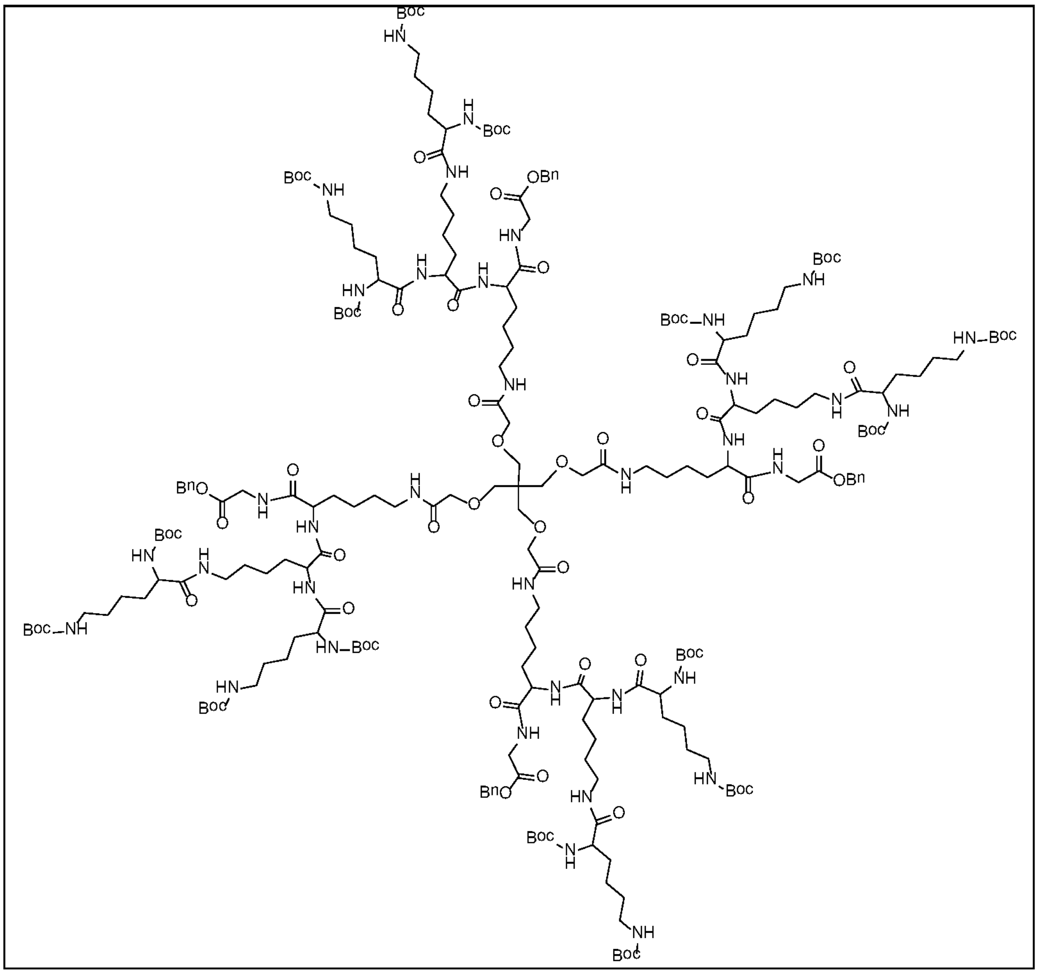

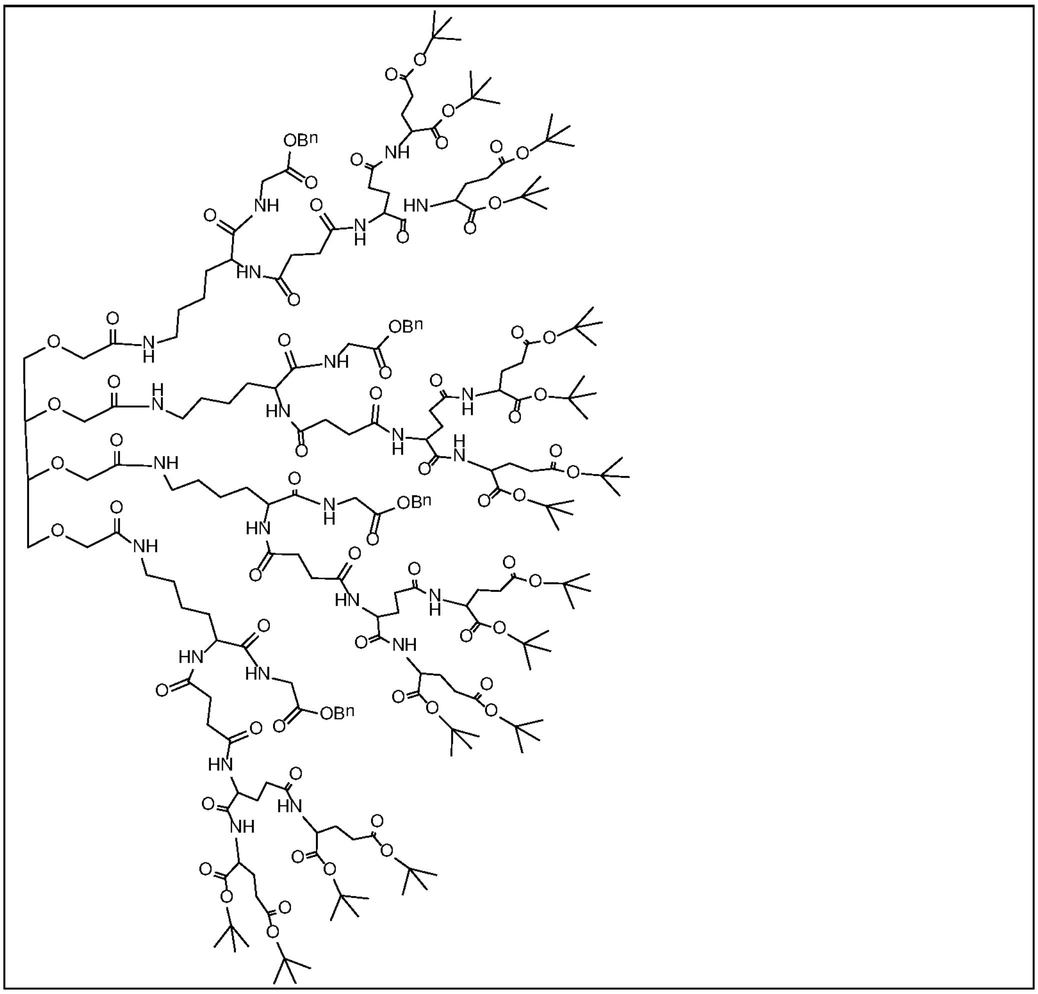

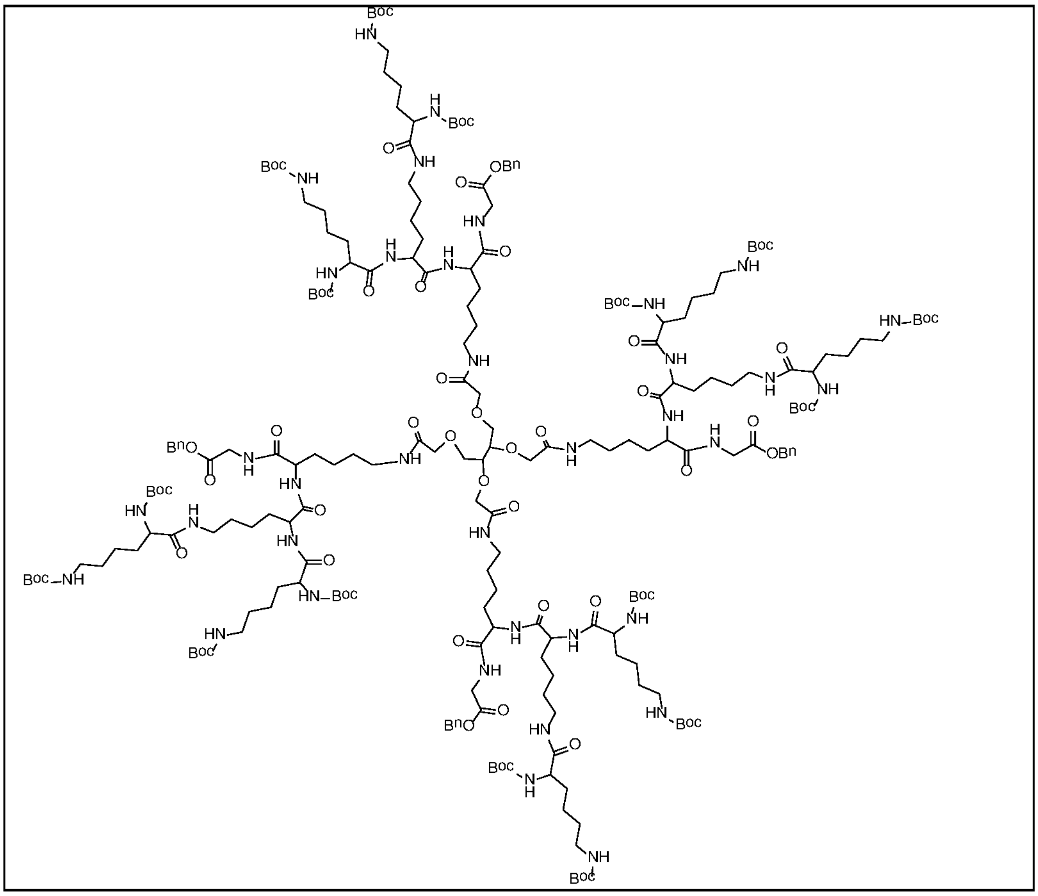

.

4. An intermediate for preparing the polyethylene glycol conjugated drug synergist or a pharmaceutically acceptable salt thereof according to any one of claims 1-2, the intermediate being selected from:

| No. | Structural formula |
| --- | --- |
| 45-136 | 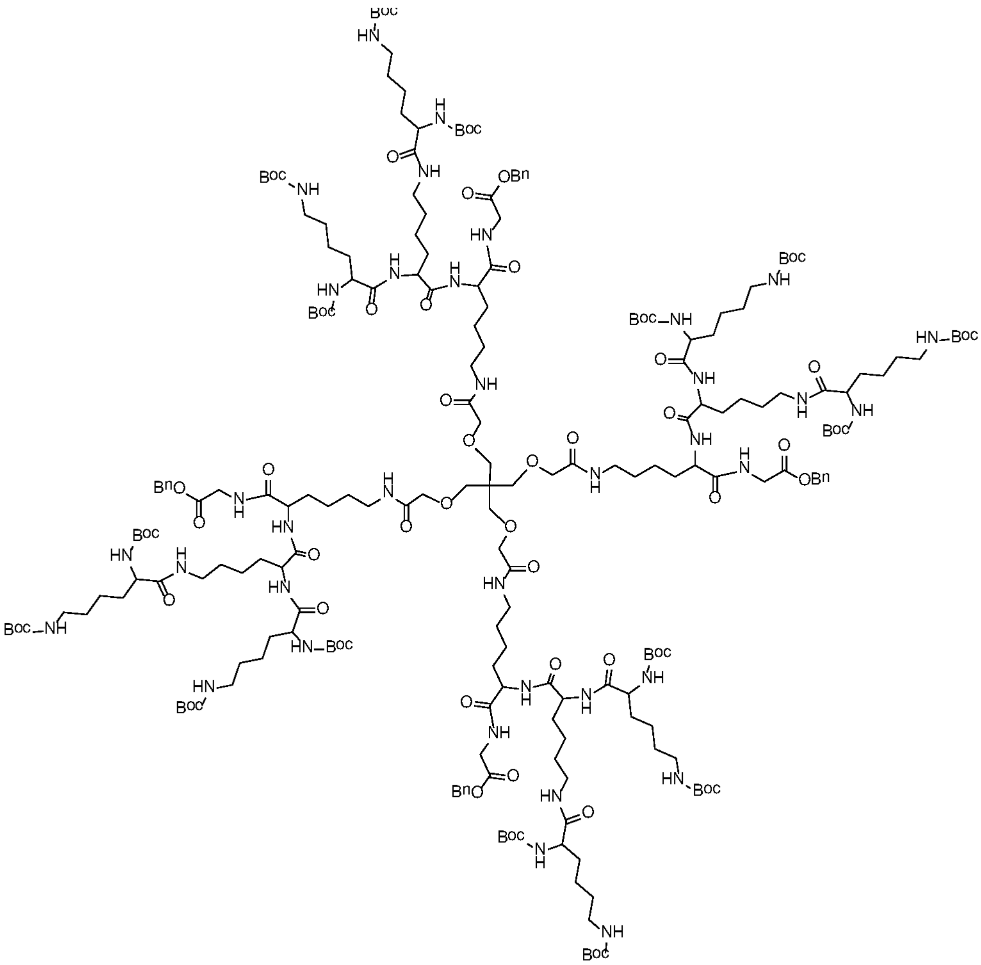 |

(continued)

| No. | Structural formula |
|---|---|
| 38-146 | 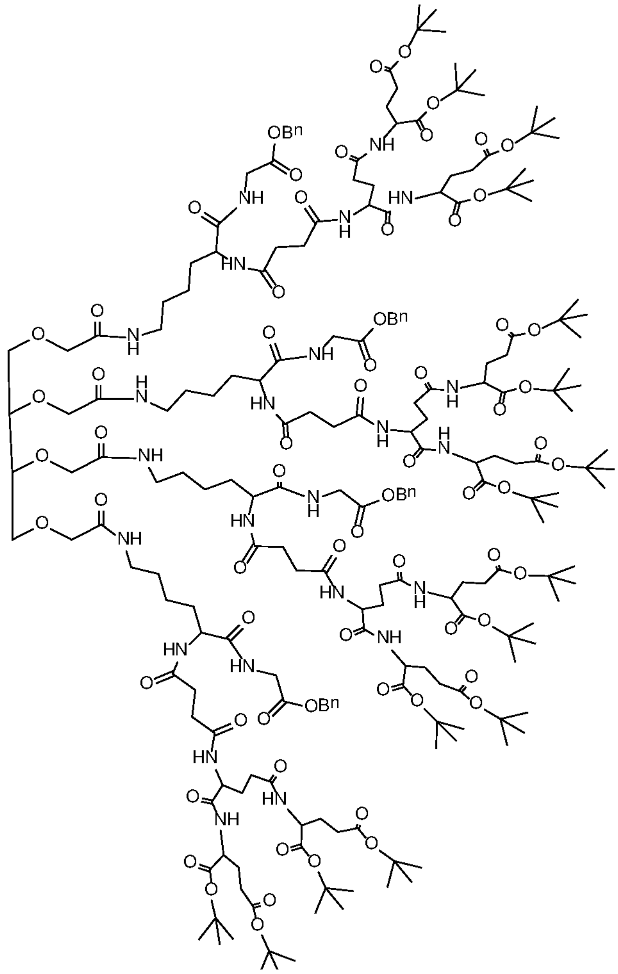 |

(continued)

| No. | Structural formula |
|---|---|
| 52-86 | 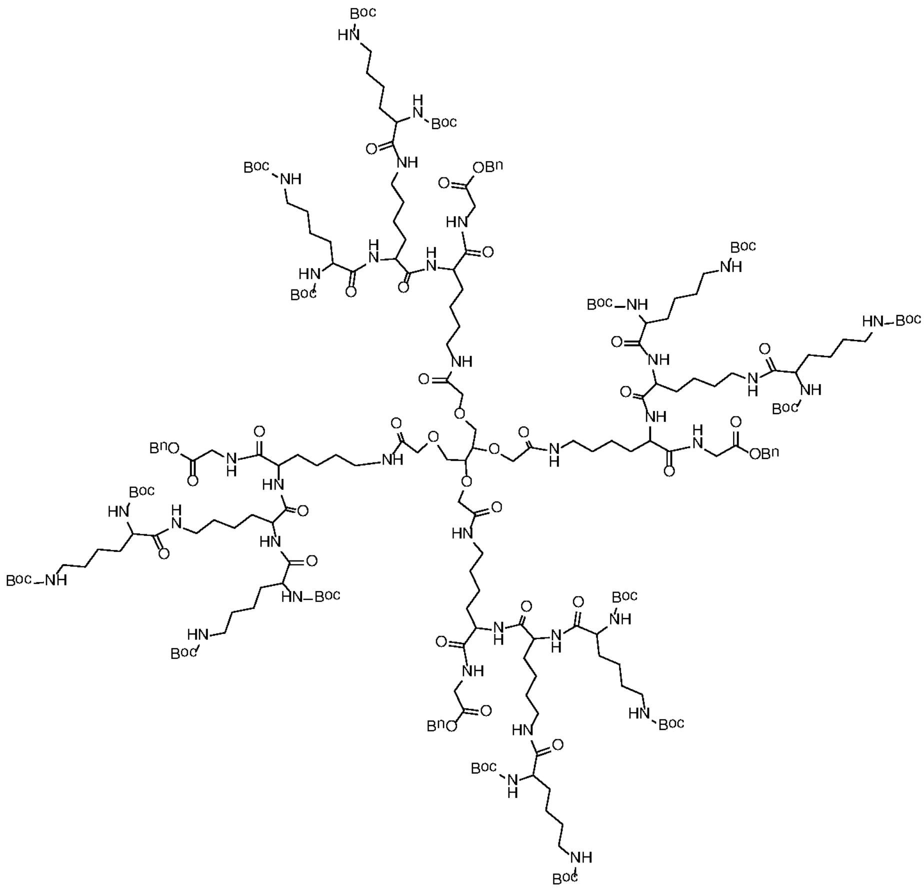 |

**5.** A composition comprising the polyethylene glycol conjugated drug synergist or a pharmaceutically acceptable salt thereof according to any one of claims 1-2; optionally, the composition further comprises one or more pharmaceutically acceptable excipients.

**6.** The composition according to claim 5, wherein, the composition further comprises an anticancer drug.

**7.** The composition according to claim 6, wherein, the anticancer drug is polyethylene glycol conjugated drug of formula (A) or a pharmaceutically acceptable salt thereof

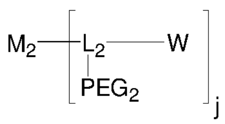

formula A

wherein:

$M_2$ is

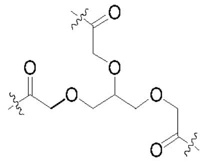 or 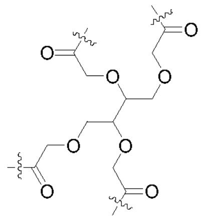 ;

j is 3 or 4;

$PEG_2$ is single-arm polyethylene glycol segment, $PEG_2$ is connected to $L_2$ through carbonyl group or $PEG_2$ has amino group or activated amino group at its terminal, $PEG_2$ has a number-average molecular weight of 5k-40k, preferably 5k-10k or 10k-40k, more preferably 5k;

$L_2$ is

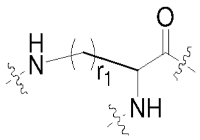 or 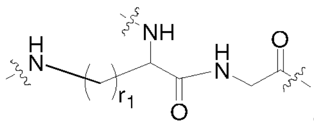 ,

each $r_1$ independently is 1, 2, 3, 4, 5 or 6, each $r_1$ independently is preferably 1, 2, 3 or 4, each $r_1$ independently is more preferably 3 or 4,

preferably, $L_2$ is

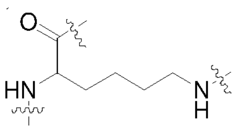 or 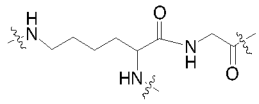 ;

Wis

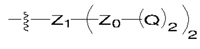 or 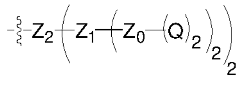 ;

$Z_2$, $Z_1$, $Z_0$ each independently are

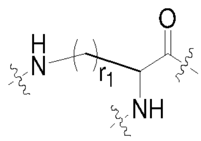 , 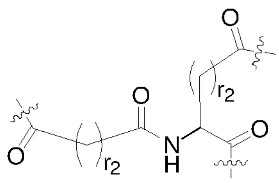

or

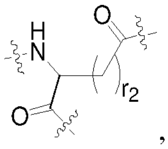 ,

$r_1$ is 1, 2, 3, 4, 5 or 6, $r_1$ is preferably 1, 2, 3 or 4, $r_1$ is more preferably 3 or 4, each $r_2$ independently is 1, 2, 3, 4, 5 or 6, each $r_2$ independently is preferably 1, 2, 3 or 4, each $r_2$ independently is more preferably 1 or 2, preferably, $Z_2$, $Z_1$, $Z_0$ each independently are

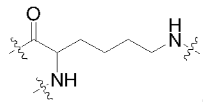, 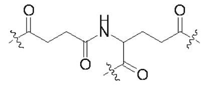

or

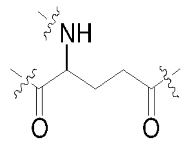,

or preferably, $Z_2$ is

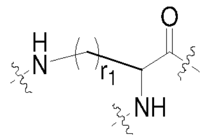,

$r_1$ is 3 or 4,
more preferably, $Z_2$ is

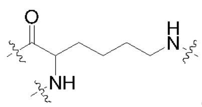,

or preferably, $Z_1$ is

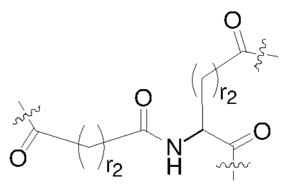 or 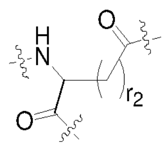,

each $r_2$ independently is 1 or 2,
more preferably, $Z_1$ is

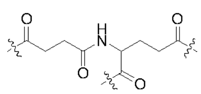 or 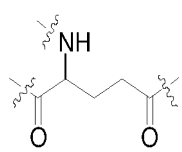,

or preferably, $Z_0$ is

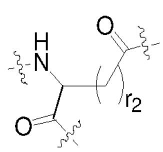,

$r_2$ is 1 or 2,
more preferably, $Z_0$ is

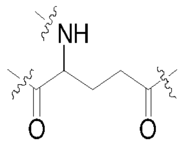 ;

Q is N-AC;
N is

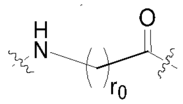

or G, $r_0$ is 1, 2, 3, 4, 5 or 6, preferably 3, 4, 5 or 6, more preferably 5 or 6,
preferably, N is

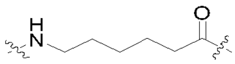

or G;
AC is SB7, NPB, SN38, LPT, PCB, DOX, PTX or AXT, preferably PTX or SN38.

**8.** The composition according to claim 7, wherein, the polyethylene glycol conjugated drug is selected from:

| No. | Structural formula |
| --- | --- |
| 49-166 | 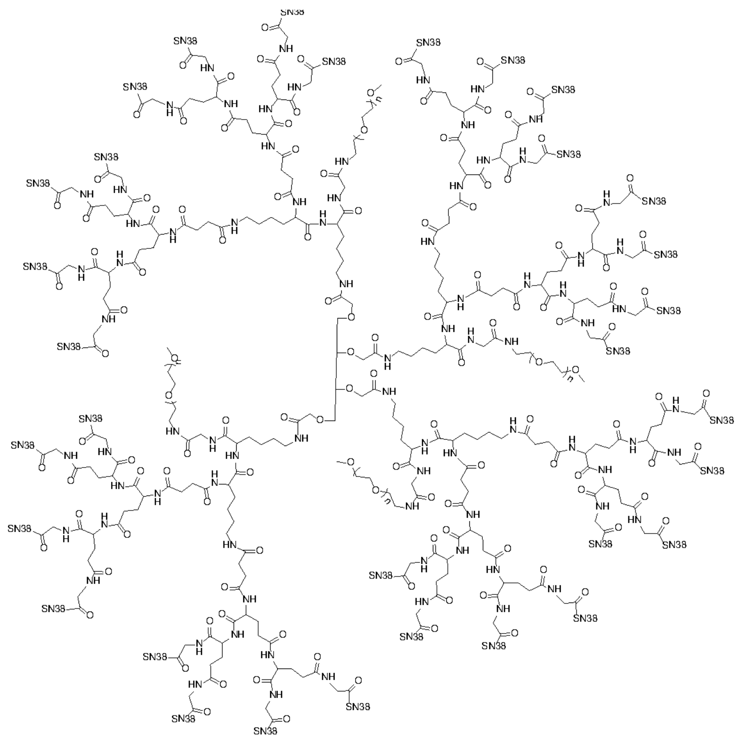 wherein, 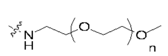 has a number-average molecular weight of 5k |

**9.** Use of the polyethylene glycol conjugated drug synergist or a pharmaceutically acceptable salt thereof according to any one of claims 1-2 or the composition according to claim 5 in the preparation of a medicament for enhancing

the therapeutic efficacy of treating and/or preventing a disease;
preferably, the disease is cancer, and the cancer is selected from: colon cancer, leukemia, lymphoma, bladder cancer, bone cancer, brain tumor, medulloblastoma, glioma, breast cancer, adenoma/carcinoid, adrenal cortical cancer, pancreatic islet cell cancer, cervical cancer, endometrial cancer, ovarian cancer, colorectal cancer, skin cancer, esophageal cancer, eye cancer, gallbladder cancer, stomach cancer, head and neck cancer, liver cancer, melanoma, Kaposi's sarcoma, kidney cancer, oral cancer, lung cancer, nasopharyngeal cancer, neuroblastoma, ovarian cancer, pancreatic cancer, thyroid cancer, parathyroid penile cancer, prostate cancer, urethral cancer, vaginal cancer, vulvar cancer, anal cancer, sarcoma, including metastasis of the aforementioned cancers.

**10.** Use of the composition according to any one of claims 6-8 in the preparation of a medicament for treating and/or preventing a disease, wherein the disease refers to a disease treated by the anticancer drug as defined in any one of claims 6-8;
preferably, the disease is cancer, and the cancer is selected from: colon cancer, leukemia, lymphoma, bladder cancer, bone cancer, brain tumor, medulloblastoma, glioma, breast cancer, adenoma/carcinoid, adrenal cortical cancer, pancreatic islet cell cancer, cervical cancer, endometrial cancer, ovarian cancer, colorectal cancer, skin cancer, esophageal cancer, eye cancer, gallbladder cancer, stomach cancer, head and neck cancer, liver cancer, melanoma, Kaposi's sarcoma, kidney cancer, oral cancer, lung cancer, nasopharyngeal cancer, neuroblastoma, ovarian cancer, pancreatic cancer, thyroid cancer, parathyroid penile cancer, prostate cancer, urethral cancer, vaginal cancer, vulvar cancer, anal cancer, sarcoma, including metastasis of the aforementioned cancers.

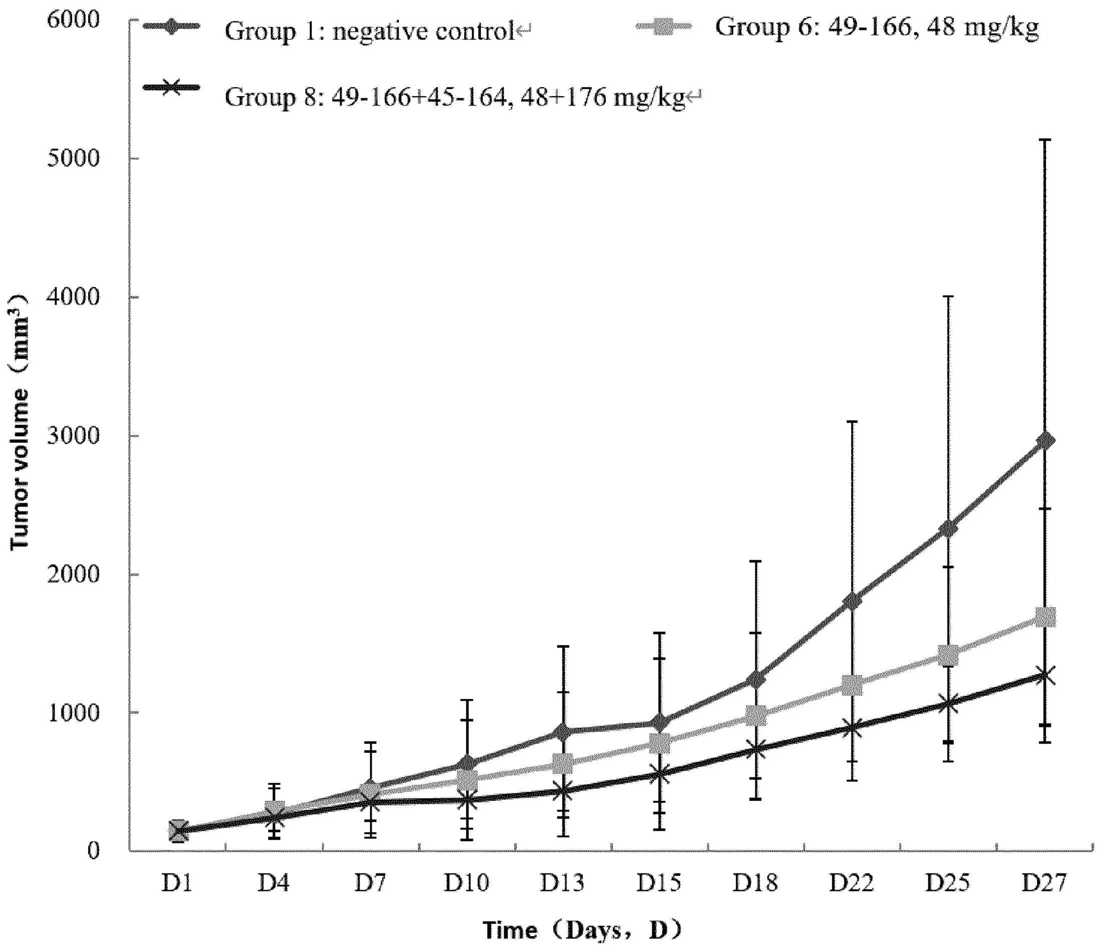
Group 1: negative control
Group 6: 49-166, 48 mg/kg
Group 8: 49-166+45-164, 48+176 mg/kg
6000
5000
4000
3000
2000
1000
0
Tumor volume（mm³）
D1
D4
D7
D10
D13
D15
D18
D22
D25
D27
Time（Days，D）

Figure 1

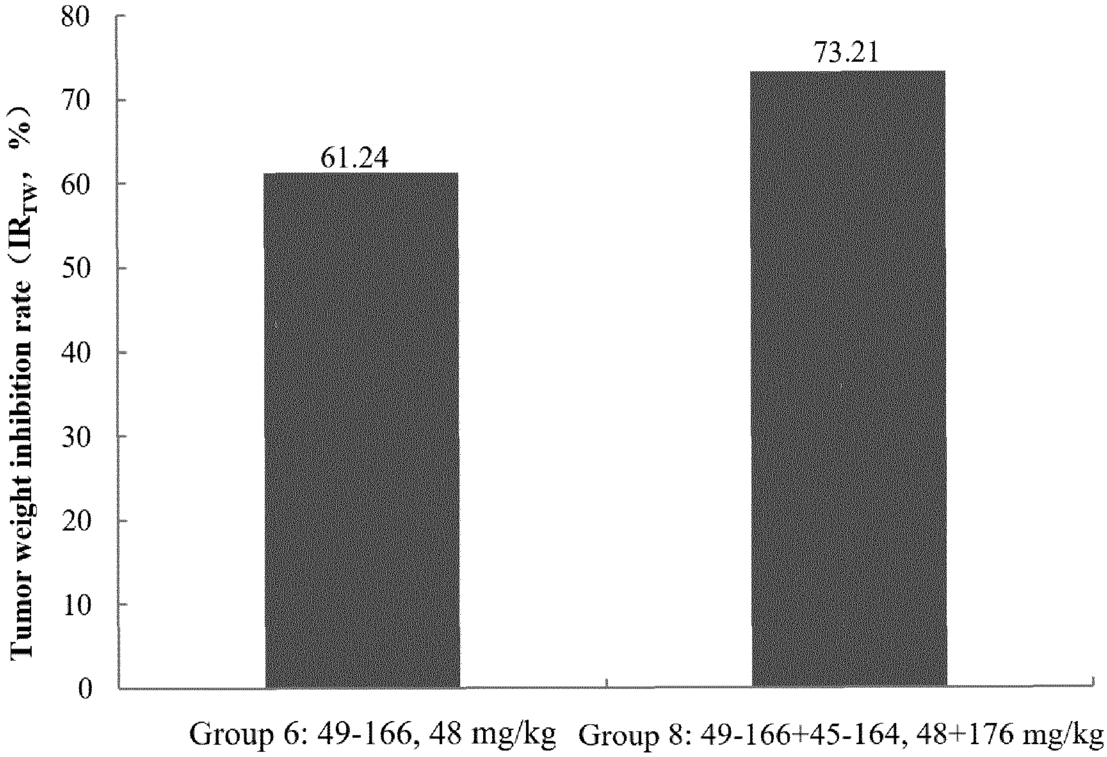
80
70
60
50
40
30
20
10
0
Tumor weight inhibition rate（$IR_{TW}$，%）
61.24
73.21
Group 6: 49-166, 48 mg/kg
Group 8: 49-166+45-164, 48+176 mg/kg

Figure 2

**INTERNATIONAL SEARCH REPORT**

International application No.
**PCT/CN2021/107555**

**A. CLASSIFICATION OF SUBJECT MATTER**

A61K 47/55(2017.01)i; A61K 47/60(2017.01)i; A61K 31/517(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS; CNTXT; DWPI; SIPOABS; CNKI; ISI web of Science; STNext: 重庆阿普格雷生物科技; 李高全; 聚乙二醇; 多臂; 叶酸; 偶联PEG; polyethylene glycol; multi arm; folic acid; FA; conjugate

**C. DOCUMENTS CONSIDERED TO BE RELEVANT**

| Category* | Citation of document, with indication, where appropriate, of the relevant passages | Relevant to claim No. |
|---|---|---|
| PX | CN 112851928 A (CHONGQING APU GELEI BIOTECHNOLOGY CO., LTD.) 28 May 2021 (2021-05-28) claims 1-21 | 1-10 |
| A | CN 108350022 A (BOARD OF REGENTS OF THE UNIVERSITY OF NEBRASKA) 31 July 2018 (2018-07-31) claims 1 and 14 | 1-10 |
| A | CN 104987504 A (NANJING MINGZHEN PHARMACEUTICAL TECHNOLOGY CO., LTD.) 21 October 2015 (2015-10-21) claims 1-10 | 1-10 |
| A | CN 101541332 A (ENZON PHARMACEUTICALS, INC.) 23 September 2009 (2009-09-23) claims 1-29 | 1-10 |
| A | CN 107670050 A (CHONGQING APU GELEI BIOTECHNOLOGY CO., LTD.) 09 February 2018 (2018-02-09) entire document | 1-10 |
| A | CN 107670048 A (CHONGQING APU GELEI BIOTECHNOLOGY CO., LTD.) 09 February 2018 (2018-02-09) entire document | 1-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 October 2021** | **20 October 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/107555**

**C. DOCUMENTS CONSIDERED TO BE RELEVANT**

| Category* | Citation of document, with indication, where appropriate, of the relevant passages | Relevant to claim No. |
|---|---|---|
| A | CHOI, Sung Won et al. "Multifunctional siRNA Delivery System: Polyelectrolyte Complex Micelles of Six-arm PEG Conjugate of siRNA and Cell Penetrating Peptide with Crosslinked Fusogenic Peptide;" *Biotechnol. Prog.;*, Vol. 26, No. 1, 13 November 2009 (2009-11-13), pp. 57-63; the abstract, and figure 1 | 1-10 |
| A | CN 110339367 A (BEIJING JENKEM TECHNOLOGY CO., LTD.) 18 October 2019 (2019-10-18) claims 1-19 | 1-10 |

Form PCT/ISA/210 (second sheet) (January 2015)

**INTERNATIONAL SEARCH REPORT**
**Information on patent family members**

International application No.
**PCT/CN2021/107555**

| Patent document cited in search report | | | Publication date (day/month/year) | Patent family member(s) | | | Publication date (day/month/year) |
|---|---|---|---|---|---|---|---|
| CN | 112851928 | A | 28 May 2021 | WO | 2021104120 | A1 | 03 June 2021 |
| CN | 108350022 | A | 31 July 2018 | AU | 2016354564 | A1 | 21 June 2018 |
| | | | | CA | 3004444 | A1 | 18 May 2017 |
| | | | | WO | 2017083794 | A1 | 18 May 2017 |
| | | | | US | 2020085842 | A1 | 19 March 2020 |
| | | | | EP | 3374369 | A1 | 19 September 2018 |
| | | | | JP | 2018537445 | A | 20 December 2018 |
| | | | | EP | 3696185 | A1 | 19 August 2020 |
| | | | | JP | 6849233 | B2 | 24 March 2021 |
| | | | | US | 10933071 | B2 | 02 March 2021 |
| | | | | AU | 2016354564 | B2 | 29 April 2021 |
| | | | | EP | 3374369 | B1 | 18 March 2020 |
| | | | | US | 10485809 | B2 | 26 November 2019 |
| | | | | US | 2018318318 | A1 | 08 November 2018 |
| CN | 104987504 | A | 21 October 2015 | CN | 104987504 | B | 01 May 2018 |
| CN | 101541332 | A | 23 September 2009 | MX | 2009002855 | A | 30 March 2009 |
| | | | | KR | 20090057383 | A | 05 June 2009 |
| | | | | AU | 2007296056 | A1 | 20 March 2008 |
| | | | | BR | PI0716812 | A2 | 05 November 2013 |
| | | | | CA | 2662981 | A1 | 20 March 2008 |
| | | | | EP | 2073820 | A4 | 16 July 2014 |
| | | | | WO | 2008034124 | A2 | 20 March 2008 |
| | | | | WO | 2008034124 | A3 | 07 August 2008 |
| | | | | EP | 2073820 | A2 | 01 July 2009 |
| | | | | JP | 2010503708 | A | 04 February 2010 |
| | | | | RU | 2009114154 | A | 20 October 2010 |
| | | | | IL | 197517 | D0 | 24 December 2009 |
| | | | | AU | 2007296056 | B2 | 13 September 2012 |
| CN | 107670050 | A | 09 February 2018 | CN | 107670050 | B | 07 June 2019 |
| CN | 107670048 | A | 09 February 2018 | WO | 2019041733 | A1 | 07 March 2019 |
| | | | | EP | 3677284 | A4 | 19 May 2021 |
| | | | | US | 2020261588 | A1 | 20 August 2020 |
| | | | | EP | 3677284 | A1 | 08 July 2020 |
| | | | | CN | 109843333 | A | 04 June 2019 |
| | | | | JP | 2020531591 | A | 05 November 2020 |
| | | | | CN | 107670048 | B | 26 April 2019 |
| CN | 110339367 | A | 18 October 2019 | WO | 2019192488 | A1 | 10 October 2019 |
| | | | | KR | 20200134287 | A | 01 December 2020 |

Form PCT/ISA/210 (patent family annex) (January 2015)